(19) Europäisches Patentamt
European Patent Office
Office européen des brevets

(11) Veröffentlichungsnummer: **0 201 852**
**B1**

(12) **EUROPÄISCHE PATENTSCHRIFT**

(45) Veröffentlichungstag der Patentschrift: **16.08.90**

(21) Anmeldenummer: **86106208.1**

(22) Anmeldetag: **06.05.86**

(51) Int. Cl.⁵: **C 07 D 231/44,**
C 07 D 401/04, A 01 N 43/56,
A 01 N 43/40

(54) **Schädlingsbekämpfungsmittel auf Basis von Pyrazolderivaten.**

(30) Priorität: **17.05.85 DE 3517843**
**30.01.86 DE 3602728**

(43) Veröffentlichungstag der Anmeldung:
**20.11.86 Patentblatt 86/47**

(45) Bekanntmachung des Hinweises auf die Patenterteilung:
**16.08.90 Patentblatt 90/33**

(84) Benannte Vertragsstaaten:
**AT BE CH DE FR GB IT LI NL SE**

(56) Entgegenhaltungen:
**EP-A-0 005 240**
**EP-A-0 009 634**
**EP-A-0 017 066**
**EP-A-0 154 115**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Jensen-Korte, Uta, Dr.**
**Geibelstrasse 9**
**D-4000 Düsseldorf (DE)**
Erfinder: **Gehring, Reinhold, Dr.**
**Dasnöckel 49**
**D-5600 Wuppertal (DE)**
Erfinder: **Schallner, Otto, Dr.**
**Noldeweg 22**
**D-4019 Monheim (DE)**
Erfinder: **Stetter, Jörg, Dr.**
**Gellertweg 4**
**D-5600 Wuppertal 1 (DE)**
Erfinder: **Wroblowsky, Heinz-Jürgen, Dr.**
**Gladbacher Strasse 34**
**D-4018 Langenfeld (DE)**
Erfinder: **Becker, Benedikt, Dr.**
**Metzkausener Strasse 14**
**D-4010 Mettmann (DE)**
Erfinder: **Homeyer, Bernhard, Dr.**
**Obere Strasse 28**
**D-5090 Leverkusen 3 (DE)**
Erfinder: **Behrenz, Wolfgang, Dr.**
**Untergründemich 14**
**D-5063 Overath-Steinenbrück (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 201 852 B1

(72) Erfinder: **Stendel, Wilhelm, Dr.**
**In den Birken 55**
**D-5600 Wuppertal 1 (DE)**
Erfinder: **Andrews, Peter, Dr.**
**Gellertweg 2**
**D-5600 Wuppertal 1 (DE)**

**Beschreibung**

Die Erfindung betrifft die Verwendung von teilweise bekannten 5-Aminopyrazolderivaten als Schädlingsbekämpfungsmittel, insbesondere als Insektizide, Akarizide und Nematizide.

Es ist bereits bekannt, daß Pyrazolderivate, wie beispielsweise das 1,4-Dimethyl-5-[N,N-(dimethyl)-carbamoyloxy]-3-methylthiomethyl-pyrazol oder das 5-[N,N-(Dimethyl)-carbamoyloxy]-1-methyl-3-methylsulfinylmethylpyrazol oder das 5-[N,N-(Dimethyl)-carbamoyloxy]-1-isopropyl-3-methylsulfonylmethylpyrazol oder das 5-[N,N-(Dimethyl)-carbamoyloxy]-1-isobutyl-5-[N,N-(dimethyl)-carbamoyloxy]-3-methylthiomethylpyrazol oder das 1-Cyclohexyl-5-[N,N-(dimethyl)-carbamoyloxy]-3-methylsulfonylpyrazol insektizide, nematizide und fungizide Wirkung besitzen. (Vgl. DE—OS 2 819 932; DE—OS 2 839 270 oder DE—OS 2 912 494).

Die Wirkungshöhe bzw. die Wirkungsdauer dieser Verbindungen sind jedoch, insbesondere bei bestimmten Insekten oder bei niedrigen Anwendungskonzentrationen nicht immer völlig zufriedenstellend.

Außerdem ist bekannt, daß bestimmte Cumarinyl-phosphorsäureester, wie beispielsweise der O,O-Diethyl-O-(3-chlor-4-methyl-cumarin-7-yl)-thionophosphorsäureester sehr gute Wirkung gegen bestimmte Ektoparasiten zeigt (vgl. z.B. "Pflanzenschutz und Schädlingsbekämpfung" K. H. Büchel, G. Thieme Verlag Stuttgart 1977, S. 38).

Aber auch die Wirksamkeit dieser Verbindungsklasse ist, insbesondere bei niedrigen Aufwandmengen und Konzentrationen nicht immer völlig zufriedenstellend.

Schießlich sind bestimmte 5-Amino-1-aryl-pyrazole bekannt (vgl. z.B. Pharmaco. Ed. Sci. *26*, 276—293 [1971] oder Mycopathologica *74*, 7—14 [1981] bzw. C.A. *96*: 1964 llj, sowie C.A. *95*: 36257 q).

Über eine Wirksamkeit dieser Verbindungsklasse gegen Insekten, Milben oder Nematoden ist bisher jedoch nichts bekannt.

Es wurde gefunden, daß die teilweise bekannten 5-Aminopyrazole der allgemeinen Formel (I)

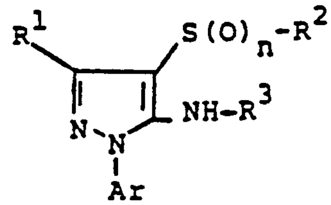

(I)

in welcher

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht oder für jeweils gegebenenfalls einfach oder mehrfach, gleich oder verschieden substituiertes Phenylalkyl oder Phenyl mit 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für Wasserstoff, geradkettiges oder verzweigtes Alkyl mit 1 bis 4 Kohlenstoffatomen oder für einen Rest

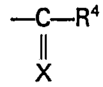

steht, wobei

X für Sauerstoff oder Schwefel steht, und

$R^4$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, außerdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten die bei $R^2$ genannten in Frage kommen,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis

zu 4 Kohlenstoffatomen und bis zu 9 gleichen oder verschiedenen Halogenatomen oder ein Rest

$$—S(O)_m—R^5$$

wobei

$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

m für eine Zahl 0, 1 oder 2 steht und

n ebenfalls für eine Zahl 0, 1 oder 2 steht,

insektizide, akarizide und nematizide Eigenschaften besitzen.

Überraschenderweise zeigen die erfindungsgemäß zu verwendenden 5-Aminopyrazole der allgemeinen Formel (I) eine erheblich verbesserte insektizide, akarizide und nematizide Wirksamkeit, als die aus dem Stand der Technik bekannten pyrazolderivate, wie beispielsweise das 1,4-Dimethyl-5-[N,N-(dimethyl)-carbamoyloxy]-3-methylthiomethyl-pyrazol oder das 5-[N,N-(Dimethyl)-carbamoyloxy]-1-methyl-3-methylsulfinylmethyl-pyrazol oder das 5-[N,N-(Dimethyl)-carbamoyloxy]-1-isopropyl-3-methylsulfinylmethyl-pyrazol oder das 5-[N,N-(Dimethyl)-carbamoyloxy]-1-isobutyl-3-methyl-sulfinylmethyl-pyrazol oder das 1-Cyclohexyl-5-[N,N-(dimethyl)-carbamoyloxy]-3-methylthiomethyl-pyrazol oder das 1-Cyclohexyl-5-[N,N-(dimethyl)-carbamoyloxy]-3-methylsulfinylpyrazol oder das 1-Cyclohexyl-5-[N,N-(dimethyl)-carbamoyloxy]-3-methylsulfonyl-pyrazol oder die aus dem Stand der Technik bekannten Cumarinyl-phosphorsäureester, wie beispielsweise der O,O-Diethyl-O-(3-chlor-4-methyl-cumarin-7-yl)-thionophosphorsäureester, welches chemisch und/oder wirkungsmäßig naheliegende Verbindungen sind.

Die erfindungsgemäß zu verwendenden 5-Aminopyrazolderivate sind durch die Formel (I) allgemein definiert. Bevorzugt sind diejenigen Verbindungen der Formel (I), bei welchen

$R^1$ für Wasserstoff, Methyl, Ethyl, n- oder i-Propyl und Trifluormethyl steht,

$R^2$ für Methyl, Ethyl, n- oder i-Propyl, n-, i-, s- oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Wasserstoff, Methyl, Ethyl, n- oder i-Propyl oder für einen Rest

$$\begin{array}{c} —C—R^4 \\ \| \\ X \end{array}$$

steht, wobei

X für Sauerstoff oder Schwefel steht und

$R^4$ für Wasserstoff, Methyl, Ethyl, n- und i-Propyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Brommethyl, 3-Chlorpropyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest

$$—S(O)_m—R^5,$$

wobei

$R^5$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht,

m für eine Zahl 0, 1 oder 2 steht und

n ebenfalls für eine Zahl 0, 1 oder 2 steht.

Im einzelnen seien außer den bei den Herstellungsbeispielen genannten Verbindungen die folgenden 5-Aminopyrazole der allgemeinen Formel (I) genannt:

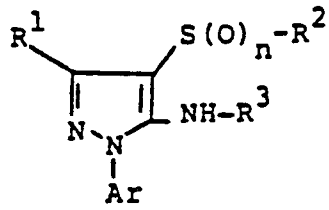

(I)

| $R^1$ | $R^2$ | $R^3$ | n | Ar |
|---|---|---|---|---|
| $CH_3$ | $CH_3$ | H | 0 | 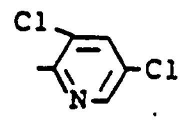 |
| $CH_3$ | $CH_3$ | H | 1 | 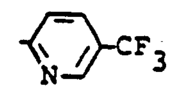 |
| $CH_3$ | $CH_3$ | $-CO-C_2H_5$ | 2 | 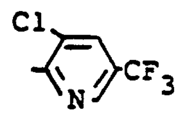 |
| $CF_3$ | $CH_3$ | H | 0 | 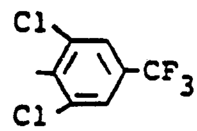 |
| $CF_3$ | $CF_3$ | H | 0 | 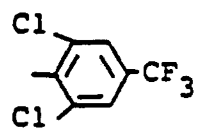 |
| $CF_3$ | $CCl_2F$ | H | 0 | 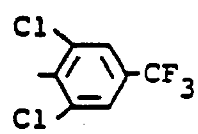 |
| $CF_3$ | $CClF_2$ | H | 0 | 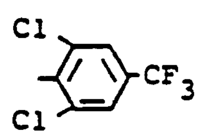 |
| $CF_3$ | $C_2H_5$ | H | 0 | 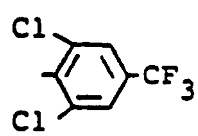 |
| $CF_3$ | $CH_3$ | H | 1 | 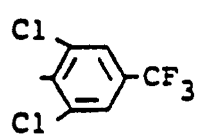 |
| $CF_3$ | $CF_3$ | H | 1 | 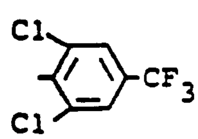 |

| $R^1$ | $R^2$ | $R^3$ | n | Ar |
|---|---|---|---|---|
| $CF_3$ | $CCl_2F$ | H | 1 | 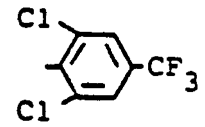 |
| $CF_3$ | $C_2H_5$ | H | 1 | 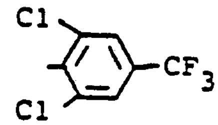 |
| $CF_3$ | $CH_3$ | H | 2 | 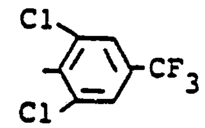 |
| $CF_3$ | $CF_3$ | H | 2 | 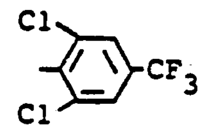 |
| $CF_3$ | $CCl_2F$ | H | 2 | 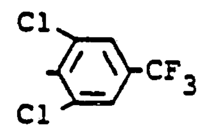 |
| $CF_3$ | $CFCl_2$ | H | 2 | 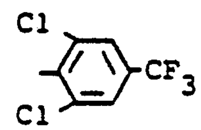 |
| $CF_3$ | $C_2H_5$ | H | 2 | 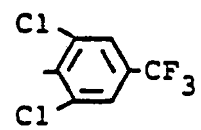 |
| $CH_3$ | $CH_3$ | H | 0 | 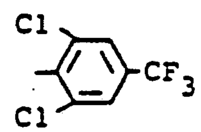 |
| $CH_3$ | $C_2H_5$ | H | 0 | 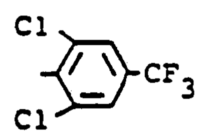 |
| $CH_3$ | $CH_3$ | H | 1 | 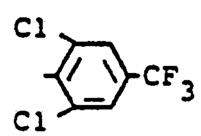 |

| $R^1$ | $R^2$ | $R^3$ | n | Ar |
|---|---|---|---|---|
| $CH_3$ | $CH_3$ | H | 2 | 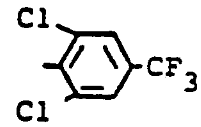 |
| $CH_3$ | $C_2H_5$ | H | 1 | 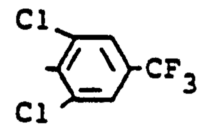 |
| $CH_3$ | $C_2H_5$ | H | 2 | 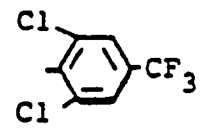 |
| $CH_3$ | $CF_2Cl$ | H | 0 | 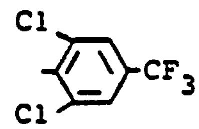 |
| $CH_3$ | $CF_2Cl$ | H | 1 | 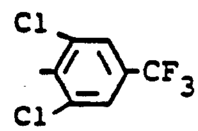 |
| $CH_3$ | $CF_2Cl$ | H | 2 | 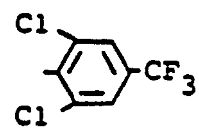 |
| $CH_3$ | $CF_3$ | H | 1 | 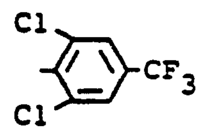 |
| $CH_3$ | $CF_3$ | H | 2 | 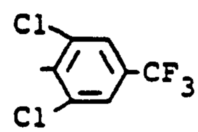 |
| $CH_3$ | $CCl_2F$ | H | 1 | 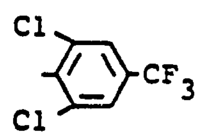 |
| $CH_3$ | $CCl_2F$ | H | 2 | 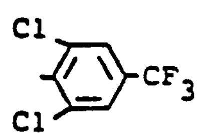 |

| $R^1$ | $R^2$ | $R^3$ | n | Ar |
|---|---|---|---|---|
| H | $CF_3$ | H | 0 | 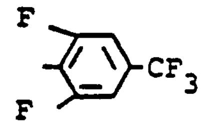 |
| H | $CF_3$ | H | 1 | 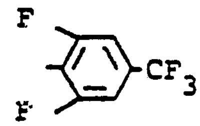 |
| H | $CF_3$ | H | 2 | 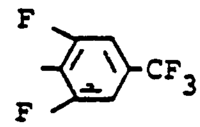 |
| H | $CCl_2F$ | H | 0 | 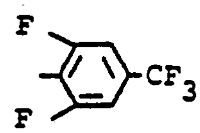 |
| H | $CCl_2F$ | H | 1 | 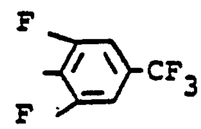 |
| H | $CCl_2F$ | H | 2 | 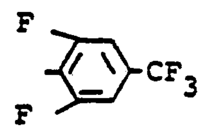 |
| H | $CF_2Cl$ | H | 0 | 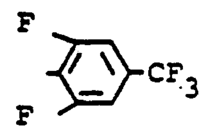 |
| H | $CF_2Cl$ | H | 1 | 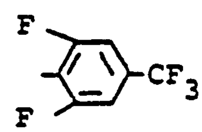 |
| H | $CF_2Cl$ | H | 2 | 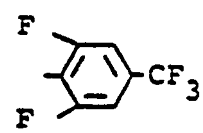 |
| $CH_3$ | $CF_3$ | H | 0 | 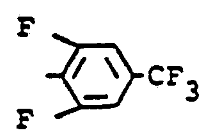 |

| $R^1$ | $R^2$ | $R^3$ | n | Ar |
|---|---|---|---|---|
| $CH_3$ | $CF_3$ | H | 1 | 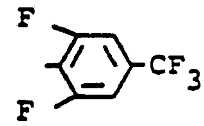 |
| $CH_3$ | $CF_3$ | H | 2 | 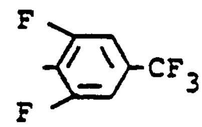 |
| $CH_3$ | $CCl_2F$ | H | 0 | 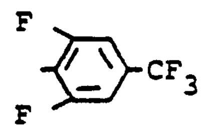 |
| $CH_3$ | $CCl_2F$ | H | 1 | 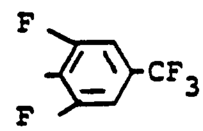 |
| $CH_3$ | $CCl_2F$ | H | 2 | 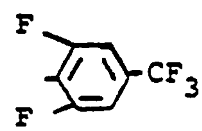 |
| H | $CH_3$ | H | 0 | 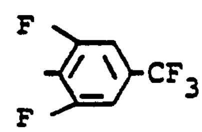 |
| H | $CH_3$ | H | 1 | 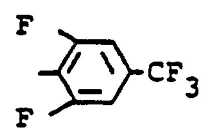 |
| H | $CH_3$ | H | 2 | 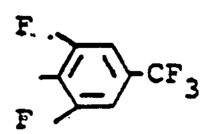 |
| H | $C_2H_5$ | H | 0 | 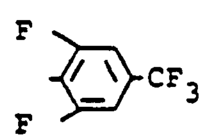 |
| H | $C_2H_5$ | H | 1 | 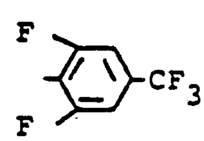 |

| $R^1$ | $R^2$ | $R^3$ | n | Ar |
|---|---|---|---|---|
| H | $C_2H_5$ | H | 2 | 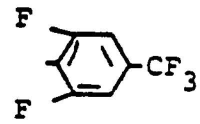 |
| H | $CF_3$ | H | 0 | 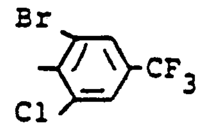 |
| H | $CF_3$ | H | 1 | 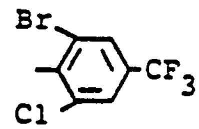 |
| H | $CF_3$ | H | 2 | 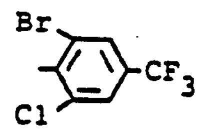 |
| H | $CCl_2F$ | H | 0 | 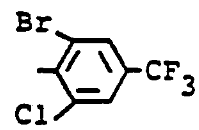 |
| H | $CCl_2F$ | H | 1 | 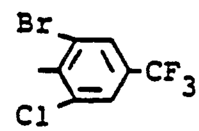 |
| H | $CCl_2F$ | H | 2 | 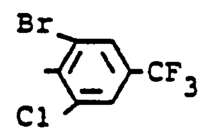 |
| $CH_3$ | $CH_3$ | H | 0 | 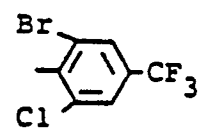 |
| $CH_3$ | $CH_3$ | H | 1 | 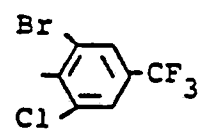 |
| $CH_3$ | $CH_3$ | $CH_3$ | 2 | 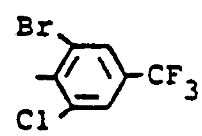 |

| $R^1$ | $R^2$ | $R^3$ | n | Ar |
|---|---|---|---|---|
| $CH_3$ | $CCl_2F$ | H | 0 | 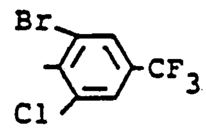 |
| $CH_3$ | $CCl_2F$ | H | 1 | 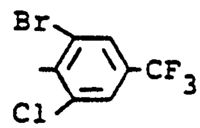 |
| $CH_3$ | $CCl_2F$ | H | 2 | 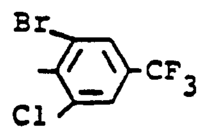 |
| $CH_3$ | $CF_3$ | H | 0 | 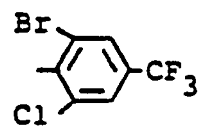 |
| $CH_3$ | $CF_3$ | H | 1 | 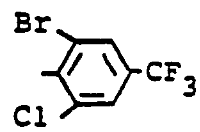 |
| $CH_3$ | $CF_3$ | H | 2 | 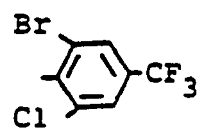 |
| $CH_3$ | $CF_3$ | H | 0 | 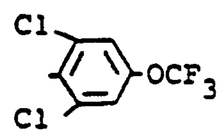 |
| $CH_3$ | $CF_3$ | H | 1 | 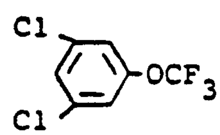 |
| $CH_3$ | $CF_3$ | H | 2 | 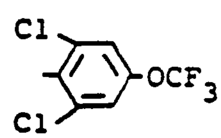 |
| $CH_3$ | $CCl_2F$ | H | 0 | 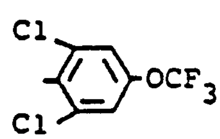 |

| $R^1$ | $R^2$ | $R^3$ | n | Ar |
|---|---|---|---|---|
| $CH_3$ | $CCl_2F$ | H | 1 | 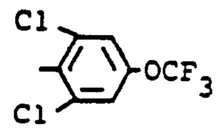 |
| $CH_3$ | $CCl_2F$ | H | 2 | 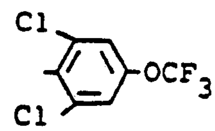 |
| H | $CH_3$ | H | 0 | 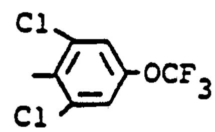 |
| H | $CH_3$ | H | 1 | 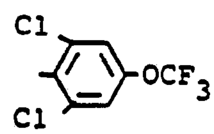 |
| H | $CH_3$ | H | 2 | 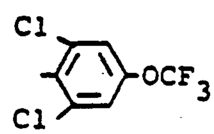 |
| H | $C_2H_5$ | H | 0 | 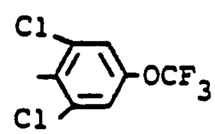 |
| H | $C_2H_5$ | H | 1 | 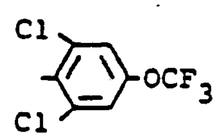 |
| H | $C_2H_5$ | H | 2 | 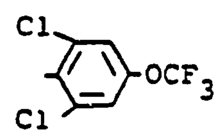 |
| $CH_3$ | $CH_3$ | H | 0 | 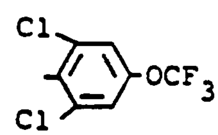 |
| $CH_3$ | $CH_3$ | H | 1 | 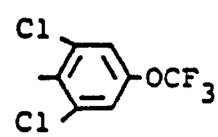 |

| $R^1$ | $R^2$ | $R^3$ | n | Ar |
|---|---|---|---|---|
| $CH_3$ | $CH_3$ | H | 2 | 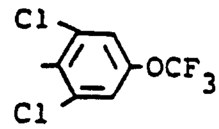 |
| H | $CF_3$ | H | 0 | 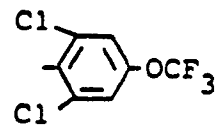 |
| H | $CF_3$ | H | 1 | 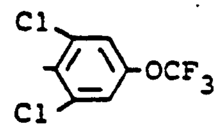 |
| H | $CF_3$ | H | 2 | 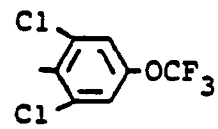 |
| H | $CCl_2F$ | $CH_3$ | 0 | 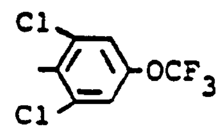 |
| H | $CClF_2$ | H | 0 | 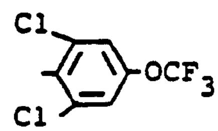 |
| H | $CClF_2$ | H | 1 | 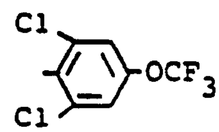 |
| H | $CClF_2$ | $CH_3$ | 1 | 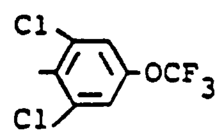 |
| H | $CClF_2$ | H | 2 | 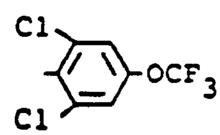 |
| H | $CF_3$ | $-CH_2CH_2-OH$ | 0 | 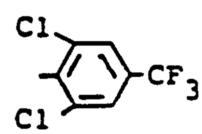 |

| $R^1$ | $R^2$ | $R^3$ | n | Ar |
|---|---|---|---|---|
| H | $CF_3$ | $-CH_2CH_2-OH$ | 1 | 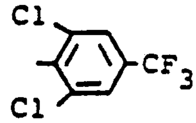 |
| H | $CF_3$ | $CH_3$ | 0 | 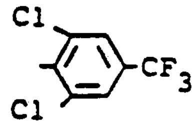 |
| H | $CCl_2F$ | $-CH_2CH_2-OH$ | 1 | 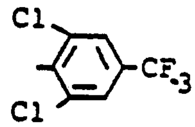 |
| H | $CF_3$ | H | 0 | 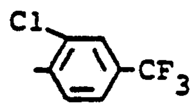 |
| H | $CF_3$ | H | 1 | 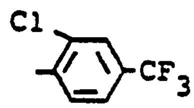 |
| H | $CF_3$ | H | 2 | 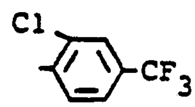 |
| $CH_3$ | $CF_3$ | H | 0 | 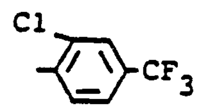 |
| $CH_3$ | $CF_3$ | H | 1 | 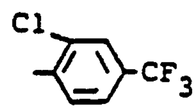 |
| $CH_3$ | $CF_3$ | H | 2 | 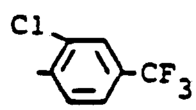 |
| $CH_3$ | $CClF_2$ | H | 0 | 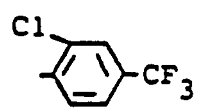 |
| H | $CClF_2$ | H | 0 | 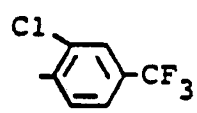 |
| $CF_3$ | $CCl_2F$ | H | 0 | 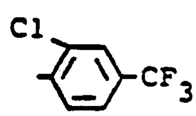 |

| $R^1$ | $R^2$ | $R^3$ | n | Ar |
|---|---|---|---|---|
| $C_2H_5$ | $CF_3$ | H | 0 | 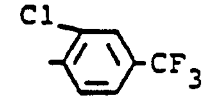 |
| H | $C_2H_5$ | H | 0 | 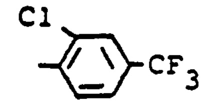 |
| H | $C_2H_5$ | H | 1 | 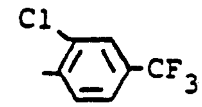 |
| H | $C_2H_5$ | H | 2 | 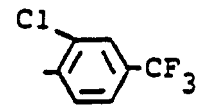 |
| H | $C_2H_5$ | $CH_3$ | 0 | 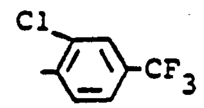 |

Die erfindungsgemäß zu verwendenden 5-Aminopyrazolderivate der Formel (I) sind teilweise bekannt (vgl. z.B. Pharmaco. Ed. Sci. *26,* 276—293 [1971] oder Mycopathologica *74,* 7—14 [1981] vgl. auch C.A. *96*: 196411j bzw. C.A. 95: 36257q) teilweise sind sie Gegenstand einer eigenen vorgängigen noch nicht publizierten Patentanmeldung (vgl. DE—P—3 402 308 vom 24.1.1984) und können in Analogie zu den dort beschriebenen Herstellungsverfahren erhalten werden. Noch nicht bekannt nach der oben angegebenen Literatur sind Verbindungen der Formel (I), bei welchen $R^1$ jeweils für Wasserstoff steht und

a) $R^2$ für gegebenenfalls substituiertes Benzyl steht (wobei $R^3$, n und Ar die oben angegebene Bedeutung haben) oder

b) Ar für unsubstituiertes oder einfach substituiertes Phenyl steht (wobei $R^2$, $R^3$ und n die oben angegebene Bedeutung haben) [mit Ausnahme der Verbindung: 5-Amino-4-(4-chlorphenylsulfonyl)-1-phenyl-pyrazol; vgl. Khim. Geterotsikl. Soedin. *1978,* 969—971 bzw. C.A. *89*: 179950x] oder

c) Ar für mehrfach substituiertes Phenyl steht, wobei jedoch nicht gleichzeitig die 2- und 4-Position substituiert sein dürfen (und $R^2$, $R^3$ und n die oben angegebene Bedeutung haben).

Diese Verbindungen können in Analogie zu den bekannten bzw. hier beschriebenen Verfahren hergestellt werden, (z.B. durch Umsetzung von substituierten Arylhydrazinen mit Acrylnitrilderivaten unter Ringschluß zu 1-Aryl-5-amino-pyrazolen und gegebenenfalls anschließende weitere Derivatisierung).

Außerdem noch nicht bekannt sind 5-Aminopyrrazole der Formel (Ia),

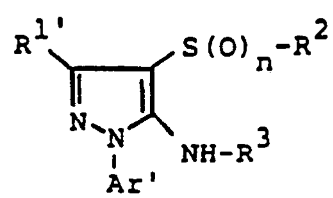

(Ia)

in welcher

$R^{1'}$ für jeweils geradkettiges oder verzweigtes Alkyl oder Halogenalkyl mit 1 bis 4 Kohlenstoffatomen und gegebenenfalls 1 bis 9 Halogenatomen steht,

Ar' für einfach oder mehrfach, gleich oder verschieden substituiertes Phenyl oder für gegebenenfalls einfach oder mehrfach, gleich oder verschieden substituiertes 2-Pyridyl, 3-Pyridyl oder 4-Pyridyl steht, wobei jeweils als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$—S(O)_m—R^5$$

wobei

$R^5$ für Amino, sowie für jeweils geradkettiges oder verzweigtes Alkyl, Alkylamino, Dialkylamino oder

Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

$R^2$ und $R^3$ die unter Formel (I) angegebene Bedeutung besitzen und m für eine Zahl 0, 1 oder 2 steht, und

n für eine Zahl 0, 1 oder 2 steht,

wobei $R^2$ nur dann für s-Butyl steht, wenn nicht gleichzeitig $R^1$ für Methyl, $R^3$ für Wasserstoff, n für 0 und Ar für einfach oder zweifach durch Chlor substituiertes Phenyl steht.

Ebenfalls noch nicht bekannt sind 5-Amino-pyrazole der Formel (Ib),

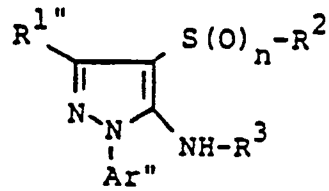

(Ib)

in welcher

$R^{1''}$ für Wasserstoff steht,

Ar'' für gegebenenfalls einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder halogenalkoxy mit jeweils bis zu 4 Kohlenstoffatomen und bis zu 9 gleichen oder verschiedenen Halogenatomen oder ein Rest

$$—S(O)_m—R^5$$

wobei

$R^5$ für Amino sowie für jeweils geradkettiges Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

m für eine ganze Zahl 0, 1 oder 2 steht,

n für eine ganze Zahl 0, 1 oder 2 steht

und $R^2$ und $R^3$ die unter Formel (I) angegebene Bedeutung besitzen.

Man erhält die noch nicht bekannten Aminopyrazole der Formel (Ia) beispielsweise, wenn man

(a) (α) 4-Thiocyanato-5-aminopyrazole der allgemeinen Formel (IIa),

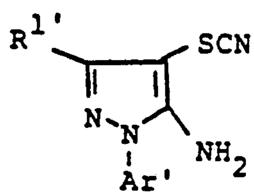

(IIa)

in welcher

$R^{1'}$ und Ar' die oben angegebene Bedeutung haben, oder

(a) (β) Bis-(pyrazolyl)-disulfide der Formel (IIIa),

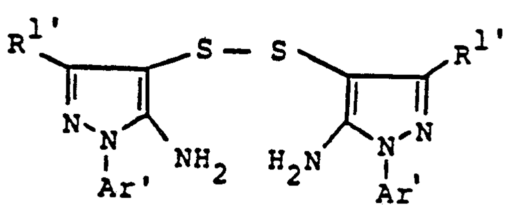

(IIIa)

in welcher

$R^{1'}$ und Ar' die oben angegebene Bedeutung haben,

mit Halogeniden der Formel (IV),

$$R^2—Hal \quad (IV)$$

in welcher

$R^2$ die oben angegebene Bedeutung hat und

Hal für Halogen steht,

gegebenenfalls in Gegenwart eines Verdünnungsmittels sowie in Gegenwart eines geeigneten Reduktionsmittels und in Gegegenwart einer Base umsetzt,

oder wenn man

(b) 4-unsubstituierte 5-Amino-pyrazole der Formel (Va),

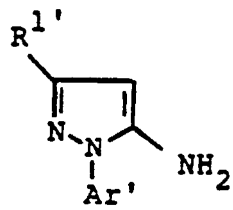

(Va)

in welcher

$R^{1'}$ und Ar' die oben angegebenen Bedeutungen haben,

mit Sulfenylhalogeniden der Formel (VI),

$$R^2—S—Hal' \qquad (VI)$$

in welcher

$R^2$ die oben angegebene Bedeutung hat und

Hal' für Halogen steht,

gegebenenfalls in Gegenwart eines Verdünnungsmittels und gegebenenfalls in Gegenwart eines Säurebindemittels umsetzt,

oder wenn man

(c) die nach Verfahren (a-α), (a-β) oder (b) erhältlichen 4-substituierten 5-Amino-pyrazole der Formel (Ic),

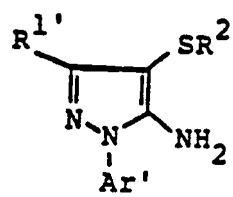

(Ic)

in welcher

$R^{1'}$, $R^2$ und Ar die oben angegebene Bedeutung haben

in allgemein üblicher Weise mit Acylierungsmitteln oder Alkylierungsmitteln der Formel (VII)

$$R^6—A \qquad (VII)$$

in welcher

$R^6$ für Alkyl oder einen Rest

$$—\overset{\overset{\displaystyle X}{\|}}{C}—R^4$$

steht, wobei X und $R^4$ die oben angegebene Bedeutung haben und

A für eine elektronenziehende Abgangsgruppe steht,

oder mit Iso(thio)cyanaten der Formel (VIII)

$$R^7—N=C=X \qquad (VIII)$$

in welcher

$R^7$ für Alkyl oder für ein gegebenenfalls substituiertes Aryl steht, und

X die oben angegebene Bedeutung hat,

gegebenenfalls in Gegenwart eines Verdünnungsmittels und gegebenenfalls in Gegenwart eines Säurebindemittels am Stickstoff der Aminogruppe in 5-Stellung des Pyrazolringes alkyliert bzw. acyliert,

oder wenn man

(d) die nach Verfahren (a-α), (a-β), (b) oder (c) erhältlichen 5-Aminopyrazole der Formel (Id),

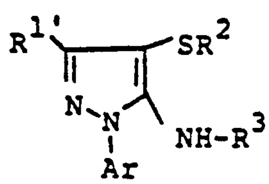

(Id)

in welcher

$R^{1'}$, $R^2$, $R^3$ und Ar' die oben angegebene Bedeutung haben,

mit Oxidationsmitteln der Formel (IX),

$$R—O—OH \qquad (IX)$$

in welcher

R für Wasserstoff oder für jeweils gegebenenfalls substituiertes Alkanoyl oder Aroyl steht,

gegebenenfalls in Gegenwart eines Verdünnungsmittels, gegebenenfalls in Gegenwart eines Katalysators sowie gegebenenfalls in Gegenwart eines Säurebindemittels am Schwefel der Sulfenylgruppe in 4-Position des Pyrazolringes oxidiert.

Analog erhält man die noch nicht bekannten 5-Aminopyrazole der Formel (Ib) beispielsweise, wenn man

(e-α) 4-Thiocyanato-5-aminopyrazole der allgemeinen Formel (IIb),

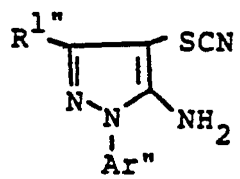

(IIb)

in welcher

$R^{1''}$ und Ar'' die oben angegebene Bedeutung haben,

oder

(e-β) Bis-(pyrazolyl)-disulfide der Formel (IIIb),

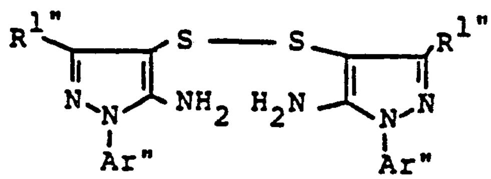

(IIIb)

in welcher

$R^{1''}$ und Ar'' die oben angegebene Bedeutung haben,

mit Halogeniden der Formel (IV),

$$R^2—Hal \qquad (IV)$$

in welcher

$R^2$ die oben angegebene Bedeutung hat und

Hal für Halogen steht,

gegebenenfalls in Gegenwart eines Verdünnungsmittels sowie in Gegenwart eines geeigneten Reduktionsmittels und in Gegenwart einer Base umsetzt,

oder wenn man

(f) 4-unsubstituierte 5-Amino-pyrazole der Formel (Vb),

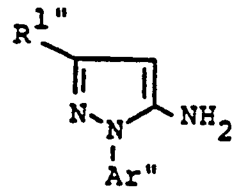

(Vb)

in welcher

$R^{1''}$ und Ar'' die oben angegebene Bedeutung haben,

mit Sulfenylhalogeniden der Formel (VI),

$$R^2—S—Hal' \qquad (VI)$$

in welcher

$R^2$ die oben angegebene Bedeutung hat und

Hal' für Halogen steht,

gegebenenfalls in Gegenwart eines Verdünnungsmittels und gegebenenfalls in Gegenwart eines Säurebindemittels umsetzt,

oder wenn man

(g) die nach Verfahren (e-α), (e-β) oder (f) erhältlichen 4-substituierten 5-Amino-pyrazole der Formel (Ie),

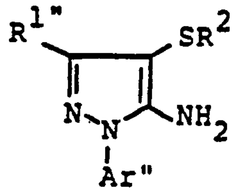

(Ie)

in welcher

$R^{1''}$, $R^2$ und Ar'' die oben angegebene Bedeutung haben,

in allgemein üblicher Weise mit Acylierungsmitteln oder Alkylierungsmitteln der Formel (VII)

$$R^6—A \qquad (VII)$$

in welcher

$R^6$ für Alkyl oder einen Rest

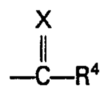

steht, wobei X und $R^4$ die oben angegebene Bedeutung haben und

A für eine elektronenziehende Abgangsgruppe steht,

oder mit Iso(thio)cyanaten der Formel (VIII)

$$R^7—N{=}C{=}X \qquad (VIII)$$

in welcher

$R^7$ für Alkyl oder für gegebenenfalls substituiertes Aryl steht, und

X die oben angegebene Bedeutung hat,

gegebenenfalls in Gegenwart eines Verdünnungsmittels und gegebenenfalls in Gegenwart eines Säurebindemittels am Stickstoff der Aminogruppe in 5-Stellung des Pyrazolringes alkyliert bzw. acyliert,

oder wenn man

(h) die nach Verfahren (e-α), (e-β), (f) oder (g) erhältlichen 5-Aminopyrazole der Formel (If),

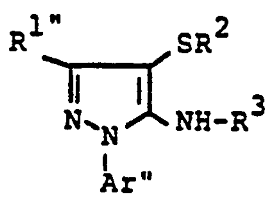

(If)

in welcher

$R^{1''}$, $R^2$, $R^3$ und Ar'' die oben angegebene Bedeutung haben,

mit Oxidationsmitteln der Formel (IX),

$$R—O—OH \qquad (IX)$$

in welcher

R für Wasserstoff oder für jeweils gegebenenfalls substituiertes Alkanoyl oder Aroyl steht,

gegebenenfalls in Gegenwart eines Verdünnungsmittels, gegebenenfalls in Gegenwart eines Katalysators sowie gegebenenfalls in Gegenwart eines Säurebindemittels am Schwefel der Sulfenylgruppe in 4-Position des Pyrazolringes oxidiert.

Verwendet man beispielsweise [5-Amino-1-(2,4-dichlorphenyl)-3-methyl-pyrazol-4-yl]-thiocyanat und Methyliodid als Ausgangsstoffe und Natriumborhydrid als Reduktionsmittel, so läßt sich der Reaktionsablauf des Herstellungsverfahrens (a-α) durch das folgende Formelschema darstellen:

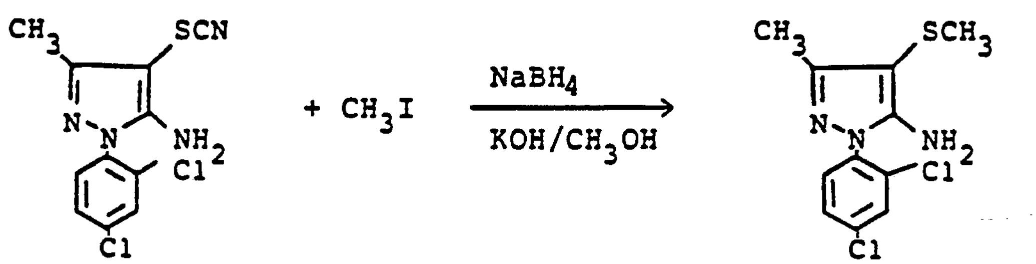

Verwendet man beispielsweise S,S'-Bis-[5-Amino-1-(2,6-dichlor-4-trifluormethylphenyl)-3-methyl-pyrazol-4-yl]-disulfid und 4-Chlorbenzylbromid als Ausgangsstoffe, sowie Natriumdithionit als Reduktionsmittel, so läßt sich der Reaktionsablauf des Herstellungsverfahrens (a-β) durch das folgende Formelschema darstellen:

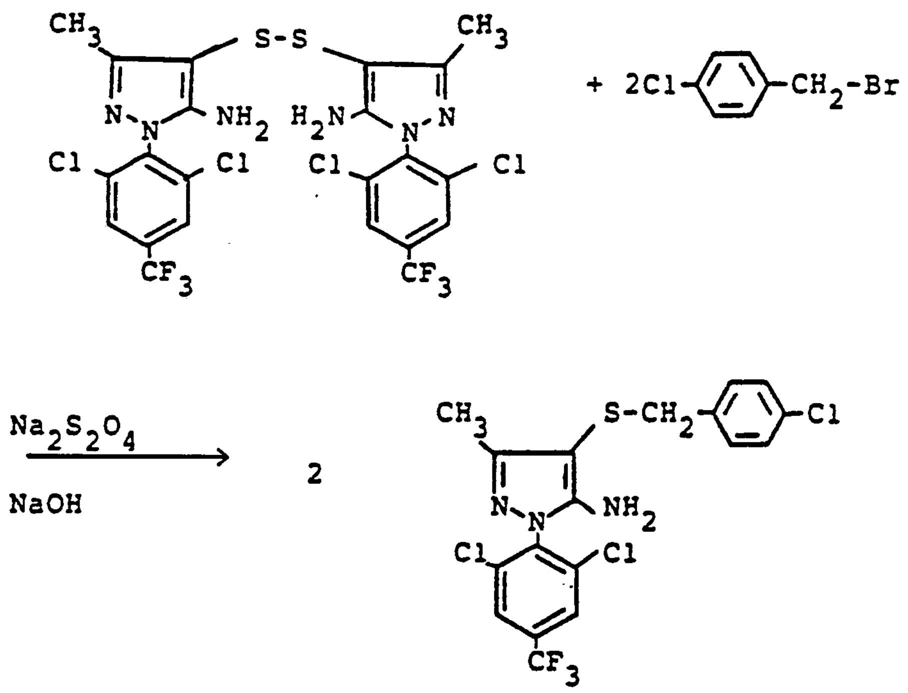


Verwendet man beispielsweise 5-Amino-3-methyl-1-(2,4,6-trichlorphenyl)-pyrazol und Dichlorfluormethansulfenylchlorid als Ausgangsstoffe, so läßt sich der Reaktionsablauf des Herstellungsverfahrens (b) durch das folgende Formelschema darstellen:

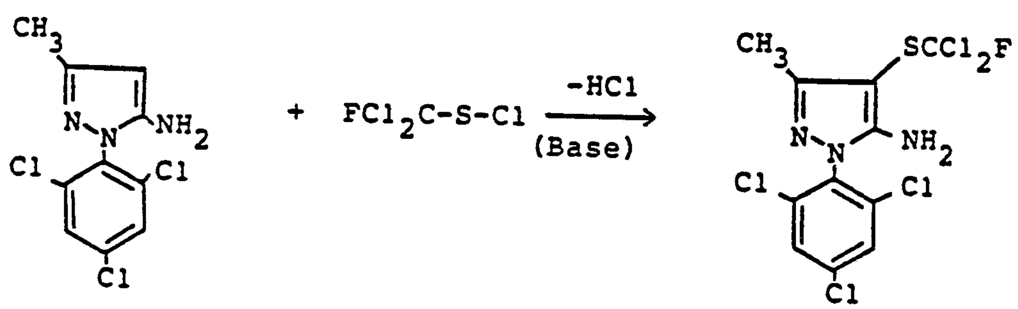


Verwendet man beispielsweise 5-Amino-3-methyl-4-trifluormethylsulfenyl-1-(3,5-dichlorpyrid-2-yl)-pyrazol und Propionylchlorid als Ausgangsstoffe, so läßt sich der Reaktionsablauf des Herstellungsverfahrens (c) durch das folgende Formelschema darstellen:

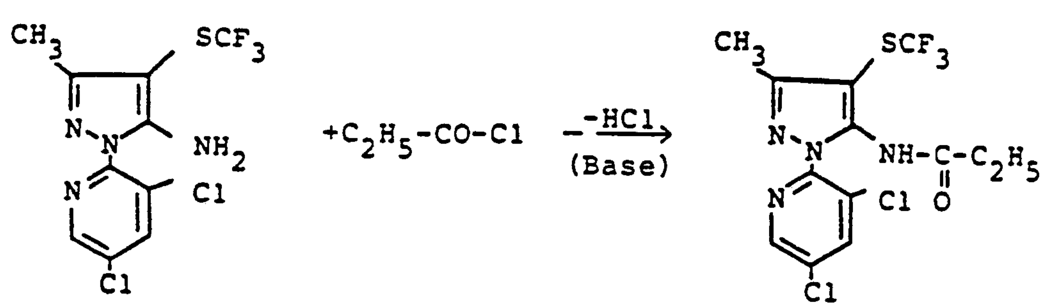


Verwendet man beispielsweise 4-Methyl-3-methylthio-1-(2,6-dichlor-4-trifluoromethylphenyl)-5-propionamido-pyrazol und m-Chlorperbenzoesäure als Ausgangsstoffe, so läßt sich der Reaktionsablauf des

Herstellungsverfahrens (d) durch das folgende Formelschema darstellen:

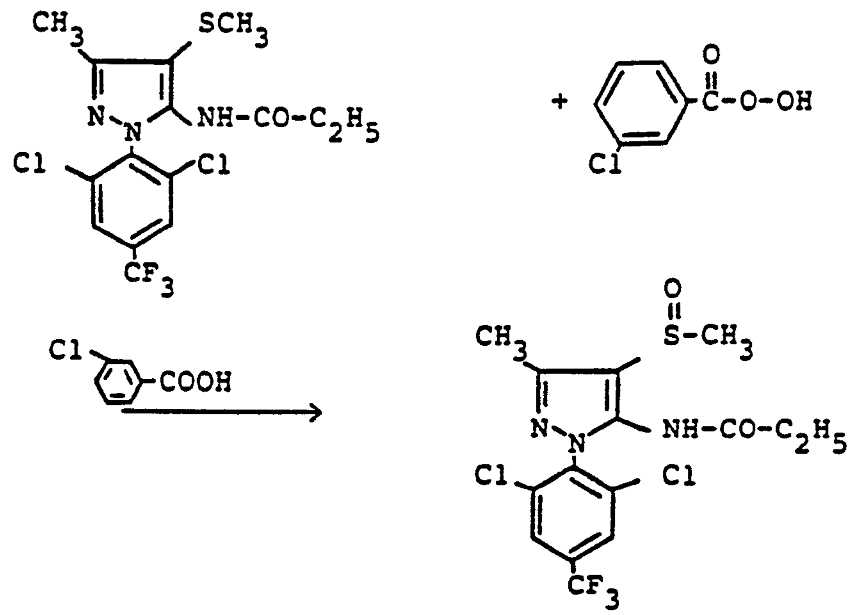

Verwendet man beispielsweise [(5-Amino-1-pyrid-2-yl)-pyrazol-4-yl]-thiocyanat und Methyliodid als Ausgangsstoffe und Natriumborhydrid als Reduktionsmittel, so läßt sich der Reaktionsablauf des Herstellungsverfahrens (e-α) durch das folgende Formelschema darstellen:

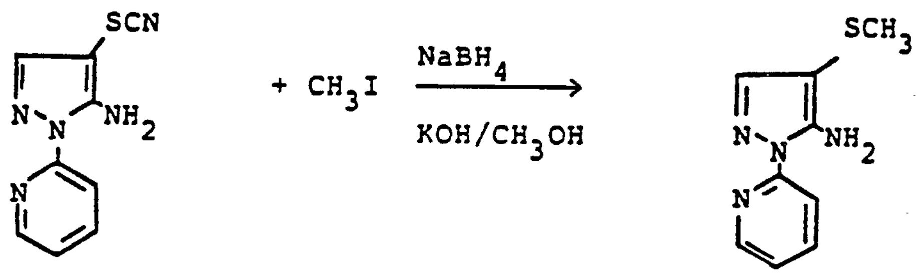


Verwendet man beispielsweise S,S'-Bis-[5-Amino-1-(3-chlor-5-trifluormethylthio-pyrid-2-yl)-pyrazol-4-yl]-disulfid und 4-Fluorbenzylbromid als Ausgangsstoffe, sowie Natriumdithionit als Reduktionsmittel, so läßt sich der Reaktionsablauf des Herstellungsverfahrens (e-β) durch das folgende Formelschema darstellen:

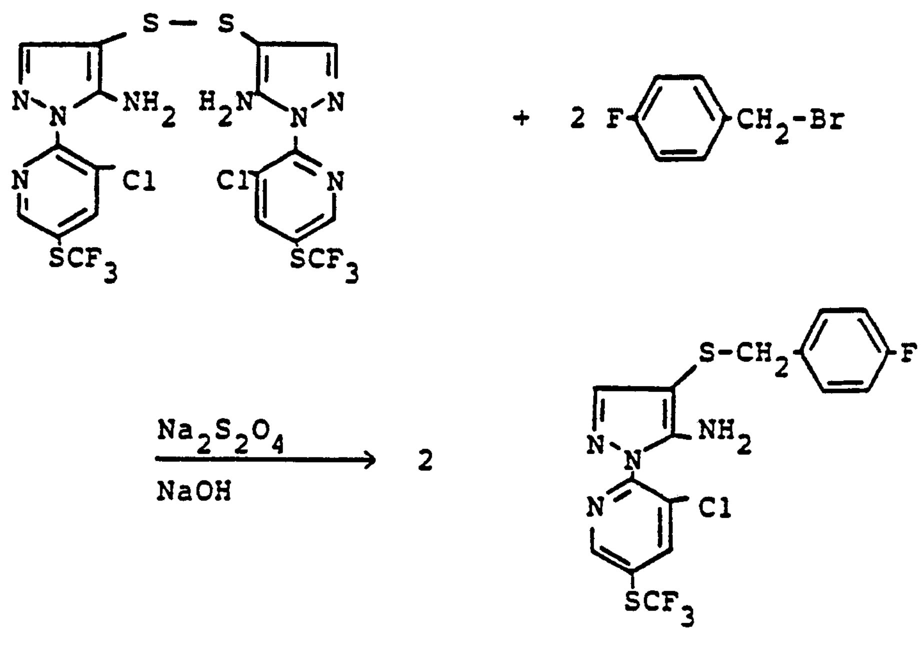

Verwendet man beispielsweise 5-Amino-1-(5-chlor-pyrid-2-yl)-pyrazol und Trichlormethylsulfenylchlorid als Ausgangsstoffe, so läßt sich der Reaktionsablauf des Herstellungsverfahrens (f) durch das folgende Formelschema darstellen:

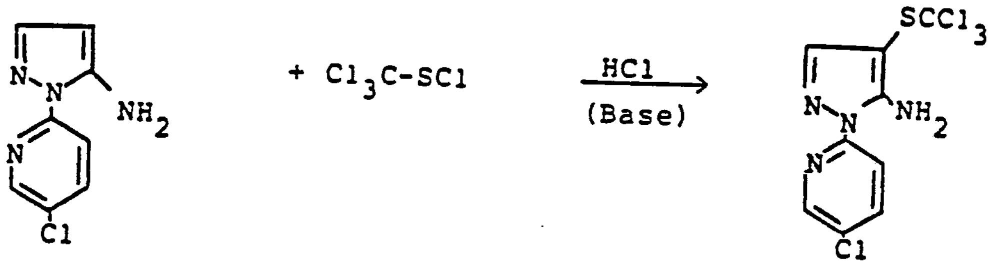


Verwendet man beispielsweise 5-Amino-4-ethylthio-1-pyrid-4-yl-pyrazol und Propionylchlorid als Ausgangsstoffe, so läßt sich der Reaktionsablauf des Herstellungsverfahrens (g) durch das folgende Formelschema darstellen:

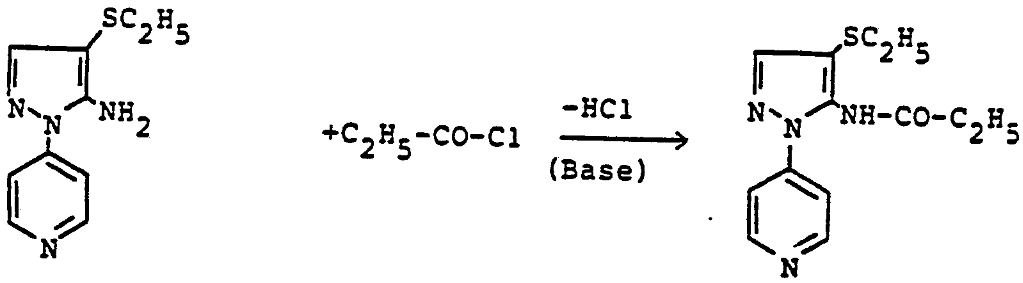


Verwendet man beispielsweise 4-Ethylthio-1-(3,5-dichlorpyrid-4-yl)-5-propionamido-pyrazol und Wasserstoffperoxid als Ausgangsstoffe, so läßt sich der Reaktionsablauf des Herstellungsverfahrens (h) durch das folgende Formelschema darstellen:

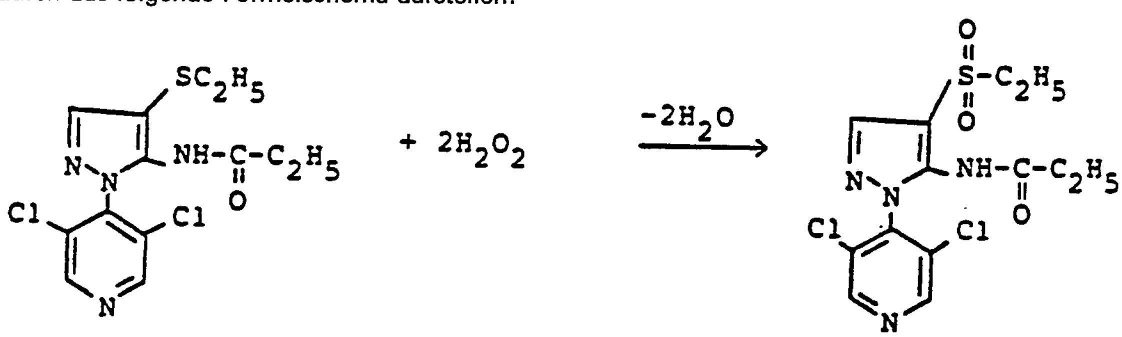


Die zur Durchführung der Herstellungsverfahren (a-α) und (e-α) als Ausgangsstoffe benötigten 4-Thiocyanato-5-aminopyrazole sind durch die Formeln (IIa) bzw. (IIb) allgemein definiert.

Die 4-Thiocyanato-5-aminopyrazole der Formeln (IIa) bzw. (IIb) sind teilweise bekannt (vgl. z.B. Farmaco Ed. Sci. *38*, 274—282 [1983]). Man erhält sie beispielsweise, wenn man 4-unsubstituierte 5-Aminopyrazole der Formel (V),

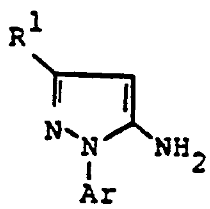

(V)

in welcher

$R^1$ für Wasserstoff, Alkyl oder Halogenalkyl steht und

Ar für substituiertes Phenyl oder für jeweils gegebenenfalls substituiertes 2-Pyridyl, 3-Pyridyl oder 4-Pyridyl steht,

mit Ammoniumthiocyanat in Gegenwart von Brom und Essigsäure bei Temperaturen zwischen −20°C und +20°C umsetzt.

Die zur Durchführung des Herstellungsverfahrens (a-β) und (e-β) als Ausgangsstoffe benötigten Bis-(pyrazolyl)-disulfide sind durch die Formeln (IIIa) bzw. (IIIb) allgemein definiert. In den Formeln (IIIa) und (IIIb) stehen die Reste $R^{1'}$ und $R^{1''}$ sowie Ar′ und Ar″ vorzugsweise für diejenigen Reste, die bereits im

Zusammenhang mit der Beschreibung der Vorprodukte der Formeln (IIa) bzw. (IIb) als bevorzugt für diese Substituenten genannt wurden.

Die Bis-(pyrazolyl)-disulfide der Formeln (IIIa) bzw. (IIIb) sind noch nicht bekannt. Man erhält sie, wenn man die oben beschriebenen 4-Thiocyanato-5-amino-pyrazole der Formeln (IIa) bzw. (IIb) mit wässriger Salzsäure, gegebenenfalls in Gegenwart eines Verdünnungsmittels, wie beispielsweise Ethanol bei Temperaturen zwischen 20°C und 120°C umsetzt.

Die zur Durchführung der Herstellungsverfahren (a-α), (a-β), (e-α) und (e-β) weiterhin als Ausgangsstoffe erforderlichen Halogenide sind durch die Formel (IV) allgemein definiert. In dieser Formel (IV) steht $R^2$ vorzugsweise für diejenigen Substituenten, die bereits im Zusammenhang mit der Beschreibung der erfindungsgemäß verwendbaren 5-Aminopyrazolderivate der Formel (I) als bevorzugt für diesen Rest genannte wurden. Hal steht vorzugsweise für Chlor, Brom oder Iod. Die Halogenide der Formel (IV) sind allgemein bekannte Verbindungen der organischen Chemie.

Die zur Durchführung der Herstellungsverfahren (b) und (f) und die zur Synthese der Vorprodukte der Formeln (IIa) und (IIb) als Ausgangsstoffe benötigten 4-unsubstituierten 5-Aminopyrazole sind durch die Formeln (Va) und (Vb) allgemein definiert. In den Formeln (Va) und (Vb) stehen die Reste $R^{1'}$ und $R^{1''}$ sowie Ar' und Ar'' vorzugsweise für diejenigen Reste, die bereits im Zusammenhang mit der Beschreibung der Vorprodukte der Formeln (IIa) und (IIb) als bevorzugt für diese Substituenten genannt wurden.

Die 4-Unsubstituierten 5-Aminopyrazole der Formeln (Va) und (Vb) sind teilweise bekannt (vgl. z.B. J. Org. Chem. *36*, 2972—2974 [1971] oder J. Heterocyclic Chemistry *7*, 345—349 [1970]; C.A. *62*: 13137c).

Man erhält sie beispielsweise, wenn man Arylhydrazine der Formel (X),

$$Ar'''—NH—NH_2 \qquad (X)$$

in welcher

Ar''' für substituiertes Phenyl oder für jeweils gegebenenfalls substituiertes 2-Pyridyl, 3-Pyridyl oder 4-Pyridyl steht,

mit Acrylnitrilderivaten der Formel (XI),

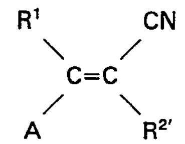

(XI)

in welcher

$R^1$ für Wasserstoff, Alkyl oder Halogenalkyl steht,

$R^{2'}$ für Wasserstoff oder Alkoxycarbonyl steht und

A für Halogen, Hydroxy, Alkoxy, Amino oder Dialkylamino steht,

entweder zunächst in einer 1. Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen −20°C und +20°C umsetzt zu den Arylhydrazinderivaten der Formel (XII),

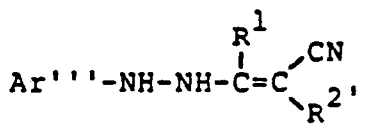

(XII)

in welcher

Ar''', $R^1$ und $R^{2'}$ die oben angegebene Bedeutung haben, und diese in einer 2. Stufe, gegebenenfalls in Gegenwart eines Verdünnungsmittels wie beispielsweise Ethylenglykolmonoethylether bei Temperaturen zwischen +50°C und +150°C cyclisiert, oder in einem Reaktionsschritt ohne Isolierung der Zwischenstufe der Formel (XII), gegebenenfalls in Gegenwart eines Verdünnungsmittels wie beispielsweise Ethylenglykolmonoethylether bei Temperaturen zwischen +50°C und + 150°C direkt cyclisiert zu den 5-Aminopyrazolen der Formel (XIII)

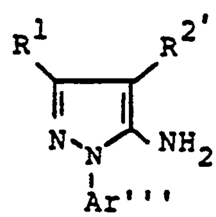

(XIII)

in welcher

$R^1$, $R^{2'}$ und Ar''' die oben angegebene Bedeutung haben, und in dem Fall, wo $R^{2'}$ für Alkoxycarbonyl steht, die Verbindungen der Formel (XIII) in allgemein üblicher Art und Weise, gegebenenfalls in

Gegenwart eines Verdünnungsmittels, wie beispielsweise Ethanol oder Isopropanol sowie gegebenenfalls in Gegenwart eines Katalysators, wie beispielsweise Bromwasserstoffsäure bei Temperaturen zwischen 50°C und 150°C verseift und decarboxyliert.

Die Arylhydrazine der Formel (X) sind bekannt (vgl. z.B. US—PS 4 127 575; US—PS 3 609 158; DE—OS 2 558 399; J. Chem. Soc. C, *1971,* 167—174) oder lassen sich nach prinzipiell bekannten Verfahren in einfacher analoger Weise herstellen (vgl. z.B. Houben-Weyl "Methoden der organischen Chemie" Band X, 2 S.203, Thieme Verlag Stuttgart 1967).

Die Acrylnitrilderivate der Formel (XI) sind allgemein bekannte Verbindungen der organischen Chemie.

Die zur Durchführung der Herstellungsverfahren (b) und (f) weiterhin als Ausgangsstoffe benötigten Sulfenylhalogenide sind durch die Formel (VI) allgemein definiert. In dieser Formel (VI) steht $R^2$ vorzugsweise für diejenigen Substituenten, die bereits im Zusammenhang mit der Beschreibung der erfindungsgemäß verwendbaren Stoffe der Formel (I) als bevorzugt für diesen Rest genannt wurden. Hal' steht vorzugsweise für Fluor, Chlor, Brom oder Iod.

Die Sulfenylhalogenide der Formel (VI) sind allgemein bekannte Verbindungen der organischen Chemie.

Die zur Durchführung der Herstellungsverfahren (c) und (g) als Ausgangsstoffe benötigen 4-substituierten 5-Aminopyrazole sind durch die Formeln (Ic) und (Ie) allgemein definiert. In diesen Formeln (Ic) und (Ie) stehen $R^{1'}$ und $R^{1''}$ sowie Ar' und Ar'' vorzugsweise für diejenigen Reste, die bereits im Zusammenhang mit der Beschreibung der Vorprodukte der Formeln (IIa) und (IIb) als bevorzugt für diese Substituenten genannt wurden. $R^2$ steht vorzugsweise für diejenigen Reste, die bereits in Zusammenhang mit der Beschreibung der erfindungsgemäß verwendbaren Stoffe der Formel (I) als bevorzugt für diesen Rest genannt wurden.

Die 4-substituierten 5-Aminopyrazole der Formeln (Ic) und (Ie) sind erfindungsgemäße Verbindungen und erhältlich mit Hilfe der Herstellungsverfahren (a-α), (a-β) oder (b) bzw. (e-α, (e-β) oder (f).

Die zur Durchführung der Herstellungsverfahren (c) und (g) weiterhin als Ausgangsstoffe benötigten Acylierungs- oder Alkylierungsmittel sind durch die Formel (VII) allgemein definiert. In dieser Formel (VII) steht $R^6$ vorzugsweise für geradkettiges oder verzweigtes Alkyl mit bis zu 4 Kohlenstoffatomen und außerdem für einen Rest

$$-\underset{\underset{X}{\|}}{C}-R^4,$$

wobei X und $R^4$ vorzugsweise für diejenigen Reste stehen, die schon bei der Beschreibung der erfindungsgemäß verwendbaren Stoffe der Formel (I) für diese Reste genannt wurden.

A steht vorzugsweise für Chlor, Brom, Iod, p-Toluolsulfonyloxy, Alkoxysulfonyloxy oder Acyloxy. Die Alkylierungs- und Acylierungsmittel der Formel (VII) sind allgemein bekannte Verbindungen der organischen Chemie.

Die alternativ zur Durchführung der Herstellungsverfahren (c) und (g) als Ausgangsstoffe verwendbaren Iso(thio)cyanate sind durch die Formel (VIII) allgemein definiert. In dieser Formel steht X vorzugsweise für Sauerstoff oder Schwefel und $R^7$ steht vorzugsweise für geradkettiges oder verzweigtes Alkyl mit bis zu 4 Kohlenstoffatomen, oder für gegebenenfalls ein- bis dreifach, gleich oder verschieden substituiertes Phenyl, wobei als Substituenten in Frage kommen: Halogen, jeweils geradkettiges oder verzweigtes Alkyl, Alkoxy oder Halogenalkyl mit jewiels bis zu 4 Kohlenstoffatomen und im Fall des Halogenalkyl mit bis zu 9 gleichen oder verschiedenen Halogenatomen.

$R^7$ steht insbesondere für Methyl, Ethyl und gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VIII) sind ebenfalls allgemein bekannte Verbindungen der organischen Chemie.

Die zur Durchführung der Herstellungsverfahren (d) und (h) als Ausgangsstoffe benötigten 5-Aminopyrazole sind durch die Formeln (Id) bzw. (If) allgemein definiert. In diesen Formeln (Id) und (If) stehen $R^{1'}$ und $R^{1''}$ sowie Ar' und Ar'' vorzugsweise für diejenigen Reste, die bereits im Zusammenhang mit der Beschreibung der Vorprodukte der Formeln (IIa) und (IIb) als bevorzugt für diese Substituenten genannt wurden. $R^2$ und $R^3$ stehen vorzugsweise für diejenigen Reste, die bereits im Zusammenhang mit der Beschreibung der erfindungsgemäß verwendbaren Stoffe der Formel (I) als bevorzugt für diese Reste genannt wurden.

Die 5-Aminopyrazole der Formeln (Id) und (If) sind erfindungsgemäße Verbindungen und erhältlich mit Hilfe der Herstellungsverfahren (a-α), (a-β), (b) oder (c) bzw. (e-α), (e-β), (f) oder (g).

Die zur Durchführung der Herstellungsverfahren (d) und (h) weiterhin als Ausgangsstoffe benötigen Oxidationsmittel sind durch die Formel (IX) allgemein definiert. In dieser Formel (IX) steht R vorzugsweise für Wasserstoff, für Acetyl, für Propionyl, für Trifluoracetyl oder für gegebenenfalls substituiertes Benzoyl, wie beispielsweise 3-Chlorbenzoyl oder 4-Nitrobenzoyl. Die Oxidationsmittel der Formel (IX) sind allgemein bekannte Verbindungen der organischen Chemie.

Als Verdünnungsmittel zur Durchführung der Herstellungsverfahren (a-α) und (a-β) sowie (e-α) und (e-β) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorokohlenstoff, Ether, wie Diethylether, Dioxan, Tetrahydrofuran oder Ethylenglykoldimethyl- oder diethylether oder Alkohole, wie Methanol, Ethanol oder Propanol.

Mit besonderem Vorteil verwendet man als Verdünnungsmittel den im Alkylrest dem Halogenid der Formel (IV) entsprechenden Alkohol, d.h. beispielsweise bei Verwendung von Methyliodid als Halogenid der Formel (IV) kommt mit besonderem Vorzug Methanol als Verdünnungsmittel in Frage.

Als Reduktionsmittel zur Durchführung der Herstellungsverfahren (a-α) und (e-α) verwendet man vorzugsweise komplexe Hydride wie Lithiumaluminiunhydrid, Lithiumborhydrid oder Natriumborhydrid. Besonders geeignet ist Natriumborhydrid.

Als Reductionsmittel zur Durchführung der Herstellungsverfahren (a-β) und (e-β) kommen alle üblicherweise für Disulfidspaltungen verwendbaren Reduktionsmittel in Frage. Mit besonderem Vorzug verwendet man Dithionite, wie beispielsweise Natriumdithionit.

Die Herstellungsverfahren (a-α), (a-β), (e-α) und (e-β) werden in Gegenwart einer geeigneten Base durchgeführt. Vorzugsweise verwendet man Alkalimetallhydroxide oder-carbonate, wie beispielsweise Natrium- oder Kaliumhydroxid oder Natrium- oder Kaliumcarbonat.

Die Reaktionstemperaturen können bei der Durchführung der Herstellungsverfahren (a-α), (a-β), (e-α) und (e-β) in einem größeren Bereich variiert werden. Im allgemeinen arbeitet man bei Temperaturen zwischen 0°C und +120°C, vorzugsweise bei Temperaturen zwischen +20°C und +90°C.

Zur Durchführung der Herstellungsverfahren (a-α) bzw. (e-α) setzt man pro Mol an 4-Thiocyanato-5-aminopyrazol der Formel (IIa) bzw. (IIb) im allgemeinen 1,0 bis 5,0 Mol, vorzugsweise 1,8 bis 2,5 Mol an Halogenid der Formel (IV) und 1,0 bis 5,0 Mol, vorzugsweise 1,8 bis 2,0 Mol an Reduktionsmittel sowie 1,0 bis 5,0 Mol, vorzugsweise 1,5 bis 3,0 Mol an Base ein. Dabei setzt man zunächst das 4-Thiocyanato-5-aminopyrazol der Formel (IIa) bzw. (IIb) in dem betreffenden Verdünnungsmittel unter Verwendung einer Stickstoff-Schutzgasatmosphäre mit dem Reduktionsmittel um und setzt nach beendeter Reaktion die Base und das Halogenid der Formel (IV) zu. Die Aufarbeitung und Isolierung der Reaktionsprodukte der Formel (Ia) bzw. (Ib) erfolgt nach üblichen Verfahren.

Zur Durchführung der Herstellungsverfahren (a-β) bzw. (e-β) setzt man pro Mol an Bis-(pyrazolyl)-disulfid der Formel (IIIa) bzw. (IIIb) im allgemeinen 1,0 bis 5,0 Mol, vorzugsweise 1,8 bis 2,5 Mol an Halogenid der Formel (IV) und 1,0 bis 5,0 Mol, vorzugsweise 1,8 bis 2,0 Mol an Reduktionsmittel sowie 1,0 bis 5,0 Mol, vorzugsweise 1,5 bis 3.0 Mol an Base ein. Dabei setzt man zunächst das Bis-(pyrazolyl)-disulfid der Formel (IIIa) bzw. (IIIb) in dem betreffenden Verdünnungsmittel in Gegenwart der Base bei der entsprechenden Reaktionstemperatur mit dem Reduktionsmittel um, setzt nach einigen Stunden das Halogenid der Formel (IV) zu und erwärmt eine weitere Stunde auf die erforderliche Reaktionstemperatur. Die Aufarbeitung und Isolierung der Reaktionsprodukte der Formeln (Ia) bzw. (Ib) erfolgt nach üblichen Verfahren.

Als Verdünnungsmittel zur Durchführung der Herstellungsverfahren (b) und (f) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die Herstellungsverfahren (b) und (f) werden gegebenenfalls in Gegenwart eines Säurebindemittels durchgeführt. As solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der Herstellungsverfahren (b) und (f) in einem größeren Bereich variiert werden. Im allgemeinen arbeitet man bei Temperaturen zwischen −20°C und +120°C, vorzugsweise bei Temperaturen zwischen 0°C und +50°C.

Zur Durchführung der Herstellungsverfahren (b) und (f) setzt man pro Mol an 4-unsubstituierten 5-Amino-pyrazol der Formel (Va) bzw. (Vb) im allgemeinen 1,0 bis 2,5 Mol, vorzugsweise 1,0 bis 1,5 Mol an Sulfenylhalogenid und 1,0 bis 2,5 Mol, vorzugsweise 1,0 bis 1,5 Mol an Säurebindemittel ein. Die Reaktionsführung, Aufarbeitung und Isolierung der Reaktionsprodukte der Formel (Ia) und (Ib) erfolgt nach allgemein üblichen Verfahren.

Als Verdünnungsmittel zur Durchführung der Herstellungsverfahren (c) und (g) kommen ebenfalls inerte orgasnische Lösungsmittel in Frage. Vorzugsweise verwendet man die bei der Beschreibung der Herstellungsverfahren (b) und (f) genannten organischen Lösungsmittel als Verdünnungsmittel.

Die Herstellungsverfahren (c) und (g) werden gegebenenfalls in Gegenwart eines Säurebindemittels durchgeführt. Als solche kommen insbesondere die bei der Beschreibung der Herstellungsverfahren (b)

und (f) genannten organischen und anorganischen Basen in Frage.

Die Reaktionstemperaturen können bei der Durchführung der Herstellungsverfahren (c) und (g) in einem größeren Bereich variiert werden. Im allgemeinen arbeitet man bei Temperaturen zwischen −20°C und +80°C, vorzugsweise bei Temperaturen zwischen −10°C und +40°C.

Zur Durchführung der Herstellungsverfahren (c) und (g) setzt man pro Mol an 4-substituierten 5-Amino-pyrazol der Formel (Ic) bzw. (Ie) im allgemeinen 1,0 bis 10,0 Mol, vorzugsweise 1,0 bis 5,0 Mol an Acylierungs- oder Alkylierungsmittel der Formel (VII) bzw. Iso(thio)cyanat der Formel (VIII] und gegebenenfalls und 1,0 bis 5,0 Mol an Säurebindemittel ein. Die Reaktionsführung, Aufarbeitung und Isolierung der Reaktionsprodukte der Formeln (Ia) bzw. (Ib) erfolgt nach allgemein bekannten, üblichen Verfahren.

Als Verdünnungsmittel zur Durchführung der Herstellungsverfahren (d) und (h) kommen ebenfalls inerte organische Lösungsmittel in Frage.

Vorzugsweise verwendet man Kohlenwasserstoffe, wie Benzin, Benzol, Toluol, Hexan oder Petrolether, chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformid.

Die Herstellungsverfahren (d) und (h) können gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Die Herstellungsverfahren (d) und (h) können gegebenenfalls in Gegenwart eines geeigneten Katalysators durchgeführt werden. Als solche kommen alle üblicherweise für derartige Schwefeloxidationen verwendbaren üblichen Katalysatoren in Frage. Beispielhaft genannt sei in diesem Zusammenhang Ammoniummolydbat.

Die Reaktionstemperaturen können bei der Durchführung der Herstellungsverfahren (d) und (h) in einem größeren Bereich variiert werden. Im allgemeinen arbeitet man bei Temperaturen zwischen −20°C und +70°C, vorzugsweise bei Temperaturen zwischen 0°C und +50°C.

Zur Durchführung der Herstellungsverfahren (d) und (h) setzt man pro Mol an 5-Aminopyrazol der Formel (Id) bzw. (If) im allgemeinen 0.8 bis 1,2 Mol, vorzugsweise äquimolare Mengen Oxidationsmittel der Formel (IX) ein, wenn man die Oxidation des Schwefels auf der Sulfoxidstufe unterbrechen will. Zur Oxidation zum Sulfon setzt man pro Mol an 5-Aminopyrazol der Formel (Id) bzw. (If) im allgemeinen 1,8 bis 3,0 Mol, vorzugsweise doppelt molare Mengen an Oxidationsmittel der Formel (IX) ein. Die Reaktionsführung, Aufarbeitung und Isolierung der Endprodukte der Formel (Ia) bzw. (Ib) erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon hymuli, Rhopalosiphum padi, Empoasca spp., Euscelis bilobatus, Nephotettix cincticeps, Lecanium corni, Saissetia oleae, Laodelphax striatellus, Nilaparvata lungens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia

brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortix virdana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Cetatophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäß verwendbaren Wirkstoffe der Formel (I) zeichnen sich durch eine hohe insektizide, akarizide und nematizide Wirksamkeit aus. Sie lassen sich insbesondere gegen pflanzenschädigende Insekten, wie beispielsweise gegen die Raupen der Kohlschabe (Plutella maculipennis) oder gengen die Larven der Meerrettichblattkäfer (Phaedon cochleariae), sowie gegen pflanzenschädigende Milben, wie beispielsweise gegen die gemeine Spinnmilbe (Tetranychus urticae) einsetzen. Sie eignen sich daneben hervorragend zur Bekämpfung von Bodeninsekten und Nematoden und lassen sich beispielsweise zur Bekämpfung von Phorbia antiqua-Maden oder von Nematoden der Gattung Meloidogyne incognita einsetzen. Auch eine nennenswerte wurzelsystemische Wirkung, beispielsweise gegen Phaedon cochleariae-Larven ist hervorzuheben. Die nematizide Wirkung der erfindungsgemäß verwendbaren Wirkstoffe läßt sich auch im in vitro Test beispielsweise gegen endoparasitisch lebende Nematoden der Gattung Caenorhabditis elegans bestätigen.

Außerdem besitzen die erfindungsgemäß verwendbaren Wirkstoffe der Formel (I) eine hohe Wirkung gegen Hygiene- und Vorratsschädlinge und lassen sich beispielsweise zur Bekämpfung der orientalischen Schabe (Blatta orientalis) oder zur Bekämpfung des gemeinen Kornkäfers (Sitophilus granarius) einsetzen. Darüber hinaus lassen sich die erfindungsgemäßen Wirkstoffe mit besonders gutem Erfolg zur Bekämpfung vom parasitisch lebenden Warmblüterschädlingen, (sowohl Ekto- als auch Endoparasiten), wie beispielsweise gegen die Larven der Goldfliege (Lucilia cuprina), gegen Rinderzecken (Boophilus microplus), gegen Räudemilben (Psoroptes ovis), gegen Stechfliegen (Stomoxys calcitrans) oder gegen die Weideviehfliege (Musca autumnalis) einsetzen.

Daneben besitzen die erfindungsgemäß verwendbaren Wirkstoffe der Formel (I) auch eine gute fungizide Wirksamkeit und lassen sich zur Bekämpfung von Pflanzenkrankheiten wie beispielsweise gegen den Erreger der Reisfleckenkrankheit (Pyricularia oryzae) oder gegen Schorf- und Botrytis- Erreger einsetzen.

In entsprechenden Aufwandmengen zeigen die erfindungsgemäß verwendbaren Wirkstoffe der Formel (I) darüber hinaus eine herbizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoff, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Tragerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung

von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalino, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butarol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäß verwendbaren Wirkstoffe können in ihren handelsüblichen Formulierungen sowie in den aus diesen Formulierungen bereiteten Anwendungsformen im Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäß verwendbaren Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variiert werden. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschlieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung von Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungungsgemäß verwendbaren Wirkstoffe geschieht auf diesen Gebieten in bekannter Weise, wie durch äußerliche Anwendung in Form beispielsweise des Tauchens (Dippen), Sprühens (Sprayen), Aufgießens (pour-on and spot-on) und des Einpuderns.

Die biologische Wirksamkeit der erfindungsgemäßen Verbindungen soll anhand der folgenden Beispiele erläutert werden.

Herstellungsbeispiele

Beispiel 1

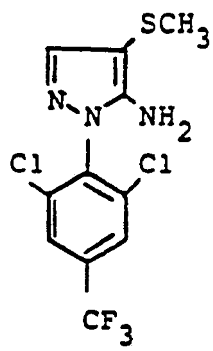

8 g (0,023 Mol) 5-Amino-1-(2,6-dichlor-4-trifluormethylphenyl)-pyrazol-4-ylthiocyanat in 100 ml absolutem Methanol werden bei Raumtemperatur in einer Stickstoffatmosphäre portionsweise mit 1,6 g (0,042 Mol) Natriumborhydrid versetzt. Nach beendeter Zugabe rührt man 45 Minuten bei Raumtemperatur und gibt dann eine Lösung von 2,4 g (0,042 Mol) Kaliumhydroxid in 40 ml absolutem Methanol und danach tropfenweise 6,0 g (0,042 Mol) Methyliodid zu. Nach beendeter Zugabe rührt man 30 Minuten bei Raumtemperatur, engt im Vakuum ein, nimmt den Rückstand in Wasser auf, extrahiert mehrmals mit Chloroform, trocknet über Natriumsulfat, entfernt das Lösungsmittel im Vakuum und Kristallisiert das zurückbleibende Öl aus Petrolether.

Man erhält 6,1 g (78% der Theorie) an 5-Amino-1-(2,6-dichlor-4-trifluormethylphenyl)-4-methylpyrazol vom Schmelzpunkt 106°C—108°C.

Herstellung der Ausgangsverbindung

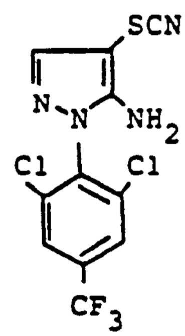

Zu 20,5 g (0,07 Mol) 5-Amino-1-(2,6-dichlor-4-trifluormethylphenyl)-pyrazol und 10,7 g (0,14 Mol) Ammoniumthiocyanat in 300 ml Eisessig tropft man bei 10°C unter Rühren 11,2 g (0,07 Mol) Brom in 70 ml Eisessig und rührt nach beendeter Zugabe weitere 10 Minuten bei +10°C. Zur Aufarbeitung gießt man den Ansatz auf Eis, stellt durch Zugabe von konzentrierter wässriger Ammoniaklösung auf pH 9 ein, saugt den ausgefallenen Feststoff ab und trocknet ihn.

Man erhält 22,9 g (93% der Theorie) an 5-Amino-1-(2,6-dichlor-4-trifluormethylphenyl)-pyrazol-4-yl-thiocynat vom Schmelpunkt 137°C.

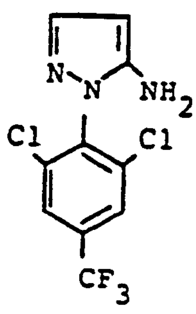

24,5 g (0,1 Mol) 2,6-Dichlor-4-trifluormethylphenyl-hydrazin werden mit 20 mg Ethylendiamin-tetraessigsäure-Dinatriumsalz (=Titriplex III) in 150 ml Methanol bei Rückflußtemperatur tropfenweise mit 25 ml (27,6 g/0,3 Mol) 2-Chloracrylnitril versetzt. Nach beendeter Zugabe erhitzt man weitere 8 Stunden auf Rückflußtemperatur, tropft dann 9 ml (0,16 Mol) 96%-ige Schwefelsäure zu und erhitzt weitere 6 Stunden auf Rückflußtemperatur. Die abgekühlte Reaktionsmischung wird mit 33,5 g (0,3 Mol) wasserfreiem

Natriumcarbonat versetzt. Nach 4 Stunden entfernt man das Lösungsmittel in Vakuum, nimmt den Rückstand in 500 ml Wasser auf und rührt 10 Stunden bei Raumtemperatur. Manfiltriert den ausgefallenen Niederschlag ab, wäscht mit Wasser nach und trocknet im Vakuum bei 50°C.

Man erhält 28,5 g (96% der Theorie) 5-Amino-1-(2,6-dichlor-4-trifluormethylphenyl)-pyrazol vom Schmelzpunkt 103°C—105°C.

Beispiel 2

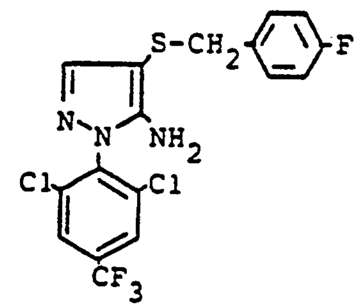

4,35 g (6,6 mMol) S,S'-Bis-[5-Amino-1-(2,6-dichlor-4-trifluormethylphenyl)-pyrazol-4-yl]-disufid, 0,5 g (13 mMol) Natriumhydroxid und 2,3 g (12 mMol) Natriumdithionit werden in einem Gemisch aus 80 ml Ethanol und 80 ml Wasser 2 Stunden unter Rückfluß erhitzt. Dann gibt man 2,45 g (13 mMol) 4-Fluorbenzylbromid zu und erhitzt eine weitere Stunde unter Rückfluß. Zur Aufarbeitung engt man im Vakuum auf die Hälfte des Volumens ein, extrahiert mehrfach mit Chloroform, trocknet die vereinigten organischen Phasen über Magnesiumsulfat und entfernt das Lösungsmittel im Vakuum. Der ölige Rückstand kristallisiert beim Verrühren mit Petrolether.

Man erhält 4,2 g (73% der Theorie) an 5-Amino-1-(2,6-dichlor-4-trifluormethylphenyl)-(4)-fluorbenzylthio)-pyrazol vom Schmelzpunkt 112°C—113°C.

Herstellung der Ausgangsverbindung

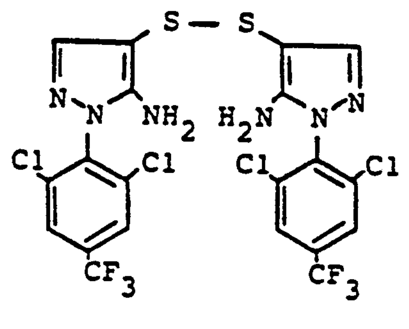

11,9 g (0,04 Mol) 5-Amino-1-(2,6-dichlor-4-trifluormethylphenyl-pyrazol-4-ylthiocyanat in einer Mischung aus 300 ml Ethanol und 300 ml Wassers werden nach Zugabe von 80 ml 10 normaler Chlorwasserstoffsäure 12 Stunden unter Rückfluß erhitzt. Zur Aufarbeitung engt man in Vakuum auf die Hälfte des Volumens ein und saugt den ausgefallenen Feststoff ab.

Nab erhält 10, 4 g (80% der Theorie) an S,S'-Bis-[5-Amino-1-(2,6-dichlor-4-trifluormethylphenyl)-pyrazol-4-yl]-disulfid vom Schmelzpunkt 148°C—150°C.

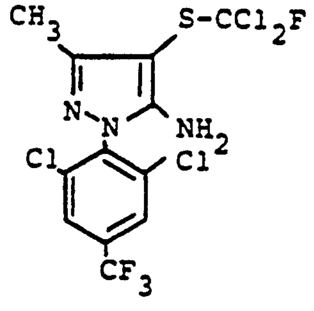

Beispiel 3

35,4 g (0,114 Mol) 5-Amino-1-(2,6-dichlor-4-trifluormethylphenyl)-3-methyl-pyrazol und 10 ml (0,125 Mol) wasserfreies Pyridin in 150 ml Dichlormethan werden bei 0°C bis 5°C unter Rühren tropfenweise mit 12,6 ml (0,12 Mol) Dichlorfluormethansulfenylchlorid versetzt. Nach beendeter Zugabe rührt man weitere 30 Minuten bei Raumtemperatur, gibt 100 ml Dichlormethan zu und wäscht nacheinander mit verdünnter Salzsäure, Wasser, Natriumbicarbonatlösung und wässriger Kochsalzlösung, trocknet über Magnesiumsulfat und entfernt das Lösungsmittel im Vakuum.

Man erhält 49,5 g (98% der Theorie) an 5-Amino-1-(2,6-dichlor-4-trifluormethylphenyl)-4-dichlorfluormethylsulfenyl-3-methyl-pyrazol vom Schmelzpunkt 131°C.

Herstellung der Ausgangsverbindung

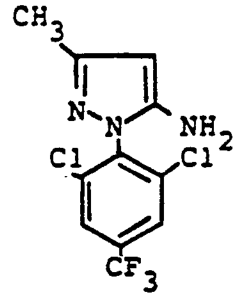

61,25 g (0,25 Mol) 2,6-Dichlor-4-trifluormethylphenylhydrazin und 21 g (0,25 Mol) Diacetonitril in 500 ml Ethanol werden 20 Stunden unter Rückfluß erhitzt. Zu der erkalteten Reaktionsmischung gibt man 4 ml konzentrierte Schwefelsäure und erhitzt für weitere 4 Stunden auf 60°C. Zur Aufarbeitung wird die Mischung im Vakuum eingedampft, der Rückstand in Chloroform aufgenommen und mit 25 prozentiger wässriger Ammoniaklösung alkalisch gestellt. Die organische Phase wird angetrennt und die wässrige Phase mit Chloroform extrahiert. Die vereinigten organischen Phasen werden über Magnesiumsulfat getrocknet und im Vakuum vom Lösungsmittel befreit.

Man erhält 62 g (80% der Theorie) an 5-Amino-1-(2,6-dichlor-4-trifluormethylphenyl)-3-methyl-pyrazol als glasartige Substanz.

$^1H$—NMR ($CDCl_3$/TMS) δ = 2,23; 3,50; 5,49; 7,68 ppm

Beispiel 4

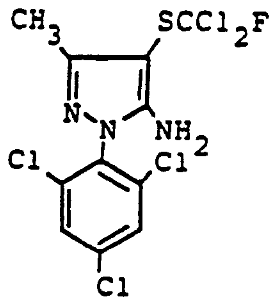

27,7 g (0,1 Mol) 5-Amino-3-methyl-1-(2,4,6-trichlorphenyl)-pyrazol und 10 ml (0,125 Mol) wasserfreies Pyridin in 150 ml Dichlormethan werden bei 0°C bis 5°C tropfenweise unter Rühren mit 12,6 ml (1,12 Mol) Dichlorfluormethansulfenylchlorid versetzt und 30 Minuten gerührt. Zur Aufarbeitung gibt man 100 ml Dichlormethan zu, wäscht nacheinander mit verdünnter Salzsäure, Wasser, Natriumcarbonatlösung und Kochsalzlösung, trocknet über Magnesiumsulfat und entfernt das Lösungsmittel im Vakuum.

Man erhält 38,2 g (93% der Theorie) an 5-Amino-4-dichlorfluormethylsulfenyl-3-methyl-1-(2,4,6-trichlorphenyl)-pyrazol vom Schmelzpunkt 125°C—127°C.

Herstellung der Ausgangsverbindung

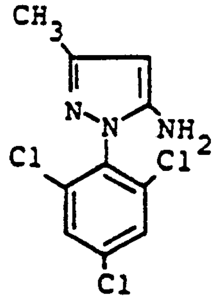

34,8 g (0,1 Mol) 5-Amino-3-methyl-1-(2,4,6-trichlorphenyl)-pyrazol-4-ylcarbonsäureethylester werden in einer Mischung aus 250 ml 48 prozentiger Bromwasserstoffsäure und 25 ml Isopropanol mehrere Stunden auf 120°C erhitzt. Nach beendeter Reaktion setzt man 200 ml Wasser zu, stellt mit 10 prozentiger Natriumhydroxidlösung auf pH 7 bis 8 ein und saugt den ausgefallenen Feststoff ab.

Man erhält 18 g (65% der Theorie) an 5-Amino-3-methyl-1-(2,4,6-trichlorphenyl)-pyrazol vom Schmelzpunkt 111°C—113°C.

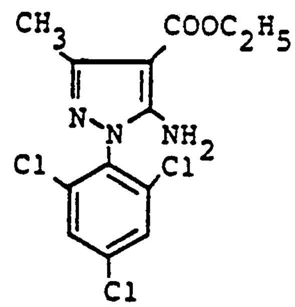

Eine Lösung aus 21,2 g (0,1 Mol) 2,4,6-Trichlorphenylhydrazin und 18,3 g (0,1 Mol) 2-Cyan-3-ethoxy-2-butensäureethylester in 250 ml Ethanol wird 14 Stunden unter Rückfluß erhitzt, dann im Vakuum eingeengt, der Rückstand in Dichlormethan aufgenommen, mit Wasser gewaschen, über Magnesiumsulfat getrocknet und im Vakuum vom Lösungsmittel befreit.

Man erhält 22 g (63% der Theorie) an 5-Amino-3-methyl-1-(2,4,6-trichlorphenyl)-pyrazol-4-ylcarbonsäureethylester vom Schmelzpunkt 150°C.

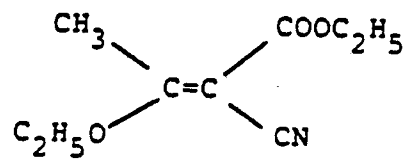

113 g (1 Mol) Cyanessigsäureethylester, 162 g (1 Mol) Triethylorthoacetat und 204 g (2 Mol) Acetanhydrid werden unter Rückfluß erhitzt, wobei Essigsäureethylester langsam abdestilliert und die Reaktionstemperatur auf 140°C ansteigt. Der Rückstand wird im Wasserstrahlvakuum destilliert.

Man erhält 57 g (32% der Theorie) an 2-Cyan-3-ethoxy-2-butensäureethylester von Siedepunkt 150°C bei 24 mbar und vom Schmelzpunkt 70°C (Umkristallisation aus Isopropanol).

Beispiel 5

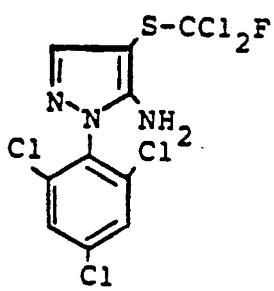

30 g (0,114 Mol) 5-Amino-1-(2,4,6-trichlorphenyl)-pyrazol und 10 ml (0,125 Mol) wasserfreies Pyridin in 150 ml Methylenchlorid werden bei 0°C bis 5°C mit 12,6 ml (0,12 Mol) Dichlorfluormethansulfenylchlorid versetzt und 30 Minuten gerührt. Zur Aufarbietung gibt man 100 ml Methylenchlorid zu und wäscht nacheinander mit verdünnter Salzsäure, Wasser, Natriumbicarbonatlösung und Kochsalzlösung, trocknet über Magnesiumsulfat und entfernt das Lösungsmittel im Vakuum.

Man erhält 44,5 g (98.7% der Theorie) an 5-Amino-4-dichlorfluormethansulfenyl-1-(2,4,6-trichlorphenyl)-pyrazol vom Schmelzpunkt 101°C—106°C.

Beispiel 6

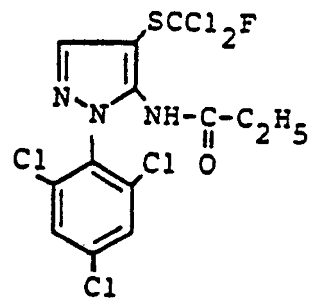

13,0 g (0,029 Mol) 5-Amino-4-dichlorfluormethylsulfenyl-1-(2,4,6-trichlorphenyl)-pyrazol werden mit 50 ml Propionsäureanhydrid und 1 ml konzentrierter Schwefelsäure 10 Stunden bei Raumtemperatur gerührt. Das Reaktionsgemisch wird dann in 100 ml eisgekühltes Methanol eingetropft, eine Stunde nachgerührt und anschließend zur Trockne eingedampft. Der Rückstand wird in Dichlormethan aufgenommen, nacheinander mit Wasser, Natriumbicarbonatlösung und Kochsalzlösung gewaschen, über Magnesiumsulfat getrocknet und im Vakuum vom Lösungsmittel befreit.

Man erhält 8,9 g (68% der Theorie) an 4-Dichlorfluormethansulfenyl-5-propionamido-1-(2,4,6-trichlorphenyl)-pyrazol vom Schmelzpunkt 129°C.

Beispiel 7

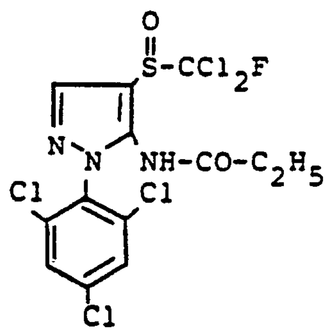

3 g (6,62 mMol) 4-Dichlorfluormethansulfenyl-5-propionamido-1-(2,4,6-trichlorphenyl)-pyrazol und 1,5 g (8,7 mMol) m-Chlorperbenzoesäure in 10 ml Dichlormethan werden 16 Stunden bei Raumtemperatur gerührt, filtriert, das Filtrat nacheinander mit Natriumbicarbonat-, Natriumthiosulfat-, nochmals mit Natriumbicarbonat- und dann mit Natriumchloridlösung gewaschen, über Magnesiumsulfat getrocknet und im Vakuum vom Lösungsmittel befreit.

Man erhält 2,8 g (90,2% der Theorie) an 4-Dichlorfluormethylsulfinyl-5-propionamido-1-(2,4,6-trichlorphenyl)-pyrazol vom Schmelzpunkt 146°C.

In entsprechender Weise und gemäß den allgemeinen Herstellungsangaben erhält man die folgenden 5-Aminopyrazolderivate der allgemeinen Formel (I):

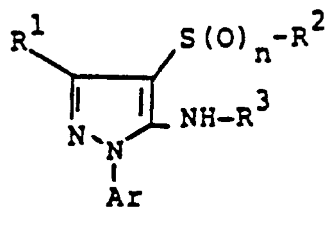

(I)

| Bsp. Nr. | $R^1$ | $R^2$ | $R^3$ | Ar | n | physikalische Eigenschaften |
|---|---|---|---|---|---|---|
| 8 | H | $CCl_2F$ | $CH_3-CH_2-CO$ | 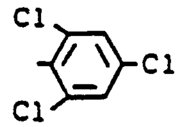 | 2 | Fp 139°C |
| 9 | H | $CF_3$ | $CH_3-CO$ | 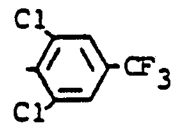 | 0 | Fp 131-138°C |
| 10 | H | $CCl_2F$ | H | 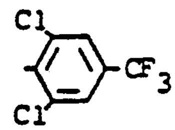 | 0 | Fp 99-105°C |
| 11 | H | $CCl_2F$ | H | 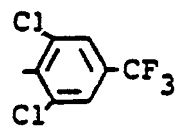 | 1 | Fp 55-62°C |
| 12 | H | $CCl_2F$ | H | 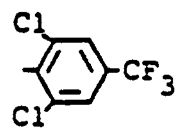 | 2 | Fp 135-138°C |
| 13 | H | $CCl_2F$ | H | 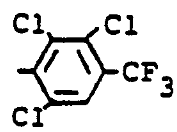 | 0 | Fp 60-65°C |
| 14 | H | $CCl_2F$ | H | 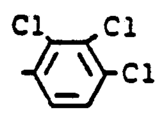 | 0 | Fp 55-62°C |
| 15 | H | $CCl_2F$ | H | 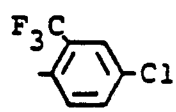 | 0 | NMR (3-H,Pyr.) 7.57 ppm (s,1H) ($CDCl_3$/TMS) |
| 16 | H | $CCl_2F$ | H | 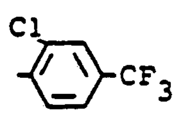 | 0 | Fp 81-87°C |
| 17 | H | $CCl_2F$ | H | 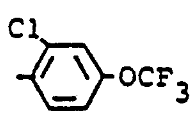 | 0 | Fp 77°C |
| 18 | H | $CCl_2F$ | H | 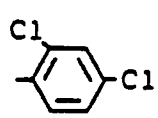 | 0 | NMR Öl,(3-H,Pyr.) 7.61 ppm (s,1H) ($CDCl_3$/TMS) |

| Bsp. Nr. | $R^1$ | $R^2$ | $R^3$ | Ar | n | physikalische Eigenschaften |
|---|---|---|---|---|---|---|
| 19 | H | $CCl_2F$ | H | 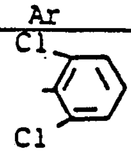 | 0 | Fp 112-121°C |
| 20 | H | $CCl_2F$ | H | 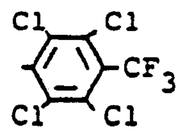 | 0 | Fp 55-61°C |
| 21 | H | $CCl_2F$ | H | 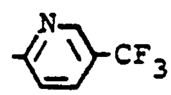 | 0 | NMR Öl (3-H, Pyr.) 7.58 ppm (s,1H) ($CDCl_3$/TMS) |
| 22 | H | $CCl_2F$ | $CH_3$ | 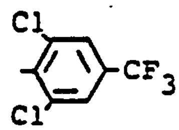 | 0 | Fp 107-110°C |
| 23 | H | $CCl_2F$ | H | 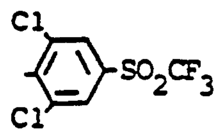 | 0 | Fp 148-153°C |
| 24 | H | $CCl_3$ | H | 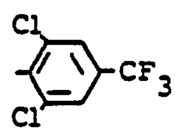 | 0 | Fp 75-88°C |
| 25 | H | $CF_3$ | H | 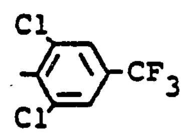 | 0 | Fp 86-94°C |
| 26 | H | 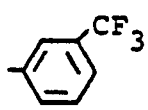 | H | 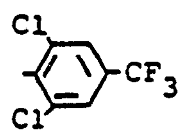 | 0 | NMR Öl (3-H,Pyr.) 7.64 ppm (s, 1H) ($CDCl_3$TMS) |
| 27 | H | $CCl_2F$ | H | 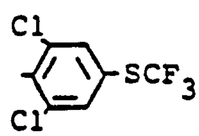 | 0 | Fp 104-107°C |
| 28 | H | $CCl_2F$ | H | 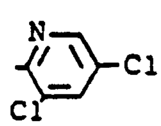 | 0 | NMR Öl(3-H, Pyr.) 7.64 (s,1H) ($CDCl_3$/TMS) |
| 29 | H | $CCl_2F$ | H | 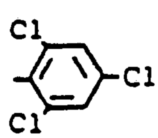 | 1 | Fp 69-75°C |

| Bsp. Nr. | $R^1$ | $R^2$ | $R^3$ | Ar | n | physikalische Eigenschaften |
|---|---|---|---|---|---|---|
| 30 | H | $CCl_2F$ | H | 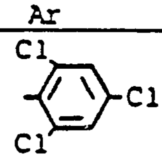 | 2 | Fp 63-69°C |
| 31 | H | $CCl_2F$ | H | 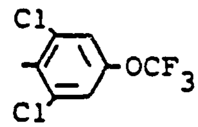 | 0 | Fp 101-107°C |
| 32 | H | $CCl_2F$ | H | 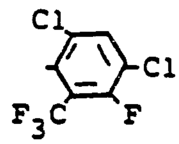 | 0 | NMR: Öl (3-H, Pyr.) 7,67 ppm (s,1H) ($CDCl_3$/TMS) |
| 33 | H | $CCl_2F$ | H | 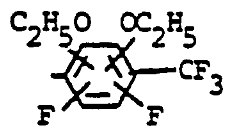 | 0 | NMR: Öl (3-H, Pyr.) 7,70 ppm (s,1H) ($CDCl_3$/TMS) |
| 34 | H | $CCl_2F$ | H | 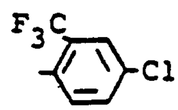 | 2 | Fp 61-65°C |
| 35 | H | $CCl_2F$ | H | 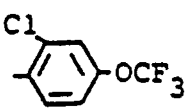 | 2 | Fp 117-124°C |
| 36 | H | $CCl_2F$ | H | 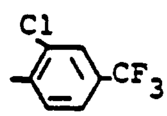 | 2 | Fp 132-137°C |
| 37 | H | $CF_3$ | H | 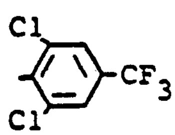 | 2 | Fp 57-63°C |
| 38 | H | $CF_3$ | H | 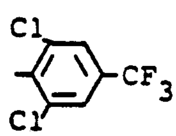 | 1 | Fp 86-92°C |
| 39 | H | $CCl_2F$ | H | 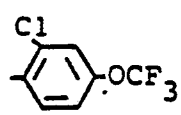 | 1 | Fp 45-55°C |
| 40 | H | $-CF_2-CCl_2F$ | H | 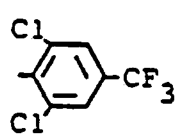 | 0 | Fp 117-119°C |

| Bsp. Nr. | $R^1$ | $R^2$ | $R^3$ | Ar | n | physikalische Eigenschaften |
|---|---|---|---|---|---|---|
| 41 | H | $CCl_2F$ | H | 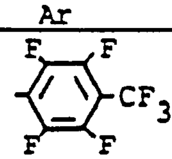 | 0 | NMR: Öl (3-H, Pyr.) 7,73 (s,1H) ($CDCl_3$/TMS) |
| 42 | $CH_3$ | $CCl_2F$ | H | 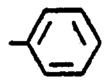 | 0 | Fp 69-70°C |
| 43 | $CH_3$ | $CCl_2F$ | $C_2H_5CO$ | 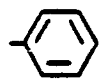 | 0 | Fp 194°C |
| 44 | $CH_3$ | $CCl_2F$ | H | 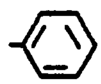 | 2 | Fp 94-95°C |
| 45 | $CH_3$ | $CCl_2F$ | H | 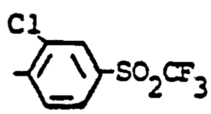 | 0 | Fp 108-110°C |
| 46 | $CH_3$ | $CCl_2F$ | $C_2H_5CO$ | 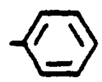 | 1 | Fp 174°C |
| 47 | $CH_3$ | $CCl_2F$ | $C_2H_5CO$ | 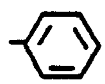 | 2 | Fp 208°C |
| 48 | $CH_3$ | $CCl_2F$ | H | 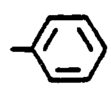 | 1 | NMR: Öl 3-$CH_3$ (Pyr.),S, 2,27 ppm ($CDCl_3$/TMS) |
| 49 | $CH_3$ | $CCl_2F$ | $C_2H_5CO$ | 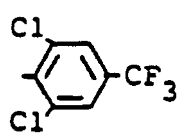 | 0 | Fp 128-130°C |
| 50 | $CH_3$ | $CCl_2F$ | $C_2H_5CO$ | 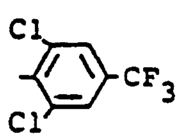 | 2 | Fp 179°C |
| 51 | $CH_3$ | $CCl_2F$ | H | 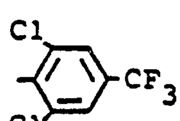 | 1 | Fp 63-64°C |
| 51a | H | $CClF_2$ | H | 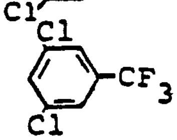 | O | |

| Bsp. Nr. | $R^1$ | $R^2$ | $R^3$ | Ar | n | physikalische Eigenschaften |
|---|---|---|---|---|---|---|
| 52 | $(CH_3)_3C$ | $CCl_2F$ | H | 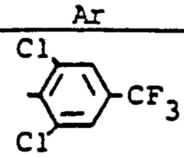 | 0 | Fp 140-144°C |
| 53 | $CH_3$ | $CCl_3$ | H | 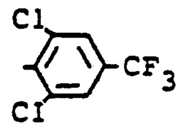 | 0 | Fp 150-152°C |
| 54 | $CH_3$ | $CF_3$ | H | 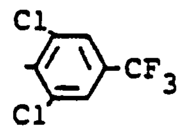 | 0 | Fp 147°C |
| 55 | $CH_3$ | 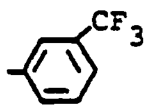 | H | 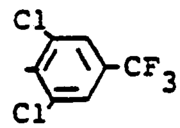 | 0 | Fp 58-82°C |
| 56 | $CH_3$ | $CCl_2F$ | H | 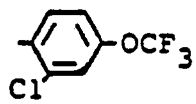 | 0 | Fp 58-61°C |
| 57 | $CH_3$ | $CCl_2F$ | H | 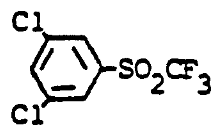 | 0 | Fp 78-80°C |
| 58 | H | $CH_3$ | H | 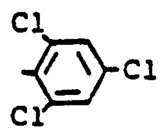 | 0 | Fp 114°C |
| 59 | H | $CH_3$ | $C_2H_5O$ | 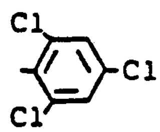 | 0 | Fp 92-95°C (Zers.) |
| 60 | H | $CH_3$ | $C_2H_5O$ | 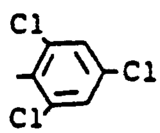 | 2 | Fp 146-150°C |
| 61 | H | $CH_3$ | H | 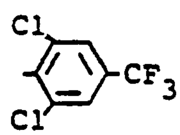 | 2 | Fp 60-65°C |
| 62 | H | $CH_3$ | H | 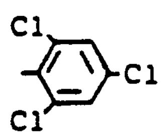 | 2 | FP 170-171°C |

| Bsp. Nr. | $R^1$ | $R^2$ | $R^3$ | Ar | n | physikalische Eigenschaften |
|---|---|---|---|---|---|---|
| 63 | H | $C_2H_5$ | H | 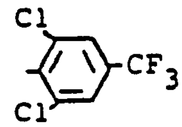 | 0 | Fp 81-82°C |
| 64 | H | $(CH_3)_2CH-$ | H | 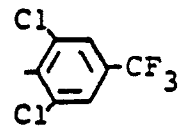 | 0 | Fp 77-79°C |
| 65 | H | 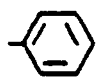 | H | 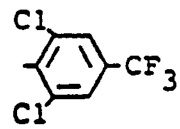 | 0 | Fp 124-127°C |
| 66 | $CH_3$ | $CH_3$ | H | 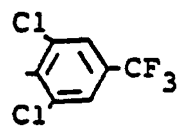 | 0 | Fp 54-57°C |
| 67 | $CH_3$ | $C_2H_5$ | H | 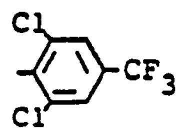 | 0 | Fp 109-110°C |
| 68 | H | $CCl_2F$ | H | 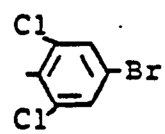 | 0 | Fp 92-94°C |
| 69 | H | $CCl_2F$ | H | 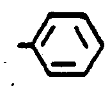 | 0 | Fp 85-89°C |
| 70 | H | $CCl_2F$ | H | 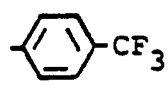 | 0 | Fp 70°C |
| 71 | H | $CCl_2F$ | H | 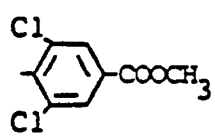 | 0 | Fp 140-144°C |
| 72 | H | $CCl_2F$ | H | 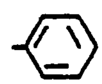 | 2 | Fp 107-110°C |
| 73 | H | $CCl_2F$ | H | 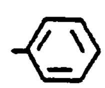 | 1 | Fp 75-77°C |

| Bsp. Nr. | $R^1$ | $R^2$ | $R^3$ | Ar | n | physikalische Eigenschaften |
|---|---|---|---|---|---|---|
| 74 | H | $CCl_2F$ | $C_2H_5O$ | 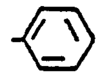 | 0 | Fp 88-80°C |
| 75 | H | $CCl_2F$ | H | 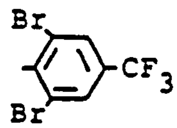 | 0 | Fp 80-83°C |
| 76 | H | $CCl_2F$ | H | 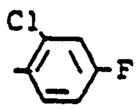 | 0 | Fp 68-72°C |
| 77 | H | $CCl_2F$ | H | 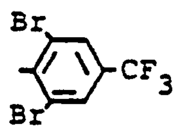 | 2 | Fp 104-106°C |
| 78 | H | $CCl_2F$ | H | 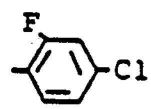 | 0 | NMR: 7,60 ppm (s,1H) 4,36 ppm (s,2H) ($CDCl_3$/TMS) |
| 79 | H | $CCl_2F$ | H | 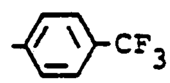 | 2 | Fp 128-132°C |
| 80 | H | $CCl_2F$ | H | 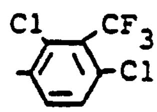 | 0 | NMR: 7,61 ppm (s,1H) 4,26 ppm (s,2H) ($CDCl_3$/TMS) |
| 81 | H | $CCl_2F$ | H | 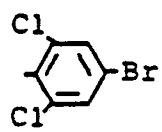 | 2 | Fp 100-101°C |
| 82 | H | $CCl_2F$ | H | 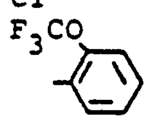 | 0 | NMR: 7,57 ppm (s,1H) 4,31 ppm (s,2H) ($CDCl_3$/TMS) |
| 83 | H | $CF_3$ | $CH_3$ | 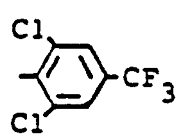 | 0 | Fp 50-60°C |
| 84 | H | $CF_3$ | H | 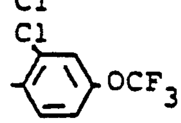 | 0 | Fp 112-113°C |
| 85 | H | $CF_3$ | H | 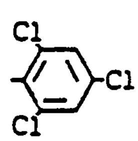 | 0 | Fp 90-91°C |

| Bsp. Nr. | $R^1$ | $R^2$ | $R^3$ | Ar | n | physikalische Eigenschaften |
|---|---|---|---|---|---|---|
| 86 | H | $CH_3$ | H | 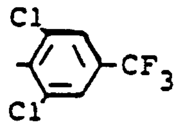 | 1 | Fp 179-182°C |
| 87 | $CH_3$ | $CH_3$ | H | 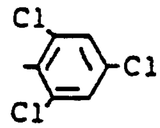 | 0 | Fp 128-131°C |
| 88 | $CH_3$ | $C_2H_5$ | H | 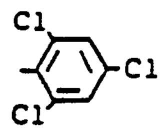 | 0 | Fp 121-123 °C |
| 89 | $CH_3$ | $(CH_3)_2CH-$ | H | 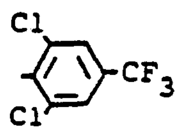 | 0 | Fp 100-101°C |
| 90 | H | $CCl_2F$ | H | 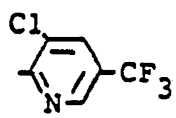 | 0 | NMR: 7,7 ppm (s,1H) 8,2 ppm (1H) 8,6 ppm (1H) ($CDCl_3$/TMS) |
| 91 | $CH_3$ | $CF_3$ | H | 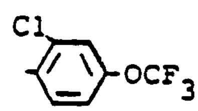 | 0 | Fp 67-70°C |
| 92 | $CH_3$ | $CF_3$ | H | 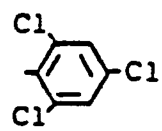 | 0 | Fp 122-125°C |
| 93 | H | $CCl_2F$ | H | 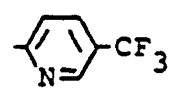 | 2 | Fp 102-110°C |
| 94 | H | $CCl_2F$ | H | 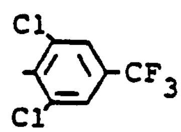 | 0 | Fp 68-74°C |
| 95 | H | $CCl_2F$ | H | 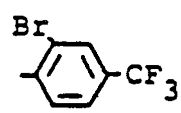 | 0 | Fp 60-70°C |
| 96 | $CH_3$ | $CF_3$ | $COC_2H_5$ | 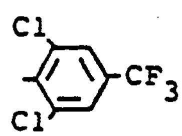 | 0 | Fp 138°C |
| 97 | H | $CF_3$ | H | 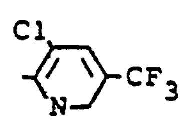 | 0 | Fp 67-68°C |

| Bsp. Nr. | $R^1$ | $R^2$ | $R^3$ | Ar | n | physikalische Eigenschaften |
|---|---|---|---|---|---|---|
| 98 | $CH_3$ | $CCl_2F$ | H | 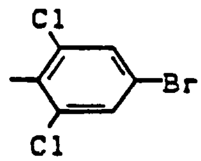 | 0 | Fp. 133-134° C |
| 99 | $CH_3$ | $CClF_2$ | H | 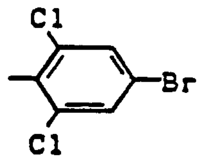 | 0 | Fp. 132-134° C |
| 100 | $CH_3$ | $CF_3$ | H | 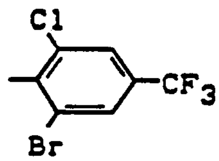 | 2 | Fp. 150-152° C |
| 101 | $CH_3$ | $CCl_2F$ | H | 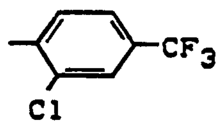 | 1 | Fp. 135-138° C |
| 102 | $CH_3$ | $CCl_2F$ | H | 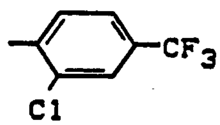 | 2 | Fp. 58-61° C |
| 103 | $CH_3$ | $CF_3$ | H | 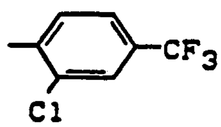 | 1 | Fp. 147° C |
| 104 | $CH_3$ | $CF_3$ | H | 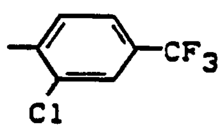 | 2 | Fp. 103-105° C |
| 105 | $CH_3$ | $CClF_2$ | H | 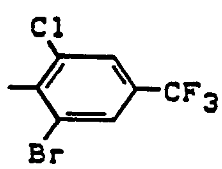 | 0 | Fp. 114-116° C |

| Bsp. Nr. | $R^1$ | $R^2$ | $R^3$ | Ar | n | physikalische Eigenschaften |
|---|---|---|---|---|---|---|
| 106 | $CH_3$ | $CF_3$ | H | 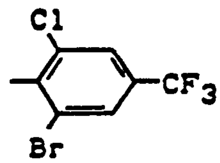 | 1 | Fp. 157-159° C |
| 107 | $CH_3$ | $CClF_2$ | H | 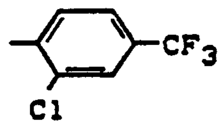 | 0 | NMR:<br>Öl 3-$CH_3$(Pyr.)<br>(s,2,30 ppm)<br>($CDCl_3$/TMS) |
| 108 | $CH_3$ | $CClF_2$ | H | 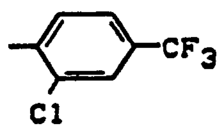 | 1 | Fp. 118-122° C |
| 109 | $CH_3$ | $CClF_2$ | H | 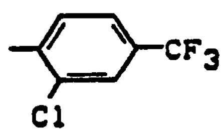 | 2 | Fp. 59-61° C |
| 110 | $CH_3$ | $CClF_2$ | H | 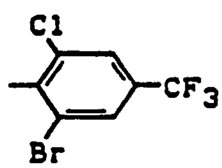 | 1 | Fp. 76-80° C |
| 111 | $CH_3$ | $CClF_2$ | H | 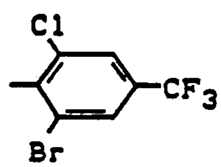 | 2 | Fp. 143-144° C |
| 112 | $CH_3$ | $CCl_2F$ | H | 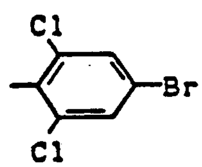 | 2 | Fp. 184-185° C |
| 113 | $CH_3$ | $CF_3$ | H | 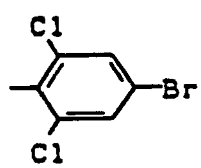 | 2 | Fp. 135-138° C |

| Bsp. Nr. | $R^1$ | $R^2$ | $R^3$ | Ar | n | physikalische Eigenschaften |
|---|---|---|---|---|---|---|
| 114 | $CH_3$ | $CClF_2$ | H | 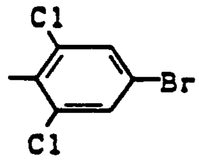 | 2 | Fp. 154-155° C |
| 115 | $CH_3$ | $CCl_2F$ | H | 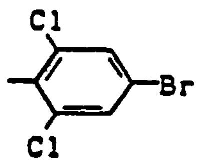 | 1 | Fp. 168-170° C |
| 116 | $CH_3$ | $CF_3$ | H | 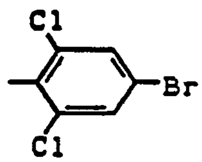 | 1 | Fp. 170° C |
| 117 | $CH_3$ | $CClF_2$ | H | 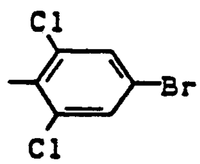 | 1 | Fp. 82-85° C |
| 118 | $CH_3$ | $CCl_2F$ | $CO-C_2H_5$ | 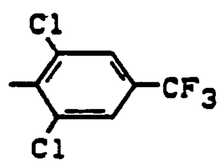 | 1 | Fp. 174° C |
| 119 | $CH_3$ | $CCl_2F$ | H | 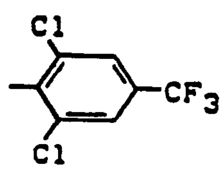 | 2 | Fp. 145° C |
| 120 | $CH_3$ | $CF_3$ | H | 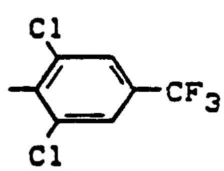 | 0 | Fp. 144-149°C |
| 121 | $CH_3$ | $CCl_2F$ | H | 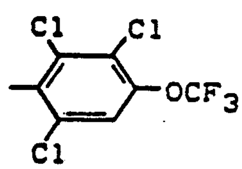 | 0 | Fp. 102-110°C |

| Bsp. Nr. | $R^1$ | $R^2$ | $R^3$ | Ar | n | physikalische Eigenschaften |
|---|---|---|---|---|---|---|
| 122 | $CH_3$ | $CCl_2F$ | H | 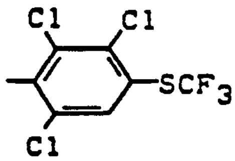 | 0 | Fp. 80-96°C |
| 123 | $CH_3$ | $CF_3$ | H | 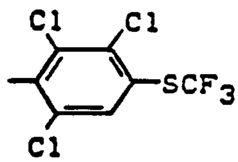 | 0 | Fp 118-121°C |
| 124 | $CH_3$ | $CCl_2F$ | H | 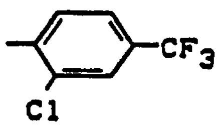 | 0 | Fp. 75-77°C |
| 125 | $CH_3$ | $CF_3$ | H | 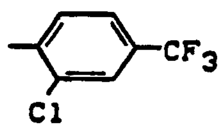 | 0 | Fp. 83°C |
| 126 | $CH_3$ | $CCl_2F$ | H | 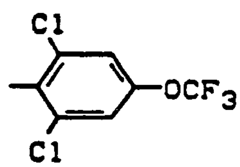 | 0 | Fp. 95-97° C |
| 127 | $CH_3$ | $CF_3$ | H | 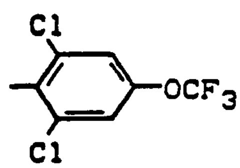 | 0 | Fp. 100-101° C |
| 128 | $C_2H_5$ | $CCl_2F$ | H | 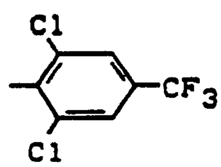 | 0 | Fp. 105-108° C |

| Bsp. Nr. | $R^1$ | $R^2$ | $R^3$ | Ar | n | physikalische Eigenschaften |
|---|---|---|---|---|---|---|
| 129 | $C_2H_5$ | $CF_3$ | H | 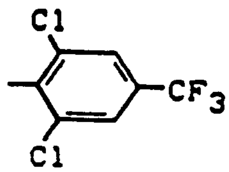 | 0 | Fp. 113-116°C |
| 130 | $C_2H_5$ | $CCl_2F$ | H | 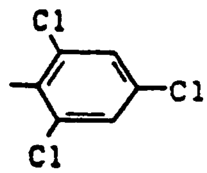 | 0 | Fp. 89-93° C |
| 131 | $C_2H_5$ | $CF_3$ | H | 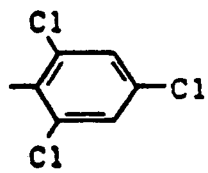 | 0 | Fp. 76-78° C |
| 132 | $CH_3$ | $CF_3$ | H | 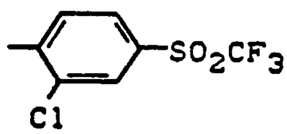 | 0 | NMR: Öl, 3-$CH_3$ (Pyr.) s, 2,30 ppm ($CDCl_3$/TMS) |
| 133 | $CH_3$ | $CF_3$ | $COC_2H_5$ | 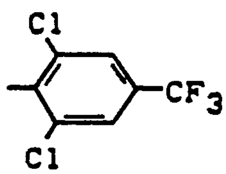 | 1 | Fp. 135-137°C |
| 134 | $CH_3$ | $CF_3$ | $COC_2H_5$ | 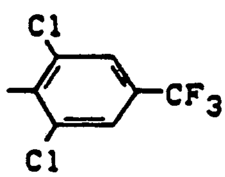 | 2 | Fp. 132-133°C |
| 135 | $CH_3$ | $CF_3$ | H | 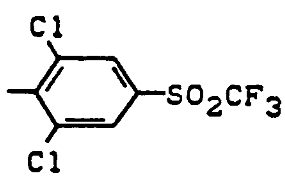 | 0 | Fp. 160-169°C |

| Bsp. Nr. | $R^1$ | $R^2$ | $R^3$ | Ar | n | physikalische Eigenschaften |
|---|---|---|---|---|---|---|
| 136 | $CH_3$ | $CF_3$ | H | 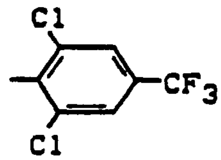 | 2 | Fp. 147-148°C |
| 137 | $CH_3$ | $CF_2Cl$ | H | 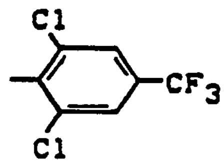 | 0 | Fp. 63-65° C |
| 138 | $CH_3$ | $CF_3$ | $COCH_3$ | 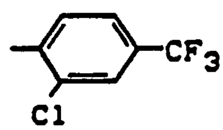 | 0 | Fp. 157-159° C |
| 139 | $CH_3$ | $CF_3$ | H | 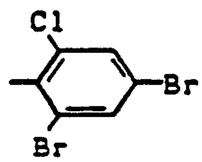 | 0 | Fp. 143°C |
| 140 | $C_2H_5$ | $CCl_2F$ | $COCH_3$ | 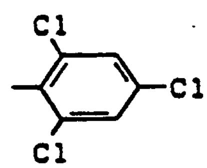 | 0 | Fp. 158-160° C |
| 141 | $C_2H_5$ | $CF_3$ | $COCH_3$ | 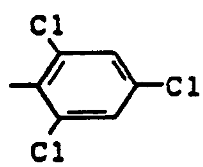 | 0 | Fp. 178-180° C |
| 142 | $CH_3$ | $CCl_2F$ | H | 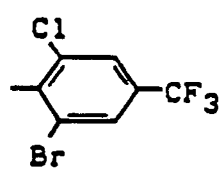 | 0 | Fp. 105-107° C |
| 143 | $CH_3$ | $CF_3$ | H | 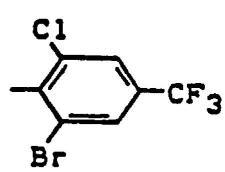 | 0 | Fp. 136-137° C |

| Bsp. Nr. | $R^1$ | $R^2$ | $R^3$ | Ar | n | physikalische Eigenschaften |
|---|---|---|---|---|---|---|
| 144 | $CH_3$ | $CF_3$ | H | 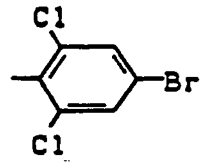 | 0 | Fp. 137-138° C |
| 145 | $C_2H_5$ | $CF_3$ | $COCH_3$ | 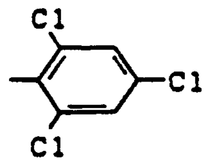 | 1 | Fp. 62-64° C |
| 146 | $C_2H_5$ | $CF_3$ | $COCH_3$ | 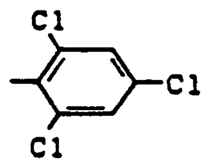 | 2 | Fp. 140-141° C |
| 147 | $C_2H_5$ | $CCl_2F$ | $COC_2H_5$ | 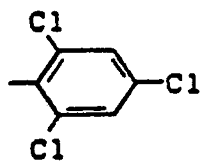 | 0 | Fp. 120-121° C |
| 148 | $C_2H_5$ | $CCl_2F$ | $COC_2H_5$ | 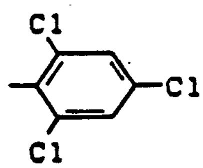 | 1 | NMR: Öl, 3-$CH_3$(Pye.) (t, 1,07 ppm) 3-$CH_2$(Pyr.) (q,2.26) (CDC$_3$/TMS) |
| 149 | $C_2H_5$ | $CCl_2F$ | $COC_2H_5$ | 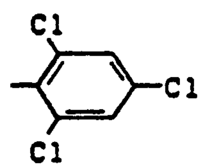 | 2 | Fp. 91-93° C |
| 150 | $C_2H_5$ | $CF_3$ | H | 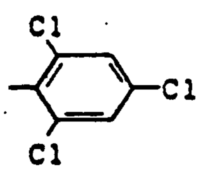 | 1 | Fp. 146-147° C |
| 151 | $C_2H_5$ | $CF_3$ | H | 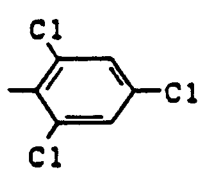 | 2 | Fp. 91-92° C |

| Bsp. Nr. | $R^1$ | $R^2$ | $R^3$ | Ar | n | physikalische Eigenschaften |
|---|---|---|---|---|---|---|
| 152 | $C_2H_5$ | $CCl_2F$ | H | 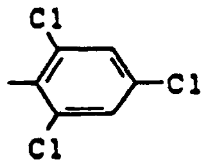 | 1 | Fp. 138-140° C |
| 153 | $C_2H_5$ | $CCl_2F$ | H | 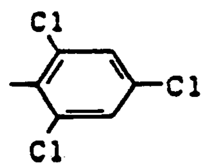 | 2 | Fp. 60-61° C |
| 154 | $CH_3$ | $CClF_2$ | H | 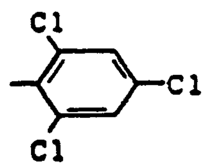 | 0 | Fp. 130-131° C |
| 155 | $CH_3$ | $CClF_2$ | $COC_2H_5$ | 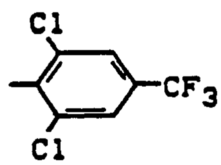 | 0 | Fp. 135°C |
| 156 | $CH_3$ | $CClF_2$ | $COC_2H_5$ | 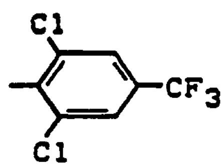 | 1 | Fp. 144-145° C |
| 157 | $CH_3$ | $CClF_2$ | $COC_2H_5$ | 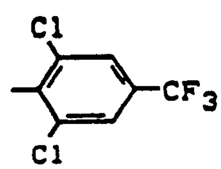 | 2 | Fp. 144° C |
| 158 | $CH_3$ | $CF_3$ | $COC_2H_5$ | 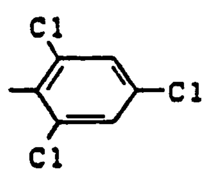 | 0 | Fp. 164° C |
| 159 | $CH_3$ | $CCl_2F$ | $COC_2H_5$ | 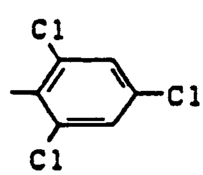 | 0 | Fp. 144-146° C |

| Bsp. Nr. | $R^1$ | $R^2$ | $R^3$ | Ar | n | physikalische Eigenschaften |
|---|---|---|---|---|---|---|
| 160 | $CH_3$ | $CClF_2$ | $COC_2H_5$ | 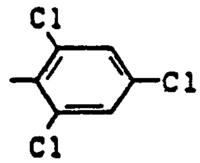 | 0 | Fp. 131°C |
| 161 | $CH_3$ | $CClF_2$ | H | 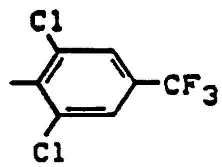 | 1 | Fp. 63-67°C |
| 162 | $CH_3$ | $CClF_2$ | H | 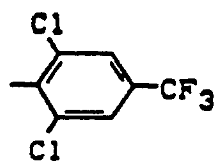 | 2 | Fp. 120-128°C |
| 163 | $CH_3$ | $CF_3$ | $COC_2H_5$ | 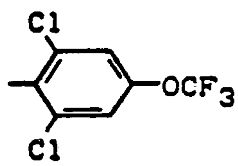 | 0 | Fp. 128-130°C |
| 164 | $CH_3$ | $CCl_2F$ | $COC_2H_5$ | 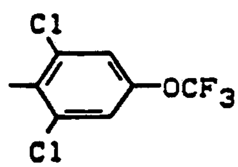 | 0 | Fp. 102°C |
| 165 | $CH_3$ | $CClF_2$ | H | 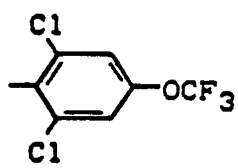 | 0 | Fp. 111-112°C |
| 166 | $CH_3$ | $CF_3$ | $COC_2H_5$ | 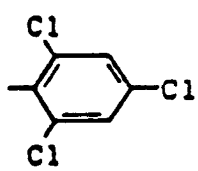 | 0 | Fp. 118-124°C |
| 167 | $CH_3$ | $CF_3$ | $COC_2H_5$ | 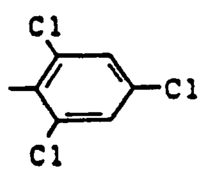 | 2 | Fp. 100-102°C |

| Bsp. Nr. | $R^1$ | $R^2$ | $R^3$ | Ar | n | physikalische Eigenschaften |
|---|---|---|---|---|---|---|
| 168 | $CH_3$ | $CCl_2F$ | $COC_2H_5$ | 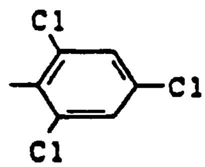 | 1 | Fp. 102-103° C |
| 169 | $CH_3$ | $CCl_2F$ | $COC_2H_5$ | 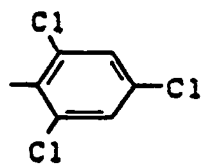 | 2 | Fp. 168-169° C |
| 170 | $CH_3$ | $CClF_2$ | $COC_2H_5$ | 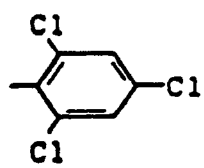 | 1 | Fp. 161-163° C |
| 171 | $CH_3$ | $CClF_2$ | $COC_2H_5$ | 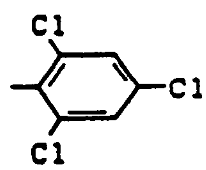 | 2 | Fp. 111-113° C |
| 172 | $CH_3$ | $CClF_2$ | $COC_2H_5$ | 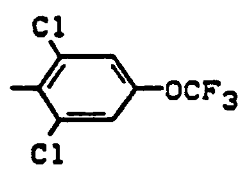 | 0 | Fp. 108-109° C |
| 173 | $CH_3$ | $CF_3$ | H | 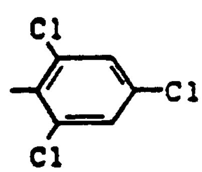 | 1 | Fp. 178-180° C |
| 174 | $CH_3$ | $CF_3$ | H | 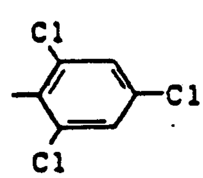 | 2 | Fp. 145-147° C |
| 175 | $CH_3$ | $CCl_2F$ | H | 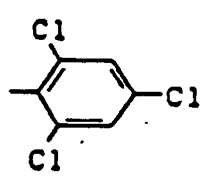 | 1 | Fp. 180-181° C |

| Bsp. Nr. | $R^1$ | $R^2$ | $R^3$ | Ar | n | physikalische Eigenschaften |
|---|---|---|---|---|---|---|
| 176 | $CH_3$ | $CCl_2F$ | H | 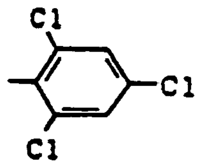 | 2 | Fp. 162-178° C |
| 177 | $CH_3$ | $CClF_2$ | H | 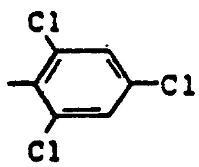 | 1 | Fp. 155-156° C |
| 178 | $CH_3$ | $CClF_2$ | H | 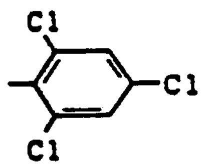 | 2 | Fp. 128-134° C |
| 179 | $CH_3$ | $CF_3$ | $COC_2H_5$ | 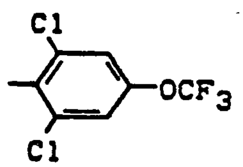 | 1 | Fp. 60-65° C |
| 180 | $CH_3$ | $CF_3$ | $COC_2H_5$ | 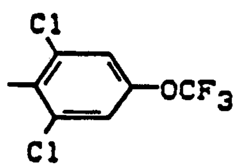 | 2 | Fp. 122-123° C |
| 181 | $CH_3$ | $CCl_2F$ | $COC_2H_5$ | 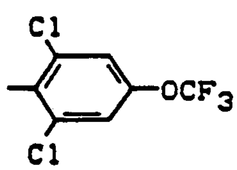 | 2 | Fp. 146-147° C |
| 182 | $CH_3$ | $CCl_2F$ | $COC_2H_5$ | 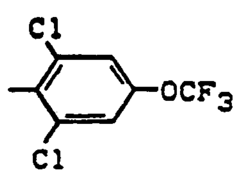 | 2 | Fp. 172°C |
| 183 | $CH_3$ | $CClF_2$ | $COC_2H_5$ | 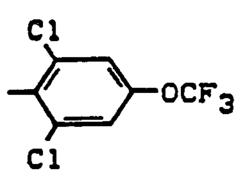 | 1 | Fp. 129-130° C |

| Bsp. Nr. | $R^1$ | $R^2$ | $R^3$ | Ar | n | physikalische Eigenschaften |
|---|---|---|---|---|---|---|
| 184 | $CH_3$ | $CClF_2$ | $COC_2H_5$ | 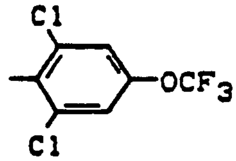 | 2 | Fp. 151°C |
| 185 | $CH_3$ | $CF_3$ | H | 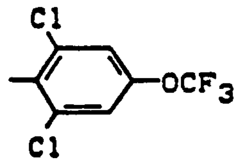 | 1 | Fp. 65-70° C |
| 186 | $CH_3$ | $CF_3$ | H | 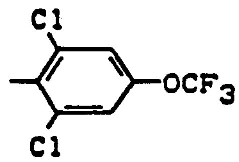 | 2 | Fp. 121-123° C |
| 187 | $CH_3$ | $CCl_2F$ | H | 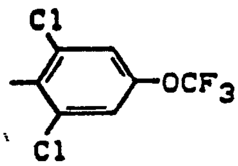 | 1 | Fp. 72-76° C |
| 188 | $CH_3$ | $CCl_2F$ | H | 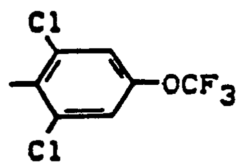 | 2 | Fp. 72-76° C |
| 189 | $CH_3$ | $CClF_2$ | H | 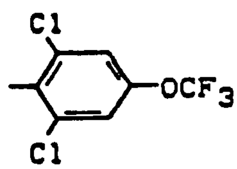 | 1 | Fp. 63-67° C |
| 190 | $CH_3$ | $CClF_2$ | H | 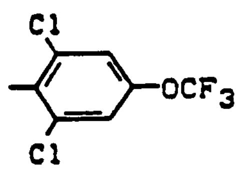 | 2 | Fp. 114-115° C |
| 191 | $CH_3$ | $CH_2$-$CH_2$- 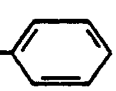 | H | 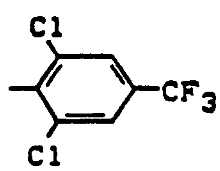 | 0 | Fp. 108-110° C |

| Bsp. Nr. | $R^1$ | $R^2$ | $R^3$ | Ar | n | physikalische Eigenschaften |
|---|---|---|---|---|---|---|
| 192 | $CH_3$ | $CH_3$ | H | 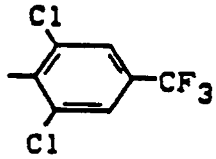 | 1 | Fp. 56-60° C |
| 193 | $CH_3$ | $CH_3$ | H | 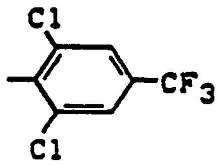 | 2 | Fp. 179-181° C |
| 194 | $CH_3$ | $C_2H_5$ | H | 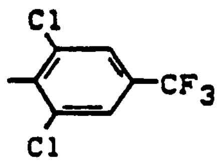 | 1 | Fp. 62-66° C |
| 195 | $CH_3$ | $C_2H_5$ | H | 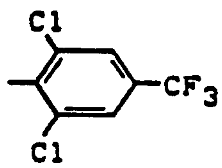 | 2 | Fp. 140-156° C |
| 196 | $(CH_3)_3C$ | $CH_3$ | H | 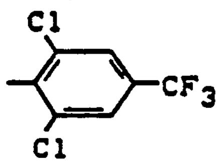 | 0 | FP 81°C |
| 197 | $CH_3$ | 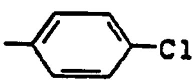 | H | 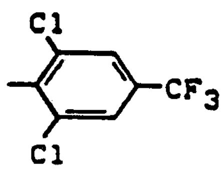 | 0 | Fp. 72° C |
| 198 | $CH_3$ | 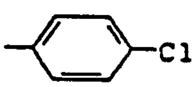 | H | 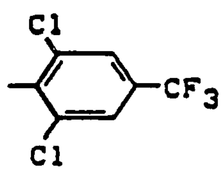 | 2 | Fp. 73-78° C |
| 199 | H | $C_2H_5$ | H | 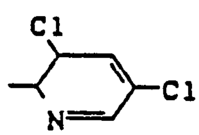 | 0 | Fp. 61° C |

| Bsp. Nr. | $R^1$ | $R^2$ | $R^3$ | Ar | n | physikalische Eigenschaften |
|---|---|---|---|---|---|---|
| 200 | $CH_3$ | $CCl_2F$ | H | 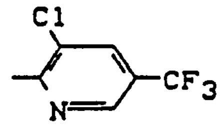 | 2 | NMR: Öl, 7,8 (s,1H) 8,2 (1H) 8,6 (1H) ($CDCl_3$/TMS) |
| 201 | $CH_3$ | $CF_3$ | H | 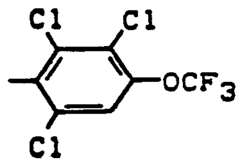 | 0 | Fp 119-123°C |
| 202 | H | $CF_2Cl$ | H | 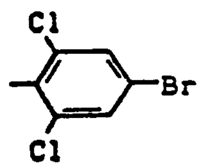 | 2 | Fp. 148-150° C |
| 203 | H | $CF_2Cl$ | H | 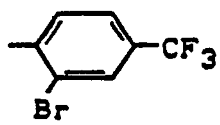 | 0 | Fp. 96-98° C |
| 204 | H | $CF_2Cl$ | H | 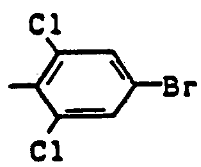 | 1 | Fp. 110-115° C |
| 205 | H | $CF_3$ | H | 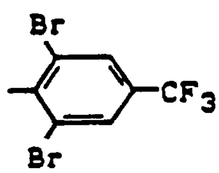 | 0 | Fp. 126° C |
| 206 | H | $CF_3$ | H | 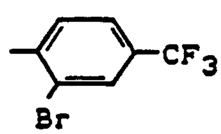 | 2 | Fp. 144° C |
| 207 | H | $CCl_2F$ | H | 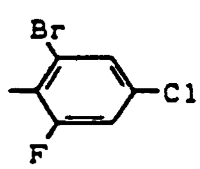 | 0 | NMR: 7,63 ppm (s,1H) 4,35 ppm (s breit,2H) |

| Bsp. Nr. | $R^1$ | $R^2$ | $R^3$ | Ar | n | physikalische Eigenschaften |
|---|---|---|---|---|---|---|
| 208 | H | $CF_3$ | H | 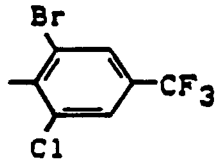 | 1 | Fp. 82-88° C |
| 209 | H | $CF_3$ | H | 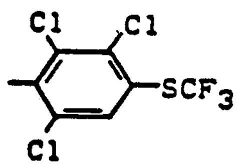 | 0 | Fp. 110-111°C |
| 210 | H | $CCl_2F$ | H | 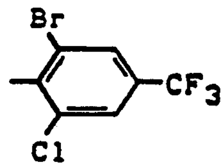 | 0 | Fp. 110-115° C |
| 211 | H | $CCl_2F$ | H | 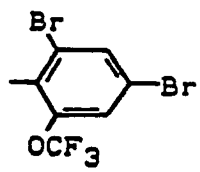 | 0 | Fp. 73° C |
| 212 | H | $CCl_2F$ | H | 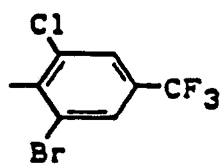 | 2 | Fp. 145° C |
| 213 | H | $CCl_2F$ | H | 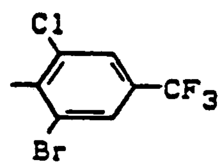 | 1 | Fp. 99-112° C |
| 214 | H | $CCl_2F$ | H | 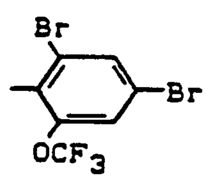 | 2 | Fp. 132° C |

| Bsp. Nr. | $R^1$ | $R^2$ | $R^3$ | Ar | n | physikalische Eigenschaften |
|---|---|---|---|---|---|---|
| 215 | H | $CCl_2F$ | H | 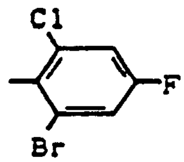 | 0 | Fp. 119° C |
| 216 | H | $CF_2Cl$ | H | 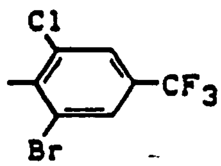 | 0 | Fp. 116-118° C |
| 217 | H | $CF_2Cl$ | H | 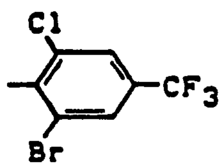 | 1 | Fp. 117-118° C |
| 218 | H | $CCl_2F$ | H | 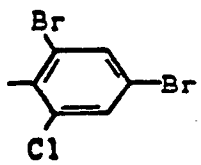 | 0 | Fp. 84-86° C |
| 219 | H | $CF_2Cl$ | H | 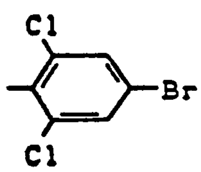 | 0 | Fp. 87-88° C |
| 220 | H | 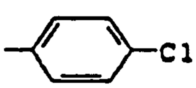 | H | 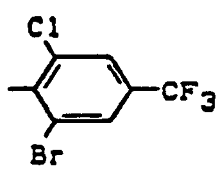 | 0 | Fp. 150-156° C |
| 221 | H | $CCl_2F$ | H | 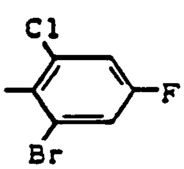 | 2 | Fp. 80-85° C |

| Bsp. Nr. | $R^1$ | $R^2$ | $R^3$ | Ar | n | physikalische Eigenschaften |
|---|---|---|---|---|---|---|
| 222 | H | $CCl_2F$ | H | 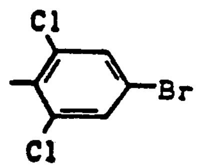 | 2 | Fp. 165-168° C |
| 223 | H | 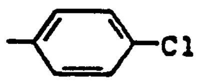 | H | 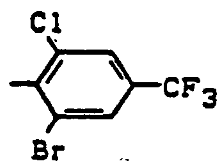 | 2 | Fp. 170-175° C |
| 224 | H | $CCl_2F$ | H | 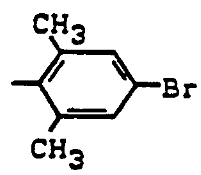 | 0 | Fp. 125-133° C |
| 225 | H | $CCl_2F$ | H | 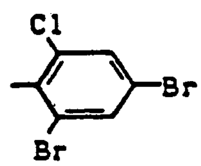 | 1 | Fp. 102-107° C |
| 226 | H | $CCl_2F$ | H | 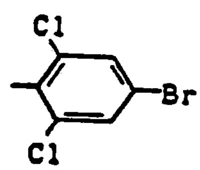 | 1 | Fp. 70-77° C |
| 227 | H | $CCl_2F$ | H | 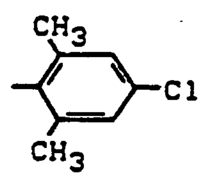 | 0 | Fp. 134-136° C |
| 228 | H | $CCl_2F$ | H | 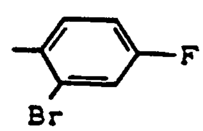 | 0 | Fp. 70° C |

| Bsp. Nr. | $R^1$ | $R^2$ | $R^3$ | Ar | n | physikalische Eigenschaften |
|---|---|---|---|---|---|---|
| 229 | H | $CF_2Cl$ | H | 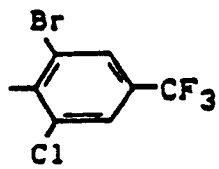 | 2 | Fp. 112-120° C |
| 230 | H | $CCl_2F$ | H | 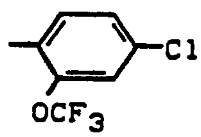 | 0 | NMR Öl, 7,62 ppm (s,1H) 4,24 ppm (s,2H) ($CDCl_3$/TMS) |
| 231 | H | $CClF_2$ | H | 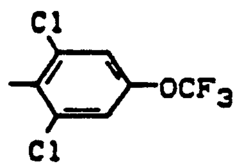 | 0 | Fp. 103-108° C |
| 232 | H | $CClF_2$ | H | 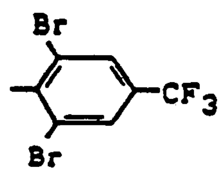 | 0 | Fp. 119-122° C |
| 233 | H | $CClF_2$ | H | 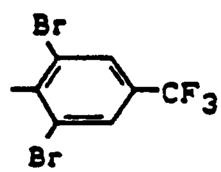 | 2 | Fp. 128-133° C |
| 234 | H | $CClF_2$ | H | 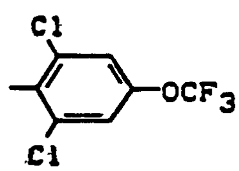 | 1 | Fp. 78-98° C |
| 235 | H | $CClF_2$ | H | 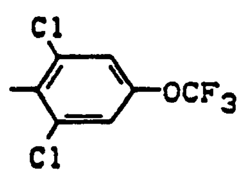 | 2 | Fp. 98-103° C |

| Bsp. Nr. | $R^1$ | $R^2$ | $R^3$ | Ar | n | physikalische Eigenschaften |
|---|---|---|---|---|---|---|
| 236 | H | $CCl_2F$ | H | 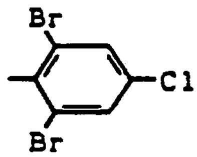 | 0 | Fp. 88-90° C |
| 237 | H | $CCl_2F$ | H | 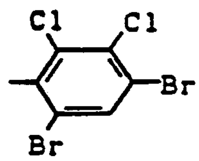 | 0 | Fp. 80-85° C |
| 238 | H | $CCl_2F$ | H | 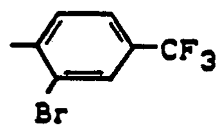 | 1 | Fp. 106-111° C |
| 239 | H | $CCl_2F$ | H | 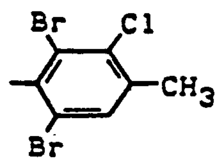 | 0 | Fp. 109-113° C |
| 240 | H | $CCl_2F$ | H | 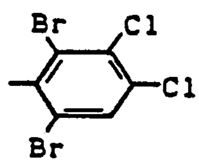 | 0 | Fp. 113-116° C |
| 241 | H | $CCl_2F$ | H | 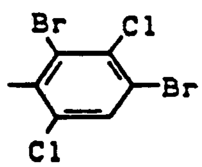 | 2 | Fp. 80° C |
| 242 | H | $CCl_2F$ | H | 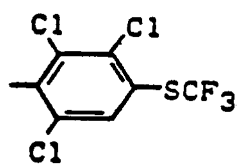 | 0 | Fp. 114-118°C |

| Bsp. Nr. | $R^1$ | $R^2$ | $R^3$ | Ar | n | physikalische Eigenschaften |
|---|---|---|---|---|---|---|
| 243 | H | $CClF_2$ | H | 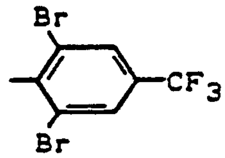 | 1 | Fp. 63-70° C |
| 244 | H | $CF_3$ | H | 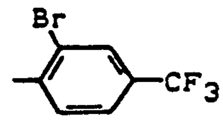 | 0 | Fp. 94° C |
| 245 | H | $CCl_2F$ | $CH_3$ | 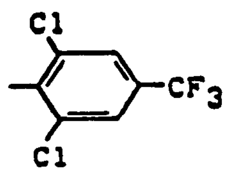 | 2 | Fp. 144-150° C |
| 246 | H | $CCl_2F$ | H | 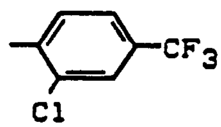 | 1 | Fp. 70-72° C |
| 247 | H | $CCl_2F$ | $CH_3$ | 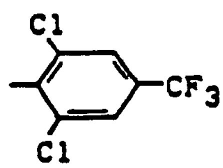 | 1 | Fp. 141-146° C |
| 248 | H | $CF_3$ | $COCH_3$ | 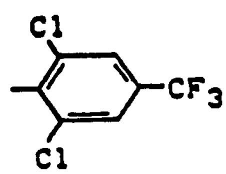 | 1 | Fp. 103° C |
| 249 | H | $CF_3$ | H | 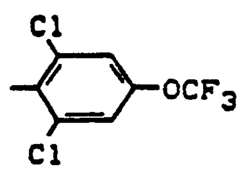 | 0 | Fp. 131-132° C |

| Bsp. Nr. | $R^1$ | $R^2$ | $R^3$ | Ar | n | physikalische Eigenschaften |
|---|---|---|---|---|---|---|
| 250 | H | $CF_3$ | H | 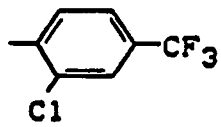 | 0 | Fp. 82-83° C |
| 251 | H | $CCl_2F$ | H | 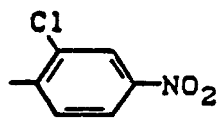 | 0 | Fp. 137-139° C |
| 252 | H | $CF_3$ | H | 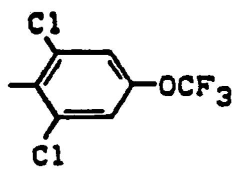 | 1 | Fp. 55-57° C |
| 253 | H | $CCl_2F$ | H | 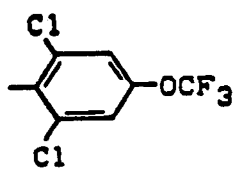 | 1 | Fp. 59-66° C |
| 254 | H | $CF_3$ | H | 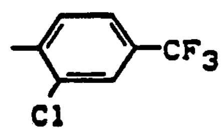 | 1 | Fp. 133° C |
| 255 | H | $CCl_2F$ | H | 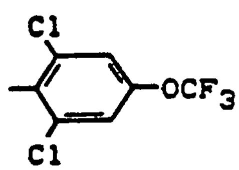 | 2 | Fp. 107-112° C |
| 256 | H | $CF_3$ | H | 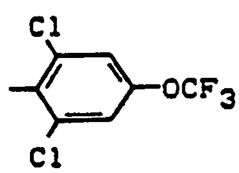 | 2 | Fp. 110-116° C |
| 257 | H | $CF_3$ | H | 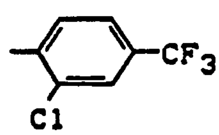 | 2 | Fp. 108-111° C |

| Bsp. Nr. | $R^1$ | $R^2$ | $R^3$ | Ar | n | physikalische Eigenschaften |
|---|---|---|---|---|---|---|
| 258 | H | $CCl_2F$ | H | 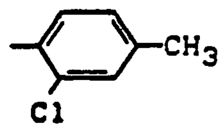 | 0 | Fp. 116-118° C. |
| 259 | H | $CCl_2F$ | H | 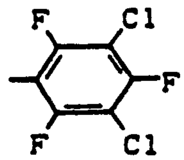 | 0 | Fp. 95-97° C |
| 260 | H | 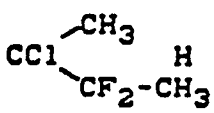 | H | 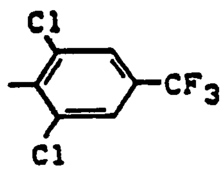 | 0 | Fp. 121° C |
| 261 | H | $CCl_2F$ | H | 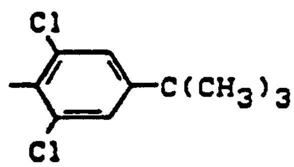 | 0 | Fp. 150-152° C |
| 262 | H | $CCl_2F$ | H | 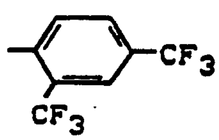 | 0 | Fp. 81-83° C |
| 263 | H | $CCl_2F$ | H | 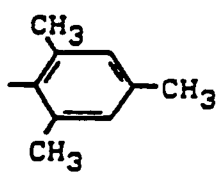 | 0 | Fp. 154-156° C |
| 264 | H | $CClF_2$ | H | 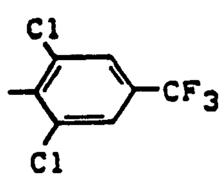 | 1 | Fp. 92-99° C |

| Bsp. Nr. | $R^1$ | $R^2$ | $R^3$ | Ar | n | physikalische Eigenschaften |
|---|---|---|---|---|---|---|
| 265 | H | $CF_3$ | H | 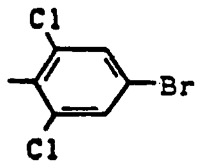 | 0 | Fp. 122-124° C |
| 266 | H | $CF_3$ | H | 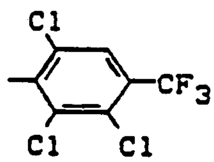 | 0 | Fp. 109-114° C |
| 267 | H | $CClF_2$ | H | 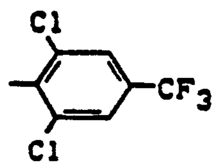 | 2 | Fp. 125-132° C |
| 268 | H | $CF_3$ | $CH_3$ | 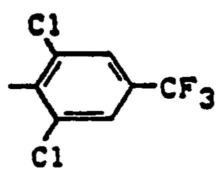 | 1 | Fp. 125-127° C |
| 269 | H | $CCl_2F$ | $COCH_3$ | 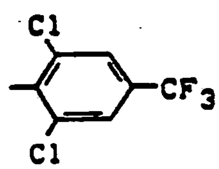 | 1 | Fp. 208-210° C |
| 270 | H | $CCl_2F$ | $CH(CH_3)_2$ | 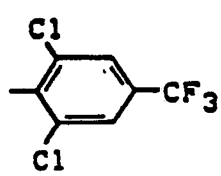 | 2 | NMR: Öl, 7,88 ppm (s,1H) 1,07 ppm (d,6H) ($CDCl_3$/TMS) |
| 271 | H | $CCl_2F$ | $COCH_3$ | 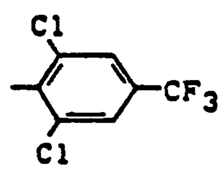 | 0 | Fp. 166-169° C |

| Bsp. Nr. | $R^1$ | $R^2$ | $R^3$ | Ar | n | physikalische Eigenschaften |
|---|---|---|---|---|---|---|
| 272 | H | $CF_3$ | H | 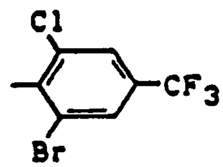 | 0 | Fp. 127-128° C |
| 273 | H | $CCl_2F$ | $COCH_3$ | 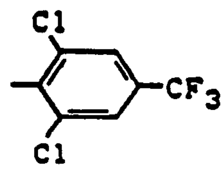 | 2 | Fp. 164-165° C |
| 274 | H | $CF_3$ | H | 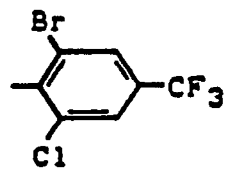 | 2 | Fp. 135-136° C |
| 275 | H | $CH(CH_3)_2$ | H | 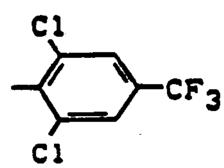 | 1 | Fp. 60-65° C |
| 276 | H | $CH_3$ | H | 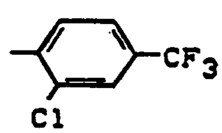 | 0 | Fp. 92° C |
| 277 | H | $C_2H_5$ | H | 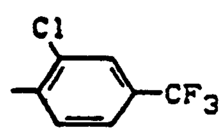 | 0 | Fp. 67-68° C |
| 278 | H | $CH(CH_3)_2$ | H | 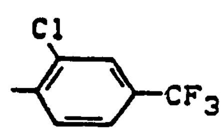 | 0 | Fp. 82-83° C |

| Bsp. Nr. | $R^1$ | $R^2$ | $R^3$ | Ar | n | physikalische Eigenschaften |
|---|---|---|---|---|---|---|
| 279 | H | $C_2H_5$ | H | 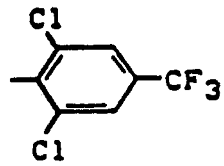 | 1 | Fp. 57-61° C |
| 280 | H | $C_2H_5$ | H | 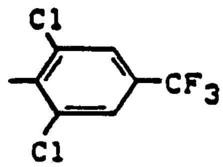 | 2 | Fp. 130° C |
| 281 | H | $CH(CH_3)_2$ | H | 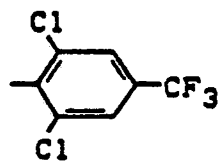 | 2 | Fp. 142° C |
| 282 | H | $CH_3$ | H | 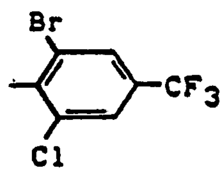 | 0 | Fp. 105° C |
| 283 | H | $C_2H_5$ | H | 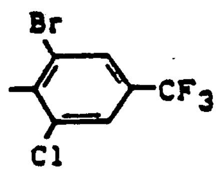 | 0 | Fp. 85° C |
| 284 | H | $CH_3$ | H | 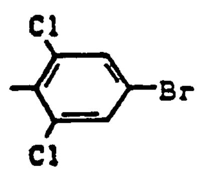 | 0 | Fp. 110° C |
| 285 | H | $C_2H_5$ | H | 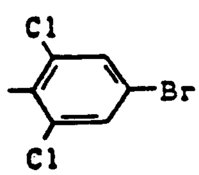 | 0 | Fp. 72-73° C |

| Bsp. Nr. | $R^1$ | $R^2$ | $R^3$ | Ar | n | physikalische Eigenschaften |
|---|---|---|---|---|---|---|
| 286 | H | $CH(CH_3)_2$ | H | 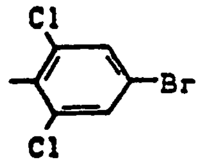 | 0 | NMR: 2,24 ppm (d,6H) 3,95 ppm (s,1H) 7,57 ppm (s,1H) 7,66 ppm (s,2H) ($CDCl_3$/TMS) |
| 287 | H | 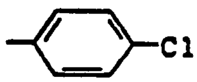 | H | 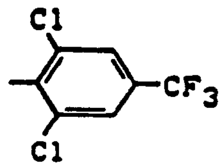 | 0 | Fp. 155° C |
| 288 | H | 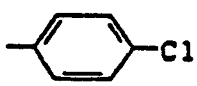 | H | 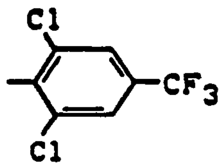 | 2 | Fp. 178° C |
| 289 | H | $CH_3$ | H | 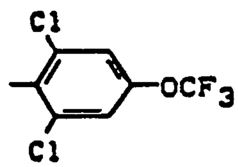 | 0 | Fp. 90° C |
| 290 | H | $C_2H_5$ | H | 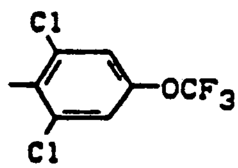 | 0 | Fp. 79° C |
| 291 | H | $CH_3$ | H | 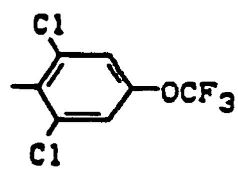 | 1 | Fp. 146° C |
| 292 | H | $CH_3$ | H | 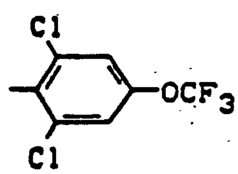 | 2 | Fp. 124° C |

| Bsp. Nr. | $R^1$ | $R^2$ | $R^3$ | Ar | n | physikalische Eigenschaften |
|---|---|---|---|---|---|---|
| 293 | H | $C_2H_5$ | H | 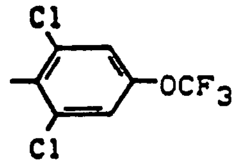 | 1 | Fp. 108-110° C |
| 294 | H | $C_2H_5$ | H | 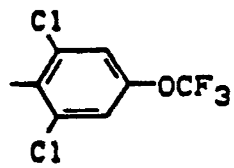 | 2 | Fp. 129° C |
| 295 | H | $CH(CH_3)_2$ | H | 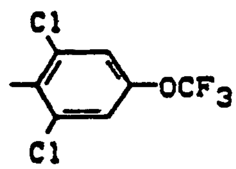 | 0 | Fp. 80° C |
| 296 | H | $CF_2CHFCl$ | H | 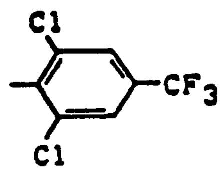 | 0 | Fp. 73° C |
| 297 | H | $CH_2$-$CH_2$- 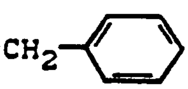 | H | 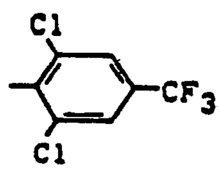 | 0 | Fp. 68-69° C |
| 298 | H | $CCl_2F$ | H | 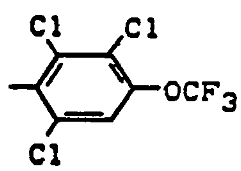 | 0 | Fp. 106-110°C |
| 299 | H | $CF_3$ | H | 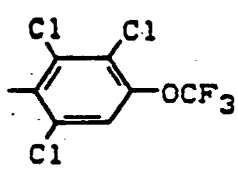 | 0 | Fp. 84-87°C |

| Bsp. Nr. | $R^1$ | $R^2$ | $R^3$ | Ar | n | physikalische Eigenschaften |
|---|---|---|---|---|---|---|
| 300 | H | $CCl_2F$ | $C_2H_5$ | 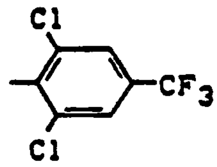 | 0 | NMR: (3-H,Pyr.) 7.75 (s,1H) ($CDCl_3$/TMS) |
| 301 | H | $CCl_2F$ | H | 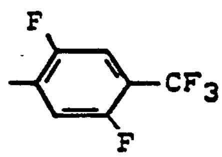 | 0 | NMR: (3-H,Pyr.) 7,71 (s,1H) ($CDCl_3$/TMS) |
| 302 | H | $CCl_2F$ | H | 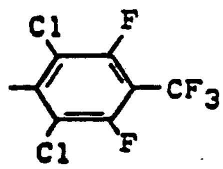 | 0 | Fp. 81-87°C |
| 303 | H | $CCl_2F$ | H | 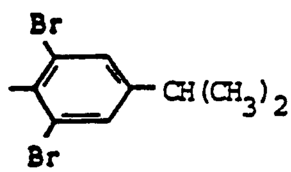 | 0 | Fp. 130-135°C |
| 304 | H | $CF_3$ | H | 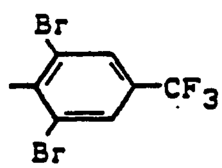 | 2 | Fp. 145-147°C |
| 305 | H | $CF_3$ | H | 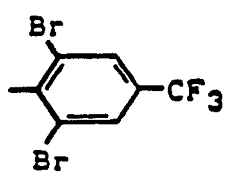 | 1 | Fp. 66-75°C |
| 306 | $CH_3$ | $CF_3$ | $CH_3$ | 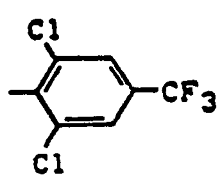 | 0 | Fp. 64-65°C |

| Bsp. Nr. | $R^1$ | $R^2$ | $R^3$ | Ar | n | physikalische Eigenschaften |
|---|---|---|---|---|---|---|
| 307 | $CH_3$ | $CF_3$ | $CH_3$ | 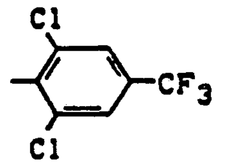 | 1 | Fp. 250°C |
| 308 | $CH_3$ | $CClF_2$ | $CH_3$ | 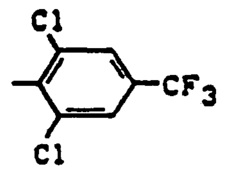 | 0 | Fp. 95-97°C |
| 309 | $CH_3$ | $CF_3$ | $CH_3$ | 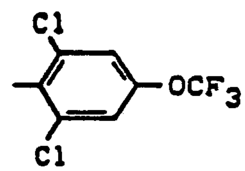 | 0 | Fp. 80-82°C |
| 310 | $CH_3$ | $CF_3$ | $CH_3$ | 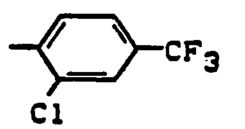 | 0 | Fp. 68-69°C |
| 311 | $CH_3$ | $CCl_2F$ | $CH_3$ | 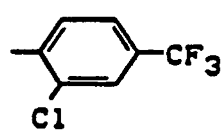 | 0 | Öl |

Anwendungsbeispiele

In den folgenden Anwendungsbeispielen werden die nachstehend aufgeführten Verbindungen als Vergleichsverbindungen einsetzt:

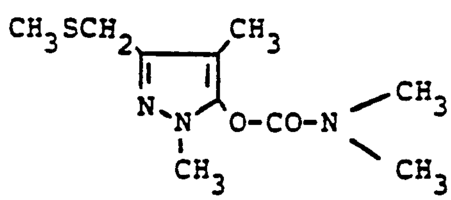

(A)

1,4-Dimethyl-5-[N,N-(dimethyl)-carbamoyloxy]-3-methylthiomethyl-pyrazol (bekannt aus DE—OS 2 912 494)

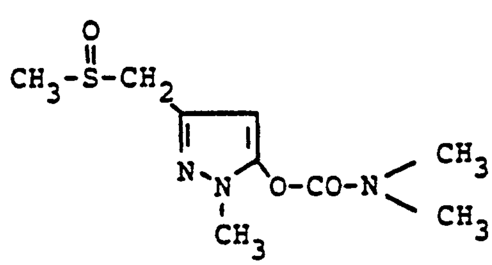

(B)

5-[N,N-(dimethyl)-carbamoyloxy]-1-methyl-3-methylsulfinylmethyl-pyrazol (bekannt aus DE—OS 2 819 932)

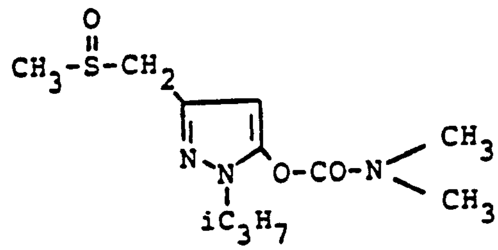

(C)

5-[N,N-(Dimethyl)-carbamoyloxy]-1-isopropyl-3-methylsulfinylmethyl-pyrazol (bekannt aus DE—OS 2 819 932)

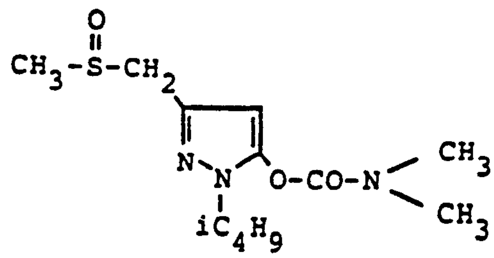

(D)

5-[N,N-(Dimethyl)-carbamoyloxy]-1-isobutyl-3-methylsulfinylmethyl-pyrazol (bekannt aus DE—OS 2 819 932)

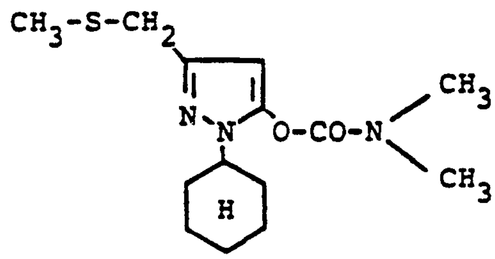

(E)

1-Cyclohexyl-5-[N,N-(dimethyl)-carbamoyloxy]-3-methylthiomethyl-pyrazol (bekannt aus DE—OS 2 839 270)

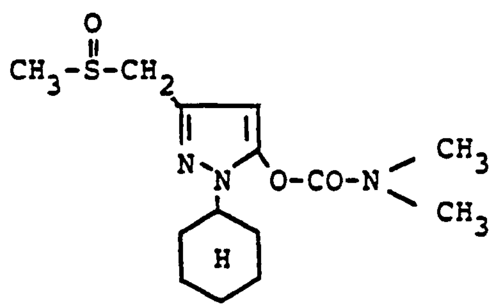

(F)

1-Cyclohexyl-5-[N,N-(dimethyl)-carbamoyloxy]-3-methylsulfinylmethyl-pyrazol (bekannt aus DE—OS 2 839 270)

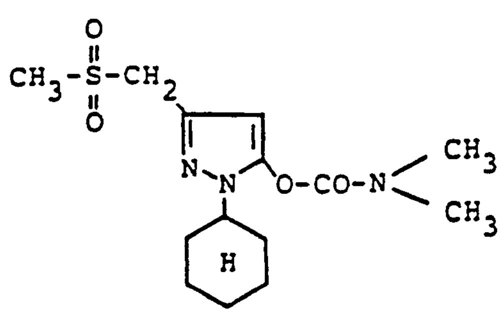

(G)

1-Cyclohexyl-5-[N,N-(dimethyl)-carbamoyloxy]-3-methylsulfonylmethyl-pyrazol (bekannt aus DE—OS 2 839 270)

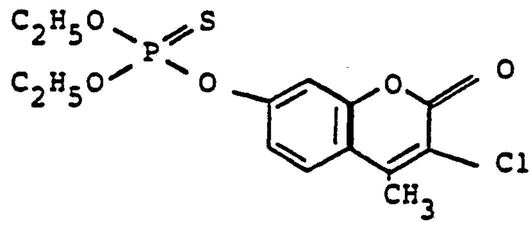

(H)

O,O-Diethyl-L-(3-chlor-4-methyl-cumarin-7-yl)-thionophosphorsäureester (bekannt aus "Pflanzenschutz und Schädlingsbekämpfung", K. H. Büchel; G. Thieme Verlag Stuttgart 1967, S. 38)

Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meeretttichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käferlarven abgetötet wurden; 0% bedeutet, daß keine Käfer-Larven abgetötet wurden.

Bei diesem Test zeigen z.B. die folgenden Verbindungen der Herstellungsbeispiele überklegene Wirksamkeit gegenüber dem Stand der Technik: 1, 5, 7, 10, 11, 13, 14, 15, 16, 17, 18, 24, 25, 29, 31, 49, 61, 62, 78, 70, 66, 63, 90, 22, 51a, 83, 92, 38, 37, 36, 77, 247.

Beispiel B

Plutella-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser aus die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 3, 5, 10, 11, 12, 13, 16, 17, 21, 23, 25, 27, 29, 30, 31, 61, 70, 90, 22, 51a, 83, 92, 38, 36, 77, 247.

Beispiel C

Tetranychus-Test (resistent)

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 10, 11, 17, 21, 70, 66, 63, 90, 51a, 37.

Beispiel D

Grenzkonzentrations-Test/Bodeninsekten

Testinsekt: Phorbia antiqua Maten im Boden

Lösingsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 3, 10, 12, 11, 9, 66.

Beispiel E

Grenzkonzentrations-Test/Wurzelsystemische Wirkung

Testinsekt: Phaedon cochleariae

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entsprechend ist allein die Wirkstoffgewichtsmenge pro Volumeneinheit Boden, welche in ppm (= mg/l) angegeben wird. Man füllt den behandelten Boden in Töpfe und beflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 3, 10, 12, 11, 9, 66.

Beispiel F

Grenzkonzentrations-Test

Testnematode: Meloidogyne incognita

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18°C.

Nach sechs Wochen werden die Kartoffelwurzeln auf Zystem untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirkung gegenüber dem Stand der Technik: 12.

Beispiel G

$LD_{100}$-Test

Testtiere: Blatta orientalis

Lösungsmittel: Aceton

2 Gewichtsteile Wirkstoff werden in 1000 Volumenteilen Lösungsmittel aufgenommen. Die so

erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m Filterpapier verschieden hoch. Anschließend gibt man die angegebene Anzahl der Testtiere in die Petrischale und bedeckt sie mit einem Glassdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik: 3, 10, 11, 16, 25.

## Beispiel H

$LD_{100}$-Test

Testtier: Sitophilus granarius

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m Filterpapier verschieden hoch. Anschließend gibt man die angegebene Anzahl der Testtiere in die Petrischale und bedeckt sie mit einem Glassdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik: 3, 10, 11, 12, 16, 17, 18, 24, 25, 27, 28, 29, 30, 31, 49, 61.

## Beispiel I

Test mit Lucilia cuprina Larven (OP-res. Goondiwindi-Stamm)

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 5, 10, 11, 12, 22, 23, 25, 27, 29, 30, 35, 36, 37, 38, 39, 66, 67.

## Beispiel K

Test mit Boophilus microplus resistent/OP-resistenter Biarra-Stamm

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 5, 10, 11, 12, 23, 25, 27, 36, 37, 38, 39, 66, 67.

## Beispiel L

Facefly-Test (Musca autumnalis)

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiden entsprechender Größe enthalten, die einen Tag vor Versuchsbeginn mit 1 ml der zu testenden Wirkstoffzubereitung durchtränkt wurden. Nach 3 Stunden wird der Abtötungsgrad in Prozent bestimmt, wobei

100% bedeuten, daß alle und 0%, daß keine fliegen abgetötet worden sind.

Bei diesem Test zeigen z.B. die folgenden Verbindungen der Herstellungsbeispiele überlegene Wirkung gegenüber dem Stand der Technik: 22, 25, 35, 36, 38.

Beispiel M

Test mit Psoroptes ovis

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen den oben angegebenen Lösungsmittel-Gemisches und verdünnt das so erhaltene Konzentrat mit Wasser auf die gewünschte Konzentration.

Etwa 10—25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 25, 35, 38, 66, 67.

Beispiel N

Test mit parasitierenden, adulten Stechfliegen (Stromoxys calcitrans)

Lösungsmittel: Cremophor

Zur Herstellung einer zweckmäßrigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Stechfliegen (Stromoxys calcitrans) werden din Petrischalen, die Sandwiches enthalten, die einen Tag vor Versuchsbeginn mit 1 ml der zu testenden Wirkstoffzubereitung durchtränkt wurden, gebracht. Nach 3 Stunden wird der Abtötungsgrad in Prozent bestimmt, wobei 100% bedeuten, daß alle und 0%, daß keine Fliege abgetötet worden sind.

Bei diesem Test zeigen z.B. die folgenden Verbindungen der Herstellungsbeispiele überlegene Wirkung gegenüber dem Stand der Technnik: 66, 67.

Beispiel P

Nematoden in vitro Test

Geprüft wird die Abtötung und Hemmung der Vermehrung des Nematoden Caenorhabditis elegans in flüssigem Medium in Gegenwart von Bakterien (E. coli), die den Nematoden als Nahrung dienen.

Die zu prüfenden Substanzen werden den Kulturen zugesetzt und die resultierende Beeinträchtigung der Vermehrung als nematizide Wirkung ausgewertet. Angegeben wird die Konzentration, die die Vermehrung verhindert.

Bei diesem Test zeigen z.B. die folgenden Verbindungen der Herstellungsbeispiele eine mindestens 95%ige Hemmung der Vermehrung des Nematoden C. elegans

— bei einer Konzentration von ≤ 100 µg/ml: 5, 7, 8, 10, 12, 13, 14, 15, 16, 17, 18, 19, 23, 25, 27, 28, 29, 41
— bei einer Konzentration von ≤ 10 µg/ml: 30, 36, 40.

**Patentansprüche**

1. Insektizide, akarizide und nematizide Mittel, gekennzeichnet durch einen Gehalt an mindestens einem 5-Aminopyrazol der allgemeinen Formel (I)

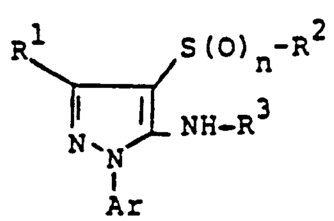

(I)

in welcher

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht oder für jeweils gegebenenfalls einfach oder mehrfach, gleich oder verschieden substituiertes Phenylalkyl oder Phenyl mit 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für Wasserstoff, geradkettiges oder verzweigtes Alkyl mit 1 bis 4 Kohlenstoffatomen oder für einen Rest

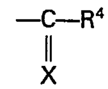

steht, wobei

X für Sauerstoff oder Schwefel steht, und

$R^4$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, außerdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten die bei $R^2$ genannten in Frage kommen,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$-S(O)_m-R^5$$

wobei

$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

m für eine Zahl 0, 1 oder 2 steht und

n ebenfalls für eine Zahl 0, 1 oder 2 steht.

2. Verfahren zur Bekämpfung von Insekten, Spinnentieren und Nematoden, dadurch gekennzeichnet, daß man 5-Aminopyrazole der Formel (I) nach Anspruch 1 auf Insekten, Spinnentiere und Nematoden und/oder ihren Lebensraum einwirken läßt.

3. Verwendung von 5-Aminopyrazolen der allgemeinen Formel (I) gemäß Anspruch 1 zur Bekämpfung von Insekten, Spinnentieren und Nematoden.

4. Verfahren zur Herstellung von insektiziden, akariziden und nematiziden Mitteln, dadurch gekennzeichnet, daß man 5-Aminopyrazole gemäß Formel (I) nach Anspruch 1 mit Streckmitteln und/oder oberflächenaktiven Mitteln vermischt.

5. 5-Aminopyrazole der allgemeinen Formel

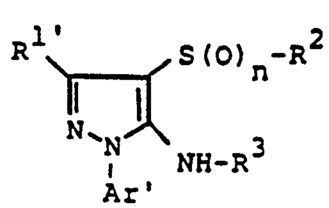

(Ia)

in welcher

$R^{1'}$ für jeweils geradkettiges oder verzweigtes Alkyl oder Halogenalkyl mit 1 bis 4 Kohlenstoffatomen und gegebenenfalls 1 bis 9 Halogenatomen steht,

Ar' für einfach oder mehrfach, gleich oder verschieden substituiertes Phenyl oder für gegebenenfalls einfach oder mehrfach, gleich oder verschieden substituiertes 2-Pyridyl, 3-Pyridyl oder 4-Pyridyl steht, wobei jeweils als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$-S(O)_m-R^5$$

wobei

$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

$R^2$ und $R^3$ die unter Formel (I) von Anspruch 1 angegebene Bedeutung besitzen und

n für eine Zahl 0, 1 oder 2 steht, sowie

m für eine Zahl 0, 1 oder 2 steht,

wobei $R^2$ nur dann für s-Butyl steht, wenn nicht gleichzeitig $R^1$ für Methyl, $R^3$ für Wasserstoff, n für 0 und Ar für einfach oder zweifach durch Chlor substituiertes Phenyl steht.

6. 5-Aminopyrazole der allgemeinen Formel

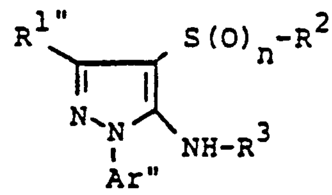

(Ib)

in welcher

$R^{1''}$ für Wasserstoff steht,

Ar'' für gegebenenfalls einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$-S(O)_m-R^5$$

wobei

$R^5$ für Amino sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

m für eine ganze Zahl 0, 1 oder 2 steht sowie

n für eine ganze Zahl 0, 1 oder 2 steht und

$R^2$ und $R^3$ die in Anspruch 1 unter Formel (I) angegebene Bedeutung besitzen.

7. Verfahren zur Herstellung von Verbindungen Formel (Ia)

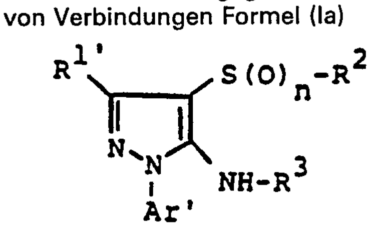

(Ia)

in welcher

$R^{1'}$ für jeweils geradkettiges oder verzweigtes Alkyl oder Halogenalkyl mit 1 bis 4 Kohlenstoffatomen und gegebenenfalls 1 bis 9 Halogenatomen steht,

Ar' für einfach oder mehrfach, gleich oder verschieden substituiertes Phenyl oder für gegebenenfalls einfach oder mehrfach, gleich oder verschieden substituiertes 2-Pyridyl, 3-Pyridyl oder 4-Pyridyl steht, wobei jeweils als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$-S(O)_m-R^5$$

wobei

$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

$R^2$ und $R^3$ die unter Formel (I) von Anspruch 1 angegebene Bedeutung besitzen und

n für eine Zahl 0, 1 oder 2 steht sowie

m für eine Zahl 0, 1 oder 2 steht,

wobei $R^2$ nur dann für s-Butyl steht, wenn nicht gleichzeitig $R^1$ für Methyl, $R^3$ für Wasserstoff, n für 0 und Ar für einfach oder zweifach durch Chlor substituiertes Phenyl steht, dadurch gekennzeichnet, daß man

(a) (α) 4-Thiocyanato-5-aminopyrazole der allgemeinen Formel (IIa),

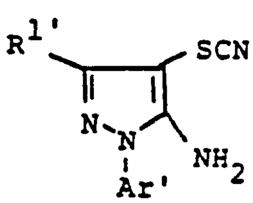

(IIa)

in welcher
$R^{1'}$ und Ar' die oben angegebene Bedeutung haben, oder
(a) (β) Bis-(pyrazolyl)-disulfide der Formel (IIIa),

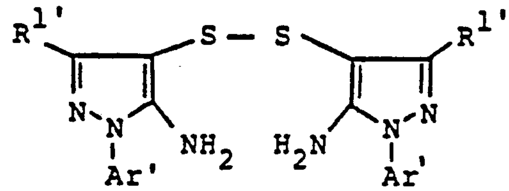
(IIIa)

in welcher
$R^{1'}$ und Ar' die oben angegebene Bedeutung haben,
mit Halogeniden der Formel (IV),

$R^2$—Hal (IV)

in welcher
$R^2$ die oben angegebene Bedeutung hat und
Hal für Halogen steht,
gegebenenfalls in Gegenwart eines Verdünnungsmittels sowie in Gegenwart eines geeigneten Reduktionsmittels und in Gegegenwart einer Base umsetzt,

oder daß man
(b) 4-unsubstituierte 5-Amino-pyrazole der Formel (Va),

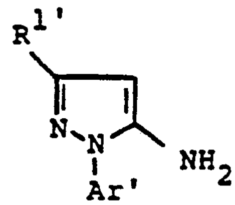
(Va)

in welcher
$R^{1'}$ und Ar' die oben angegebenen Bedeutungen
mit Sulfenylhalogeniden der Formel (VI),

$R^2$—S—Hal' (VI)

in welcher
$R^2$ die oben angegebene Bedeutung hat und
Hal' für Halogen steht,
gegebenenfalls in Gegenwart eines Verdünnungsmittels und gegebenenfalls in Gegenwart eines Säurebindemittels umsetzt,

oder daß man
(c) die nach Verfahren (a-α), (a-β) oder (b) erhältlichen 4-substituierten 5-Amino-pyrazole der Formel (Ic),

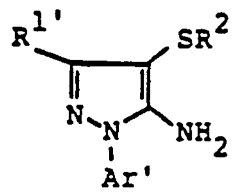
(Ic)

in welcher
$R^{1'}$, $R^2$ und Ar die oben angegebene Bedeutung haben
in allgemein üblicher Weise mit Acylierungsmitteln oder Alkylierungsmitteln der Formel (VII)

$R^6$—A (VII)

in welcher

$R^6$ für Alkyl oder einen Rest

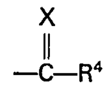

steht, wobei X und $R^4$ die oben angegebene Bedeutung haben und

A für eine elektronenziehende Abgangsgruppe steht,

oder mit Iso(thio)cyanaten der Formel (VIII)

$$R^7—N=C=X \quad (VIII)$$

in welcher

$R^7$ für Alkyl oder für ein gegebenenfalls substituiertes Aryl steht, und

X die oben angegebene Bedeutung hat,

gegebenenfalls in Gegenwart eines Verdünnungsmittels und gegebenenfalls in Gegenwart eines Säurebindemittels am Stickstoff der Aminogruppe in 5-Stellung des Pyrazolringes alkyliert bzw. acyliert,

oder daß man

(d) die nach Verfahren (a-α), (a-β), (b) oder (c) erhältlichen 5-Aminopyrazole der Formel (Id),

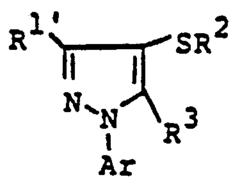

(Id)

in welcher

$R^{1'}$, $R^2$, $R^3$ und Ar' die oben angegebene Bedeutung haben,

mit Oxidationsmitteln der Formel (IX),

$$R—O—CH \quad (IX)$$

in welcher

R für Wasserstoff oder für jeweils gegebenenfalls substituiertes Alkanoyl oder Aroyl steht,

gegebenenfalls in Gegenwart eines Verdünnungsmittels, gegebenenfalls in Gegenwart eines Katalysators sowie gegebenenfalls in Gegenwart eines Säurebindemittels am Schwefel der Sulfenylgruppe in 4-Position des Pyrazolringes oxidiert.

8. Verfahren zur Herstellung von Verbindungen der Formel (Ib)

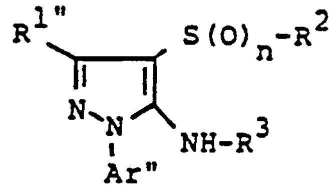

(Ib)

in welcher

$R^{1''}$ für Wasserstoff steht,

Ar'' für gegebenenfalls einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest

$$—S(O)_m—R^5$$

wobei

$R^5$ für Amino sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht,

m für eine ganze Zahl 0, 1 oder 2 steht sowie

n für eine Zahl 0, 1 oder 2 steht und

$R^2$ und $R^3$ die in Anspruch 1 unter Formel (I) angegebene Bedeutung besitzen, dadurch gekennzeichnet, daß man

(e-α) 4-Thiocyanato-5-aminopyrazole der allgemeinen Formel (IIb),

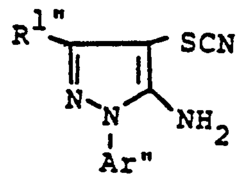

(IIb)

in welcher

$R^{1''}$ und Ar'' die oben angegebene Bedeutung haben,

oder

(e-β) Bis-(pyrazolyl)-disulfide der Formel (IIIb),

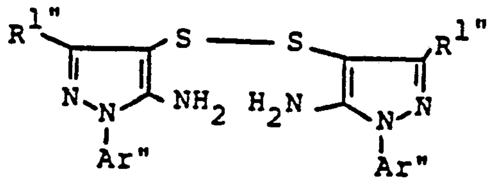

(IIIb)

in welcher

$R^{1''}$ und Ar'' die oben angegebene Bedeutung haben,

mit Halogeniden der Formel (IV),

$$R^2—Hal \qquad (IV)$$

in welcher

$R^2$ die oben angegebene Bedeutung hat und

Hal für Halogen steht,

gegebenenfalls in Gegenwart eines Verdünnungsmittels sowie in Gegenwart eines geeigneten Reduktionsmittels und in Gegenwart einer Base umsetzt,

oder daß man

(f) 4-unsubstituierte 5-Amino-pyrazole der Formel (Vb),

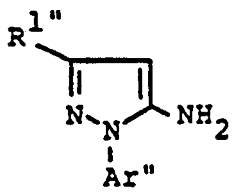

(Vb)

in welcher

$R^{1''}$ und Ar'' die oben angegebene Bedeutung haben,

mit Sulfenylhalogeniden der Formel (VI),

$$R^2—S—Hal' \qquad (VI)$$

in welcher

$R^2$ die oben angegebene Bedeutung hat und

Hal' für Halogen steht,

gegebenenfalls in Gegenwart eines Verdünnungsmittels und gegebenenfalls in Gegenwart eines Säurebindemittels umsetzt,

oder daß man

(g) die nach Verfahren (e-α), (e-β) oder (f) erhältlichen 4-substituierten 5-Amino-pyrazole der Formel (Ie),

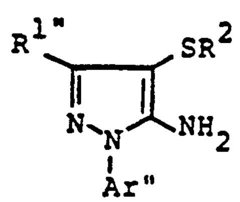

(Ie)

in welcher
$R^{1''}$, $R^2$ und Ar'' die oben angegebene Bedeutung haben,
in allgemein üblicher Weise mit Acylierungsmitteln oder Alkylierungsmitteln der Formel (VII)

$$R^6—A \qquad (VII)$$

in welcher
$R^6$ für Alkyl oder einen Rest

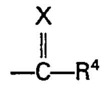

steht, und wobei X und $R^4$ die oben angegebene Bedeutung haben, und
A für eine elektronenziehende Abgangsgruppe steht,
oder mit Iso(thio)cyanaten der Formel (VIII)

$$R^7—N=C=X \qquad (VIII)$$

in welcher
$R^7$ für Alkyl oder für gegebenenfalls substituiertes Aryl steht, und
X die oben angegebene Bedeutung hat,
gegebenenfalls in Gegenwart eines Verdünnungsmittels und gegebenenfalls in Gegenwart eines Säurebindemittels am Stickstoff der Aminogruppe in 5-Stellung des Pyrazolringes alkyliert bzw. acyliert,

oder daß man
(h) die nach Verfahren (e-α), (e-β), (f) oder (g) erhältlichen 5-Aminopyrazole der Formel (If),

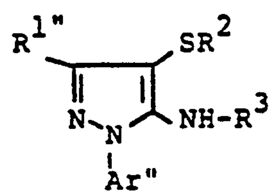

(If)

in welcher
$R^{1''}$, $R^2$, $R^3$ und Ar'' die oben angegebene Bedeutung haben,
mit Oxidationsmitteln der Formel (IX),

$$R—O—OH \qquad (IX)$$

in welcher
R für Wasserstoff oder für jeweils gegebenenfalls substituiertes Alkanoyl oder Aroyl steht,
gegebenenfalls in Gegenwart eines Verdünnungsmittels, gegebenenfalls in Gegenwart eines Katalysators sowie gegebenenfalls in Gegenwart eines Säurebindemittels am Schwefel der Sulfenylgruppe in 4-Position des Pyrazolringes oxidiert.

**Revendications**

1. Compositions insecticides, acaricides et nématicides, caractérisées par une teneur en au moins un 5-aminopyrazole de formule générale (I)

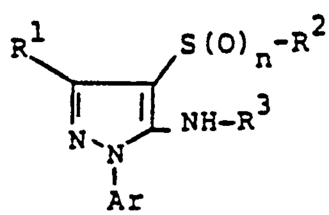

(I)

dans laquelle
$R^1$ désigne un groupe alkyle ou halogénalkyle à chaîne droite ou ramifiée ayant chacun 1 à 4 atomes de carbone et, le cas échéant, 1 à 9 atomes d'halogènes identiques ou différentes, ou l'hydrogène,
$R^2$ est un groupe alkyle ou halogénalkyle à chaîne droite ou ramifiée, ayant chacun 1 à 8 atomes de carbone et, le cas échéant, 1 à 17 atomes d'halogènes identiques ou différentes, ou un groupe phénylalkyle ou phényle portant éventuellement un ou plusieurs substituants identiques ou différents et ayant 1 à 4

atomes de carbone dans la partie alkyle à chaîne droite ou ramifiée, et on considère alors comme substituants du groupe phényle: un halogène, un radical cyano, nitro, un radical alkyle, alkoxy, alkylthio, alkylsulfinyle, alkylsulfonyle, halogénalkyle, halogénalkoxy, halogénalkylthio, halogénalkylsulfinyle ou halogénalkylsulfonyle à chaîne droite ou ramifiée, ayant chacun 1 à 4 atomes de carbone dans les parties alkyle individuelles et, le cas échéant, 1 à 9 atomes d'halogènes identiques ou différents,

$R^3$ représente l'hydrogène, un groupe alkyle à chaîne droite ou ramifiée ayant 1 à 4 atomes de carbone ou une reste

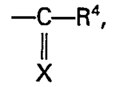

dans lequel

X désigne l'oxygène ou le soufre, et

$R^4$ est l'hydrogène, un groupe alkyle, alcényle, alcynyle, alkoxyalkyle, alkylthioalkyle, alkoxy, alkylthio, alkylamino, dialkylamino ou halogénalkyle à chaîne droite ou ramifiée ayant chacun jusqu'a 4 atomes de carbone dans les parties alkyle individuelles et, dans le cas du groupe halogénalkyle, jusqu'à 9 atomes d'halogènes indentiques ou différents, en outre un groupe cycloalkyle de 3 à 7 atomes de carbone portant éventuellement un ou plusieurs substituants halogéno, alkyle inférieur ou halogénalkyle inférieur identiques ou différents, ainsi qu'un groupe phényle, phénoxy, phénylthio ou phénylamino portant chacun, le cas échéant, un ou plusieurs substituants identiques ou différents, et on considère alors comme substituants du groupe phényle les substituants mentionnés pour $R^2$,

Ar désigne un groupe phényle, 2-pyridyle, 3-pyridyle ou 4-pyridyle portant éventuellement un ou plusieurs substituants identiques ou différents, et on considère alors comme substituants: un radical cyano, nitro, halogéno, un radical alkyle, alkoxy ou alkoxycarbonyle à chaîne droite ou ramifiée ayant chacun jusqu'à 4 atomes de carbone, en outre un groupe halogénalkyle ou halogénalkoxy à chaîne droite ou ramifiée ayant chacun jusqu'à 4 atomes de carbone et jusqu'à 9 atomes d'halogènes identiques ou différents ou un reste $—S(O)_m—R^5$

où

$R^5$ désigne un groupe amino, ainsi qu'un groupe alkyle, alkylamino, dialkylamino ou halogénalkyle à chaîne droite ou ramifiée ayant chacun jusqu'à 4 atomes de carbone dans les parties alkyle individuelles et, dans le cas du groupe halogénalkyle, jusqu'à 9 atomes d'halogènes identiques ou différents,

*m* représente le nombre 0, 1 ou 2 et

*n* représente également le nombre 0, 1 ou 2.

2. Procédé pour combattre des insectes, des acariens et des nématodes, caractérisé en ce qu'on fait agir des 5-aminopyrazoles de formule (I) suivant la revendication 1 sur insectes, les acariens et les nématodes et/ou sur leur milieu.

3. Utilisation de 5-aminopyrazoles de formule générale (I) suivant la revendication 1 pour combattre des insectes, des acariens et des nématodes.

4. Procédé de préparation de compositions insecticides, acaricides et nématicides, caractérisé en ce qu'on mélange des 5-aminopyrazoles de formule (I) suivant la revendication 1 avec des diluants et/ou des agents tensio-actifs.

5. 5-aminopyrazoles de formule générale

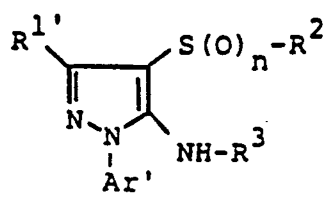

(Ia)

dans laquelle

$R^{1'}$ désigne un groupe alkyle ou halogénalkyle à chaîne droite ou ramifiée ayant 1 à 4 atomes de carbone et, le cas échéant, 1 à 9 atomes d'halogènes,

Ar' est un groupe phényle, portant un ou plusieurs substituants identiques ou différents, ou on groupe 2-pyridyle, 3-pyridyle ou 4-pyridyle, portant éventuellement un ou plusieurs subsituants identiques ou différents, et on considère alors dans chaque cas comme substituants: un radical cyano, nitro, halogéno, un radical alkyle, alkoxy ou alkoxycarbonyle à chaîne droite ou ramifiée ayant chacun jusqu'à 4 atomes de carbone, en outre un groupe halogénalkyle ou halogénalkoxy à chaîne droite ou ramifiée ayant chacun jusqu'à 4 atomes de carbone et jusqu'à 9 atomes d'halogènes identiques ou différents ou un reste $—S(O)_m—R^5$ dans lequel $R^5$ est un radical amino, ainsi qu'un groupe alkyle, alkylamino, dialkylamino ou halogénalkyl à chaîne droite ou ramifiée ayant chacun jusqu'à 4 atomes de carbone dans les parties alkyle individuelles et, dans le cas du groupe halogénalkyle, jusqu'à 9 atomes d'halogènes identiques ou différents,

$R^2$ et $R^3$ ont la dèfinition indiquée à propos de la formule (I) dans la revendication 1 et *n* est le nombre 0, 1 ou 2, de même que *m* et le nombre 0, 1 ou 2,

$R^2$ ne représentant un groupe sec.-butyle que lorsqu'il n'y a pas simultanément les valeurs méthyle pour $R^1$, hydrogène pour $R^3$, O pour *n* et un group phényle monochloré ou dichloré pour Ar.

6. 5-aminopyrazoles de formule générale

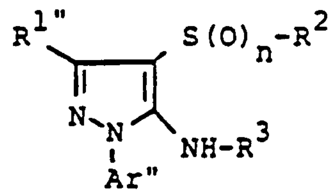

(Ib)

dans laquelle

$R^{1'''}$ représente l'hydrogène,

Ar'' est un groupe 2-pyridyl, 3-pyridyle ou 4-pyridyle, portant éventuellement un ou plusieurs subsituants identiques ou différents, et on considère alors comme substituants: un radical cyano, nitro, halogéno, un radical alkyle, alkoxy ou alkoxycarbonyle à chaîne droite ou ramifiée ayant chacun jusqu'à 4 atomes de carbone, en outre un radical halogénalkyle ou halogénalkoxy à chaîne droite ou ramifiée ayant chacun jusqu'à 4 atomes de carbone et jusqu'à 9 atomes d'halogènes identiques ou différents ou un reste $—S(O)_m—R^5$ où $R^5$ désigne un radical amino, ainsi qu'un radical alkyle, alkylamino, dialkylamino ou halogénalkyl à chaîne droite ou ramifiée ayant chacun jusqu'à 4 atomes de carbone dans les parties alkyle individuelles et, dans le cas du radical halogénalkyle, jusqu'à 9 atomes d'halogènes identiques ou différents,

*m* représente un nombre entier égal à 0, 1 ou 2 de même que

*n* représente un nombre entier égal à 0, 1 ou 2 et

$R^2$ et $R^3$ ont la dèfinition indiquée dans la revendication 1 pour la formule (I).

7. Procédé de préparation de composés de formule (Ia)

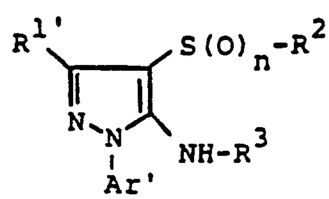

(Ia)

dans laquelle

$R^{1'}$ désigne un groupe alkyle ou halogénalkyle à chaîne droite ou ramifiée ayant 1 à 4 atomes de carbone et, le cas échéant, 1 à 9 atomes d'halogènes,

Ar' est un groupe phényle, portant un ou plusieurs substituants identiques ou différents, ou on groupe 2-pyridyle, 3-pyridyle ou 4-pyridyle, portant éventuellement un ou plusieurs subsituants identiques ou différents, et on considère alors dans chaque cas comme substituants: un radical cyano, nitro, halogéno, un radical alkyle, alkoxy ou alkoxycarbonyle à chaîne droite ou ramifiée ayant chacun jusqu'à 4 atomes de carbone, en outre un groupe halogénalkyle ou halogénalkoxy à chaîne droite ou ramifiée ayant chacun jusqu'à 4 atomes de carbone et jusqu'à 9 atomes d'halogènes identiques ou différents ou un reste $—S(O)_m—R^5$ dans lequel $R^5$ est un radical amino, ainsi qu'un groupe alkyle, alkylamino, dialkylamino ou halogénalkyl à chaîne droite ou ramifiée ayant chacun jusqu'à 4 atomes de carbone dans les parties alkyle individuelles et, dans le cas groupe halogénalkyle, jusqu'à 9 atomes d'halogènes identiques ou différents,

$R^2$ et $R^3$ ont la dèfinition indiquée à propos de la formule (I) dans la revendication 1 et *n* est le nombre 0, 1 ou 2, de même que *m* et le nombre 0, 1 ou 2,

$R^2$ ne représentant un groupe sec.-butyle que lorsqu'il n'y a pas simultanément les valeurs méthyle pour $R^1$, hydrogène pour $R^3$, O pour *n* et un group phényle monochloré ou dichloré pour Ar, caractérisé en ce que

(a) (α) on fait réagir des 4-thiocyanato-5-aminopyrazoles de formule générale (IIa)

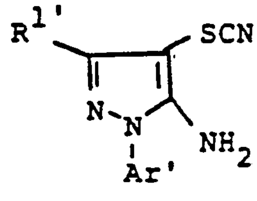

(IIa)

dans laquelle

$R^{1'}$ et Ar' ont la définition indiquée ci-dessus, ou bien

(a) (β) on fait réagir des disulfures de bis(pyrazolyle) de formule (IIIa)

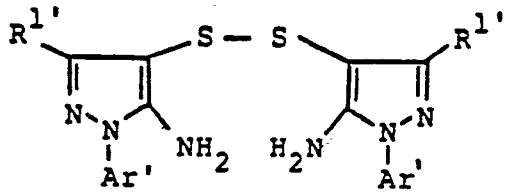

(IIIa)

dans laquelle

$R^{1'}$ et Ar' ont la définition indiquée ci-dessus, avec des halogénures de formule (IV)

$$R^2—Hal \quad (IV)$$

dans laquelle

$R^2$ a la définition indiquée ci-dessus et

Hal désigne un halogène, le cas échéant en présence d'un diluant ainsi qu'en la présence d'un agent réducteur approprié et en présence d'une base, ou bien

(b) on fait réagir des 5-aminopyrazoles non substitués en position 4 de formule (Va)

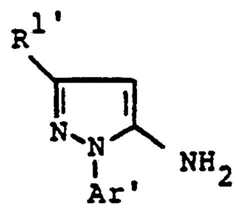

(Va)

dans laquelle

$R^{1'}$ et Ar' ont les définition indiquées ci-dessus, avec des halogénures de sulfényle de formule (VI)

$$R^2—S—Hal' \quad (VI)$$

dans laquelle

$R^2$ a la définition indiquée ci-dessus et Hal' désigne un halogène, le cas échéant en présence d'un diluant et en la présence éventuelle d'une accepteur d'acide, ou bien

(c) on alkyle ou acyle les 5-aminopyrazoles substitués en position 4, obtenus par le procédé (a-α), (a-β) ou (b), de formule (Ic)

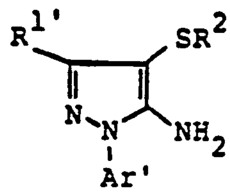

(Ic)

dans laquelle

$R^{1'}$, $R^2$ et Ar ont la définition indiquée ci-dessus d'une manière générale classique avec des agents acylants ou des agents alkylants de formule (VII)

$$R^6—A \quad (VII)$$

dans laquelle

$R^6$ est un groupe alkyle ou un reste

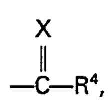

où X et

$R^4$ ont la définition indiquée ci-dessus et

A est un groupe partant attirant les électrons, ou avec des iso(thio)cyanates de formule (VIII)

$$R^7—N=C=X \quad (VIII)$$

dans laquelle

$R^7$ est un groupe alkyle ou un groupe aryle éventuellement substitué, et

X a la définition indiquée ci-dessus, le cas échéant en présence d'un diluant et en la présence éventuelle d'un accepteur d'acide, sur l'atome d'azote du groupe amino en position 5 du noyau de pyrazole, ou bien

(d) on oxyde les 5-aminopyrazoles obtenus par le procédé (a-α), (a-β), (b) ou (c) de formule (Id)

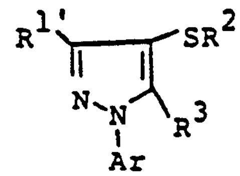

(Id)

dans laquelle

$R^{1'}$, $R^2$, $R^3$ et Ar' ont la définition indiquée ci-dessus, avec des agents oxydants de formule (IX)

R—O—OH (IX)

dans laquelle

R est l'hydrogène ou un groupe alcanoyle ou aroyle chacun éventuellement substitué, le cas échéant en présence d'un diluant, en la présence éventuelle d'un catalyseur ainsi que, le cas échéant, en présence d'un accepteur d'acide, sur l'atome de soufre du groupe sulfényle en position 4 du noyau de pyrazole.

8. Procédé de production de composés de formule (Ib)

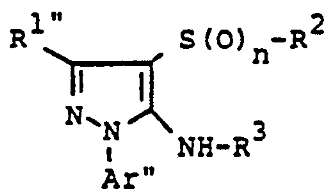

(Ib)

dans laquelle

$R^{1''}$ représente l'hydrogène,

Ar'' est un groupe 2-pyridyle, 3-pyridyle ou 4-pyridyle, portant éventuellement un ou plusieurs subsituants identiques ou différents, et on considère alors comme substituants: un radical cyano, nitro, halogéno, un radical alkyle, alkoxy ou alkoxycarbonyle à chaîne droite ou ramifiée ayant chacun jusqu'à 4 atomes de carbone, en outre un groupe halogénalkyle ou halogénalkoxy à chaîne droite ou ramifiée ayant chacun jusqu'à 4 atomes de carbone et jusqu'à 9 atomes d'halogènes identiques ou différents ou un reste $—S(O)_m—R^5$ où $R^5$ désigne un radical amino, ainsi qu'un radical alkyle, alkylamino, dialkylamino ou halogénalkyle à chaîne droite ou ramifiée ayant chacun jusqu'à 4 atomes de carbone dans les parties alkyle individuelles et, dans le cas du radical halogénalkyle, jusqu'à 9 atomes d'halogènes identiques ou différents,

*m* représente un nombre entier égal à 0, 1 ou 2 de même que

*n* représente un nombre entier égal à 0, 1 ou 2 et

$R^2$ et $R^3$ ont la dèfinition indiquée dans la revendication 1 pour la formule (I), caractérisé en ce que

(e-α) on fait réagir des 4-thiocyanato-5-aminopyrazoles de formule générale (IIb)

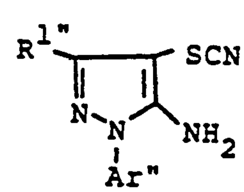

(IIb)

dans laquelle

$R^{1''}$ et Ar'' ont la définition indiquée ci-dessus, ou bien

(e-β) on fait réagir des disulfures de bis-(pyrazolyle) de formule (IIIb)

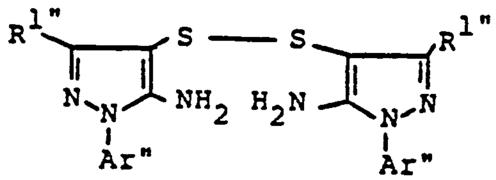

(IIIb)

dans laquelle

$R^{1''}$ et Ar'' ont la définition indiquée ci-dessus, avec des halogénures de formule (IV)

$$R^2\text{—Hal} \qquad \text{(IV)}$$

dans laquelle

$R^2$ a la définition indiquée ci-dessus, et

Hal est un halogène, le cas échéant en présence d'un diluant ainsi qu'en la présence d'un réducteur approprié et en présence d'une base, ou bien

(f) on fait réagir des 5-aminopyrazoles non substitués en position 4 de formule (Vb)

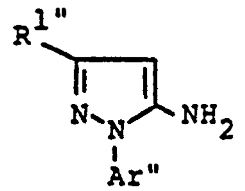

(Vb)

dans laquelle

$R^{1''}$ et Ar'' ont la définition indiquée ci-dessus, avec des halogénures de sulfényle de formule (VI)

$$R^2\text{—S—Hal}' \qquad \text{(VI)}$$

dans laquelle

$R^2$ a la définition indiquée ci-dessus, et

Hal' représente un halogène, le cas échéant en présence d'un diluant la présence éventuelle d'un accepteur d'acide, ou bien

(g) on alkyle ou on acyle les 5-aminopyrazoles substitués en position 4 obtenus par le procédé (e-α), (e-β) ou (f) de formule (Ie)

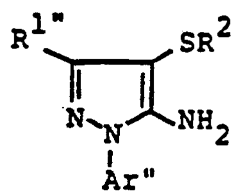

(Ie)

dans laquelle

$R^{1''}$, $R^2$ et Ar'' ont la définition indiquée ci-dessus, d'une manière généralement classique avec des agents d'acylation ou des agents d'alkylation de formule (VIII)

$$R^6\text{—A} \qquad \text{(VII)}$$

dans laquelle

$R^6$ est un groupe alkyle ou un reste

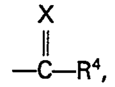

et X et $R^4$ ont la définition indiquée ci-dessus,

et

A est un groupe partant attirant les électrons, ou avec des iso(thio)cyanates de formule (VIII)

$$R^7\text{—N=C=X} \qquad \text{(VIII)}$$

dans laquelle

$R^7$ est un groupe alkyle ou un groupe aryle éventuellement substitué, et

X a la définiton indiquée ci-dessus, le cas échéant en présence d'un diluant et un présence éventuelle d'un accepteur d'acide, sur l'atome d'azote du groupe amino en position 5 du noyau de pyrazole, ou bien

(h) on oxyde des 5-aminopyrazoles obtenus par le procédé (e-α), (e-β), (f) ou (g) de formule (If)

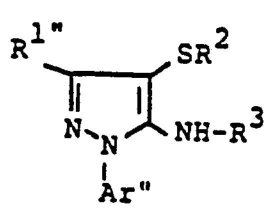

(If)

dans laquelle

$R^{1''}$, $R^2$, $R^3$ et Ar'' ont la définition indiquée ci-dessus, avec des agents oxydants de formule (IX)

$$R—O—OH \qquad (IX)$$

dans laquelle

R est l'hydrogène ou un groupe alcanoyle ou aroyle chacun éventuellement substitué, le cas échéant en présence d'un diluant, en la présence éventuelle d'un catalyseur ainsi que, le cas échéant, en présence d'un accepteur d'acide, sur l'atome de soufre du groupe sulfényle en position 4 du noyau de pyrazole.

## Claims

1. Insecticidal, acaricidal and nematicidal agents, characterized in that they contain at least one 5-aminopyrazole of the general formula (I)

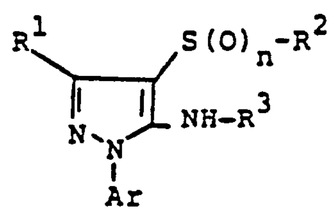

(I)

in which

$R^1$ represents in each case straight-chain or branched alkyl or halogenoalkyl with 1 to 4 carbon atoms and, where appropriate, 1 to 9 identical or different halogen atoms, or represents hydrogen,

$R^2$ represents in each case straight-chain or branched alkyl or halogenoalkyl with in each case 1 to 8 carbon atoms and, where appropriate, 1 to 17 identical or different halogen atoms, or represents phenylalkyl or phenyl with 1 to 4 carbon atoms in the straight-chain or branched alkyl part and in each case optionally monosubstituted or polysubstituted by identical or different substituents, possible substituents on the phenyl in each case being: halogen, cyano, nitro and in each case straight-chain or branched alkyl, alkoxy, alkylthio, alkylsuphinyl, alkylsulphonyl, halogenoalkyl, halogenoalkoxy, halogenoalkylthio, halogenoalkylsulphinyl and halogenalkylsulphonyl with in each case 1 to 4 carbon atoms in the individual alkyl parts and, where appropriate, 1 to 9 identical or different halogen atoms,

$R^3$ represents hydrogen, straight-chain or branched alkyl with 1 to 4 carbon atoms or a radical

$$—\underset{\underset{X}{\|}}{C}—R^4$$

wherein

X represents oxygen or sulphur and

$R^4$ represents hydrogen, or represents in each case straight-chain or branched alkyl, alkenyl, alkinyl, alkoxyalkyl, alkylthioalkyl, alkoxy, alkylthio, alkylamino, dialkylamino or halogenoalkyl with in each case up to 4 carbon atoms in the individual alkyl parts and, in the case of halogenalkyl, with up to 9 identical or different halogen atoms, or furthermore represents cycloalkyl which has 3 to 7 carbon atoms and is optionally monosubstituted or polysubstituted by identical or different substituents, from the group comprising halogen, lower alkyl and lower halogenoalkyl, or represents phenyl, phenoxy, phenylthio or phenylamino, in each case optionally monosubstituted or polysubstituted by identical or different substituents, possible substituents on the phenyl being those mentioned in the case of $R^2$,

Ar represents phenyl, 2-pyridyl, 3-pyridyl or 4-pyridyl, in each case optionally monosubstituted or polysubstituted by identical or different substituents, possible substituents being: cyano, nitro, halogen, in each case straight-chain or branched alkyl, alkoxy and alkoxycarbonyl with in each case up to 4 carbon atoms, furthermore in each case straight-chain or branched halogenoalkyl and halogenoalkoxy with in each case up to 4 carbon atoms and up to 9 identical or different halogen atoms, or a radical $—S(O)_m—R^5$, wherein

$R^5$ represents amino, or represents in each case straight-chain or branched alkyl, alkylamino, dialkylamino or halogenoalkyl with in each case up to 4 carbon atoms in the individual alkyl parts and, in the case of the halogenoalkyl, with up to 9 identical or different halogen atoms, and m represents the number 0, 1 or 2, and n likewise represents the number 0, 1 or 2.

2. Method of combating insects, arachnids and nematodes, characterized in that 5-aminopyrazoles of the formula (I) according to Claim 1 are allowed to act on insects, arachnids and nematodes and/or their environment.

3. Use of 5-aminopyrazoles of the general formula (I) according to Claim 1 for combating insects, arachnids, and nematodes.

4. Process for the preparation of insecticidal, acaricidal and nematicidal agents, characterized in that 5-aminopyrazoles according to formula (I) of Claim 1 are mixed with extenders and/or surface-active agents.

5. 5-Aminopyrazoles of the general formula

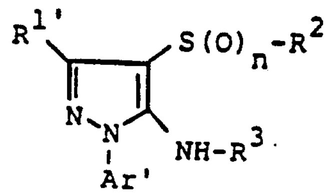

(Ia)

in which

$R^{1'}$ represents in each case straight-chain or branched alkyl or halogenoalkyl with 1 to 4 carbon atoms and, where appropriate, 1 to 9 halogen atoms,

Ar' represents phenyl which is monosubstituted or polysubstituted by identical or different subsituents, or represents 2-pyridyl, 3-pyridyl or 4-pyridyl, in each case optionally monosubstituted or polysubstituted by identical or different substituents, possible substituents in each case being: cyano, nitro, halogen, in each case straight-chain or branched alkyl, alkoxy or alkoxycarbonyl with in each case up to 4 carbon atoms, furthermore in each case straight-chain or branched halogenoalkyl and halogenoalkoxy with in each case up to 4 carbon atoms and up to 9 identical or different halogen atoms, or a radical $-S(O)_m-R^5$,

wherein

$R^5$ represents amino, or represents in each case straight-chain or branched alkyl, alkylamino, dialkylamino or halogenoalkyl with in each case up to 4 carbon atoms in the individual alkyl parts and, in the case of the halogenoalkyl, with up to 9 identical or different halogen atoms,

$R^2$ and $R^3$ have the meaning indicated for formula (I) of Claim 1, and n represents the number 0, 1, 2, and m also represents the number 0, 1 or 2, and wherein $R^2$ only represents s-butyl if, at the same time, $R^1$ does not represent methyl, $R^3$ does not represent hydrogen, n does not represent O and Ar does not represent phenyl which is monosubstituted or disubstituted by chlorine.

6. 5-Aminopyrazoles of the general formula

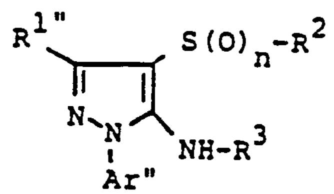

(Ib)

in which

$R^{1''}$ represents hydrogen,

Ar'' represents 2-pyridyl, 3-pyridyl or 4-pyridyl, in each case optionally monosubstituted or polysubstituted by identical or different substituents, possible substituents being: cyano, nitro, halogen, in each case straight-chain or branched alkyl, alkoxy or alkoxycarbonyl with in each case up to 4 carbon atoms, furthermore in each case straight-chain or branched halogenoalkyl and halogenoalkoxy with in each case up to 4 carbon atoms and up to 9 identical or different halogen atoms, or a radical $-S(O)_m-R^5$,

wherein

$R^5$ represents amino, or represents in each case straight-chain or branched alkyl, alkylamino, dialkylamino or halogenoalkyl with in each case up to 4 carbon atoms in the individual alkyl parts and, in the case of the halogenoalkyl, with up to 9 identical or different halogen atoms, and m represents the integer 0, 1 or 2, and n also represents the integer 0, 1 or 2, and

$R^2$ and $R^3$ have the meaning indicated in Claim 1 in the case of formula (I).

7. Process for the preparation of compounds of the formula (Ia)

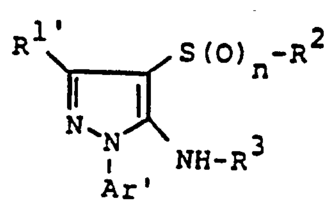

(Ia)

in which

$R^{1'}$ represents in each case straight-chain or branched alkyl or halogenoalkyl with 1 to 4 carbon atoms and, where appropriate, 1 to 9 halogen atoms,

Ar' represents phenyl which is monosubstituted or polysubstituted by identical or different subsituents, or represents 2-pyridyl, 3-pyridyl or 4-pyridyl, in each case optionally monosubstituted or

polysubstituted by identical or different substituents, possible substituents in each case being: cyano, nitro, halogen, in each case straight-chain or branched alkyl, alkoxy an alkoxycarbonyl with in each case up to 4 carbon atoms, furthermore in each case straight-chain or branched halogenoalkyl and halogenoalkoxy with in each case up to 4 carbon atoms and up to 9 identical or different halogen atoms, or a radical $-S(O)_m-R^5$,

wherein

$R^5$ represents amino, or represents in each case straight-chain or branched alkyl, alkylamino, dialkylamino or halogenoalkyl with in each case up to 4 carbon atoms in the individual alkyl parts and, in the case of the halogenoalkyl, with up to 9 identical or different halogen atoms,

$R^2$ and $R^3$ have the meaning indicated for formula (I) of Claim 1, and n represents the number 0, 1, 2, and m also represents the number 0, 1 or 2,

and wherein

$R^2$ only represents s-butyl if, at the same time, $R^1$ does not represent methyl, $R^3$ does not represent hydrogen, n does not represent O and Ar does not represent phenyl which is monosubstituted or disubstituted by chlorine, characterized in that

(a) (α) 4-thiocyanato-5-aminopyrazoles of the general formula (IIa)

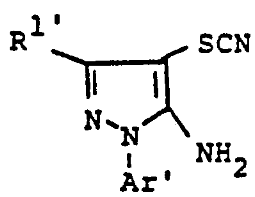

(IIa)

in which

$R^{1'}$ and Ar' have the abovementioned meaning, or

(a) (β) bis-(pyrazolyl) disulphides of the formula (IIIa)

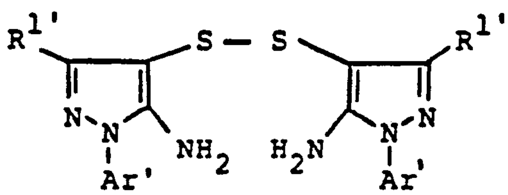

(IIIa)

in which

$R^{1'}$ and Ar' have the abovementioned meaning, are reacted with halides of the formula (IV)

$$R^2\text{—Hal} \quad (IV)$$

in which

$R^2$ has the abovementioned meaning and

Hal represents halogen, if appropriate in the presence of a diluent and also in the presence of a suitable reducing agent and in the presence of a base, or in that

(b) 4-unsubstituted 5-amino-pyrazoles of the formula (Va)

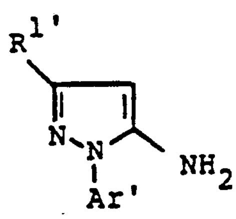

(Va)

in which

$R^{1'}$ and Ar' have the abovementioned meanings, are reacted with sulphenyl halides of the formula (VI)

$$R^2\text{—S—Hal}' \quad (VI)$$

in which

$R^2$ has the abovementioned meaning and

Hal' represents halogen, if appropriate in the presence of a diluent and if appropriate in the presence of an acid-binding agent, or in that

(c) the 4-substituted 5-amino-pyrazoles, obtainable by process (a-α), (a-β) or (b), of the formula (Ic)

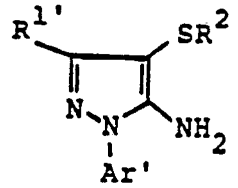

(Ic)

in which

$R^{1'}$, $R^2$ and Ar have the abovementioned meaning, are alkylated or acylated on the nitrogen of the amino group in the 5-position of the pyrazole ring in the generally customary manner with acylating agents or alkylating agents of the formula (VII)

$$R^6—A \qquad (VII)$$

in which

$R^6$ represents alkyl or a radical

$$—\overset{\overset{X}{\|}}{C}—R^4,$$

wherein

X and $R^4$ have the abovementioned meaning and

A represents an electron-drawing leaving group, or with iso(thio)cyanates of the formula (VIII)

$$R^7—N=C=X \qquad (VIII)$$

in which

$R^7$ represents alkyl or an optionally substituted aryl and

X has the abovementioned meaning, if appropriate in the presence of a diluent and if appropriate in the presence of an acid-binding agent, or in that

(d) the 5-aminopyrazoles, obtainable by process (a-α), (a-β), (b) or (c), of the formula (Id)

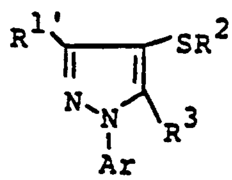

(Id)

in which

$R^{1'}$, $R^2$, $R^3$ and Ar' have the abovementioned meaning, are oxidized on the sulphur of the sulphenyl group in the 4-position of the pyrazole ring with oxidizing agents of the formula (IX)

in which

R represents hydrogen, or represents in each case optionally substituted alkanoyl or aroyl, if appropriate in the presence of a diluent, if appropriate in the presence of a catalyst and if appropriate in the presence of an acid-binding agent.

8. Process for the preparation of compounds of the formula (Ib)

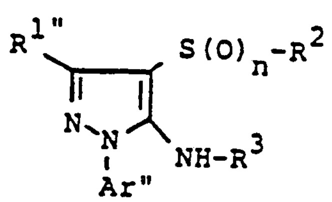

(Ib)

in which

$R^{1''}$ represents hydrogen,

Ar'' represents 2-pyridyl, 3-pyridyl or 4-pyridyl, in each case optionally monosubstituted or polysubstituted by identical or different substituents, possible substituents being: cyano, nitro, halogen, in each case straight-chain or branched alkyl, alkoxy an alkoxycarbonyl with in each case up to 4 carbon atoms, furthermore in each case straight-chain or branched halogenoalkyl and halogenoalkoxy with in each case up to 4 carbon atoms and up to 9 identical or different halogen atoms, or a radical $—S(O)_m—R^5$, wherein

$R^5$ represents amino, or represents in each case straight-chain or branched alkyl, alkylamino, dialkylamino or halogenoalkyl with in each case up to 4 carbon atoms in the individual alkyl parts and, in

the case of the halogenoalkyl, with up to 9 identical or different halogen atoms, and m represents the integer 0, 1 or 2, and n also represents the integer 0, 1 or 2, and

$R^2$ and $R^3$ have the meaning indicated in Claim 1 in the case of formula (I), characterized in that (e-α) 4-thiocyanato-5-aminopyrazoles of the general formula (IIb)

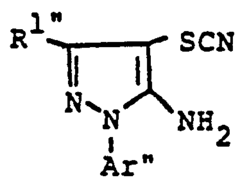

(IIb)

in which

$R^{1''}$ and Ar'' have the abovementioned meaning, or

(e-β) bis-(pyrazolyl) disulphides of the formula (IIIb)

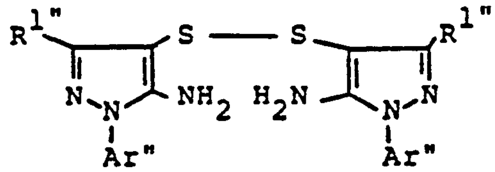

(IIIb)

in which

$R^{1''}$ and Ar'' have the abovementioned meaning, are reacted with halides of the formula (IV)

$$R^2\text{—Hal} \qquad (IV)$$

in which

$R^2$ has the abovementioned meaning and

Hal represents halogen, if appropriate in the presence of a diluent and also in the presence of a suitable reducing agent and in the presence of a base, or in that

(f) 4-unsubstituted 5-amino-pyrazoles of the formula (Vb)

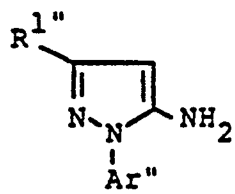

(Vb)

in which

$R^{1''}$ and Ar'' have the abovementioned meaning, are reacted with sulphenyl halides of the formula (VI)

$$R^2\text{—S—Hal}' \qquad (VI)$$

in which

$R^2$ has the abovementioned meaning and

Hal' represents halogen, if appropriate in the presence of a diluent and if appropriate in the presence of an acid-binding agent, or in that

(g) the 4-substituted 5-amino-pyrazoles, obtainable by process (e-α), (e-β) or (f), of the formula (Ie)

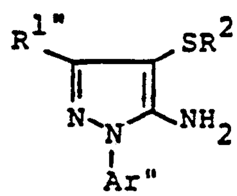

(Ie)

in which

$R^{1''}$, $R^2$ and Ar'' have the abovementioned meaning, are alkylated or acylated on the nitrogen of the amino group in the 5-position of the pyrazole ring in the generally customary manner with acylating agents or alkylating agents of the formula (VII)

$$R^6\text{—A} \qquad (VII)$$

in which

$R^6$ represents alkyl or a radical

$$-\overset{\overset{X}{\|}}{C}-R^4,$$

wherein

X and $R^4$ have the abovementioned meaning and

A represents an electron-drawing leaving group, or with iso(thio)cyanates of the formula (VIII)

$$R^7{-}N{=}C{=}X \qquad \text{(VIII)}$$

in which

$R^7$ represents alkyl or optionally substituted aryl and

X has the abovementioned meaning, if appropriate in the presence of a diluent and if appropriate in the presence of an acid-binding agent, or in that

(h) the 5-aminopyrazoles, obtainable by process (e-α), (e-β), (f) or (g), of the formula (If)

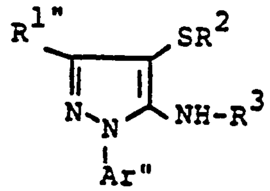

(If)

in which

$R^{1''}$, $R^2$, $R^3$ and Ar'' have the abovementioned meaning, are oxidized on the sulphur of the sulphenyl group in the 4-position of the pyrazole ring with oxidizing agents of the formula (IX)

$$R{-}O{-}OH \qquad \text{(IX)}$$

in which

R represents hydrogen, or represents in each case optionally substituted alkanoyl or aroyl, if appropriate in the presence of a diluent, if appropriate in the presence of a catalyst and if appropriate in the presence of an acid-binding agent.